(19) Europäisches Patentamt
European Patent Office
Office européen des brevets

(11) EP 2 510 124 B1

(12) # EUROPEAN PATENT SPECIFICATION

(45) Date of publication and mention of the grant of the patent:
**26.07.2017 Bulletin 2017/30**

(21) Application number: **10816306.4**

(22) Date of filing: **08.12.2010**

(51) Int Cl.:
***C12Q 1/68*** *(2006.01)*

(86) International application number:
**PCT/IB2010/003397**

(87) International publication number:
**WO 2011/070441 (16.06.2011 Gazette 2011/24)**

(54) **CATEGORIZATION OF DNA SAMPLES**
KLASSIFIZIERUNG VON DNA-PROBEN
CATÉGORISATION D'ÉCHANTILLONS D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2010 US 306201 P**
**20.04.2010 US 325977 P**
**11.12.2009 US 285758 P**

(43) Date of publication of application:
**17.10.2012 Bulletin 2012/42**

(73) Proprietor: **Nucleix**
**46710 Herzelya (IL)**

(72) Inventors:
- **FRUMKIN, Dan**
  **61999 Tel-aviv (IL)**
- **WASSERSTROM, Adam**
  **74100 Nes-Ziona (IL)**

(74) Representative: **Vossius & Partner**
**Patentanwälte Rechtsanwälte mbB**
**Siebertstrasse 3**
**81675 München (DE)**

(56) References cited:
**EP-A1- 2 071 035** **EP-A2- 1 748 080**
**WO-A1-2009/083989**

- **D. FRUMKIN ET AL.: "Authentication offorensic DNA samples", FORENSIC SCI. INT. GENET., 20 September 2001 (2001-09-20), XP26829808,**
- **DAN FRUMKIN ET AL: "DNA methylation-based forensic tissue identification", FORENSIC SCIENCE INTERNATIONAL: GENETICS, ELSEVIER BV, NETHERLANDS, vol. 5, no. 5, 6 December 2010 (2010-12-06), pages 517-524, XP028275689, ISSN: 1872-4973, DOI: 10.1016/J.FSIGEN.2010.12.001 [retrieved on 2010-12-13]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present inventive technology relates to methods for accurate and cost-effective categorization and profiling of DNA samples to identify the originating source and identity of the DNA sample or mixed DNA samples.

### SUMMARY OF THE INVENTION

The present invention relates to methods for accurate and cost-effective categorization of DNA samples into different types of *in vitro* generated DNA or different types of natural DNA such as from different tissues and/or physiological/pathological states. The invention achieves categorization by comparing "signal ratios" that are correlated to ratios of methylation levels at specific genomic loci, but does not rely on calculation of actual methylation levels at any genomic locus. Therefore the invention setup eliminates the requirement for external DNA species and/or controls, thereby both simplifying and increasing the accuracy of the assay. The inventive technology also enables performing the categorization of DNA together with DNA profiling in the same reaction, thereby allowing for concomitant categorization and determination of identity of the samples.

One aspect of the present invention is a method for categorization of a DNA sample according to claim 1. The method comprising:

(A) digesting a DNA sample with a methylation-sensitive and/or methylation-dependent restriction endonuclease;

(B) performing PCR on the digested DNA amplifying at least two genomic loci of which at least one is a restriction locus;

(C) determining the intensity of the signal of each amplification product;

(D) calculating "signal ratios" between the intensities of the signals produced by the loci; and

(E) comparing the signal ratios to reference values corresponding to different categories of DNA, wherein the category whose reference values correspond best to the signal ratios is determined to be the category of the DNA sample. That is, for instance, the approximation of the value of a signal ratio to the value of a reference value of a particular DNA category indicates the categorical source of the DNA sample. So if a signal ratio that has a value that is close to the value of a reference category, then the likelihood that the tested DNA sample originated from that particular type of category - based on the similarity in values between the signal ratio and the reference value - is high. Thus, the signal ratio(s) that best correspond to, or best approximate, a particular reference value(s) for a particular DNA category makes it likely that that DNA sample originated from that particular DNA categorical source. For examples about how to compare the signal ratios and category reference values see the disclosure elsewhere in this application, such as in the section entitled Algorithm and Software, which explains how to calculate probability distributions and likelihood probalities that a test DNA sample can be categorized to one or more reference categories.

In one embodiment, DNA digestion and PCR are performed in a single biochemical reaction in which the DNA template, digestion and amplification enzymes, buffers, primers, and accessory ingredients are placed together in a test tube and then the test tube is closed and placed in a thermal cycler, where the reaction takes place.

In one embodiment, the methylation-sensitive restriction endonuclease is unable to cut or digest a DNA if its recognition sequence is methylated. In one embodiment, a methylation-sensitive restriction endonuclease is selected from the group consisting of AatII, Acc65I, AccI, AciI, ACII, AfeI, AgeI, ApaI, ApaLI, AscI, AsiSI, AvaI, AvaII, BaeI, BanI, BbeI, BceAI, BcgI, BfuCI, BglI, BmgBI, BsaAI, BsaBI, BsaHI, BsaI, BseYI, BsiEI, BsiWI, BslI, BsmAI, BsmBI, BsmFI, BspDI, BsrBI, BsrFI, BssHII, BssKI, BstAPI, BstBI, BstUI, BstZ17I, Cac8I, ClaI, DpnI, DrdI, EaeI, EagI, EagI-HF, EciI, EcoRI, EcoRI-HF, FauI, Fnu4HI, FseI, FspI, HaeII, HgaI, HhaI, HincII, HincII, HinfI, HinP1I, HpaI, HpaII, Hpy166ii, Hpy188iii, Hpy99I, HpyCH4IV, KasI, MluI, MmeI, MspAII, MwoI, NaeI, NarI, NgoNIV, Nhe-HFI, NheI, NlaIV, NotI, NotI-HF, NruI, Nt.BbvCI, Nt.BsmAI, Nt.CviPII, PaeR7I, PleI, PmeI, PmlI, PshAI, PspOMI, PvuI, RsaI, RsrII, SacII, SalI, SalI-HF, Sau3AI, Sau96I, ScrFI, SfiI, SfoI, SgrAI, SmaI, SnaBI, TfiI, TscI, TseI, TspMI, and ZraI.

In another embodiment, a methylation dependent restriction enzyme can be used to digest a DNA sample. A methylation dependent restriction endonuclease digests only methylated DNA. Examples of such restriction enzymes include but are not limited to McrBC, McrA, and MrrA.

In one embodiment, at least one restriction locus is known to be methylated in natural DNA.

In another embodiment, at least one restriction locus is known to be unmethylated in natural DNA.

In another embodiment, at least two restriction loci are known to be differentially methylated in natural DNA.

In another embodiment, the signal ratio between at least two loci is known to be different for at least two potential categories.

In another embodiment, primer pairs used to amplify loci are selected to amplify the core short tandem repeat (STR) loci identified in the Federal Bureau of Investigation's Combined DNA Index System (CODIS).

In one embodiment, the CODIS loci are human genomic loci selected from the group consisting of D16S539 (SEQ ID NO. 1), D7S820 (SEQ ID NO. 2), D13S317 (SEQ ID NO. 3), D5S818 (SEQ ID NO. 4), CSF1PO (SEQ ID NO. 5), TPOX (SEQ ID NO. 6), TH01 (SEQ ID NO. 7), vWA (SEQ ID NO. 8), FGA (SEQ ID NO. 9), D21S11 (SEQ ID NO. 10), D8S1179 (SEQ ID NO. 11), D18S51 (SEQ ID NO. 12), and D3S1358 (SEQ ID NO. 13).

In a particular embodiment, the forward and reverse primers of a restriction and/or profile locus are designed to anneal to SEQ ID NO. 1, 2, 3, 4, 5, 6, 7, 8, 9, 10, 11, 12, 13, 14, 15, 16, 17, 18, 19, 20, 21, 22, 23, 24, or 25.

In one embodiment, the forward and reverse primers of a locus are available in a Promega Corporation commercial kit selected from the group consisting of PowerPlex® 16 HS (Cat.# DC2100, DC2101), PowerPlex® 16 (Cat.# DC6530, DC6531), PowerPlex® 2.1 (Cat.# DC6470, DC6471), PowerPlex® 16 BIO (Cat.# DC6540, DC6541), and PowerPlex® ES Systems (Cat.# DC6730, DC6731).

In one embodiment, the forward and reverse primers of a locus are available in an Applied Biosystems/Life commercial kit selected from the group consisting of SGM, SGM+, AmpFlSTR Identifiler, AmpFlSTR Profiler, AmpFlSTR ProfilerPlus, AmpFlSTR ProfilerPlusID, AmpFlSTR SEfiler, AmpFlSTR SEfiler Plus, AmpFlSTR Cofiler, AmpFlSTR Identifiler Direct, AmpFlSTR Identifiler Plus, AmpFlSTR NGM, AmpFlSTR Y-filer, AmpFlSTR Minifiler.

In one embodiment, the forward and reverse primers of a locus are available in a Qiagen commercial kit selected from the group consisting of Investigator ESSPlex, Investigator ESSPlex SE, Investigator Nonaplex ESSPlex, Investigator Hexaplex ESSPlex, Investigator Triplex AFS QS, Investigator Triplex DSF, Investigator IDplex, Investigator Decaplex SE, Investigator HDplex, Investigator Argus X-12, Investigator Y-12 QS, Investigator DIPplex.

In another embodiment, the restriction loci contain a HpaII recognition sequence and are selected from the group consisting of ADD6, ADD10, ADD 17 (SEQ ID NOs. 26-28) and Hypo23, Hypo28, Hypo33 (SEQ ID NOs. 29-31).In one embodiment, the DNA sample is mammalian DNA or plant DNA. In one embodiment, the mammalian DNA is DNA from a mammal selected from the group consisting of human, ape, monkey, rat, mouse, rabbit, cow, pig, sheep, and horse. In a particular embodiment, the mammalian DNA is human DNA. In one embodiment, the human DNA is from a male. In another embodiment, the human DNA is from a female.

In one embodiment, the human DNA sample is obtained from a male or female who has a disorder characterized by abnormal DNA methylation. In one embodiment, the disorder is a neurodevelopmental disorder of ICF (immunodeficiency, centromeric instability, and facial anomalies), Rett syndrome, and fragile X syndrome.

In one embodiment, the plant DNA is DNA from a plant. In one embodiment, the plant is a monocotyledonous plant selected from the group consisting of wheat, turf grass, cereal, maize, rice, oat, barley, sorghum, orchid, iris, lily, onion, banana, sugarcane, sorghum, and palm. In another embodiment, the plant is a dicotyledonous plant selected from the group consisting of avocado, potato, tobacco, tomato, sugarbeet, broccoli, cassava, sweet potato, pepper, cotton, poinsettia, legumes, alfalfa, soybean, carrot, strawberry, lettuce, oak, maple, walnut, rose, mint, squash, daisy, and cactus.

In one embodiment of the method, at least two loci are amplified by PCR with fluorescently labeled primers and wherein pairwise comparisons of the intensity of the fluorescent signals corresponding to the loci is performed in order to calculate signal ratios for each pair of loci.

In one embodiment, primer pairs are used to amplify pairs of loci selected from the group of pairs consisting of D3S1358/D18S51, D3S1358/D7S820, D3S1358/Penta_D, D3S1358/TPOX, D3S1358/FGA, TH01/Penta_D, D21S11/D18S51, D21S11/D7S820, D21S11/Penta_D, D21S11/AMEL, D21S11/TPOX, D21S11/FGA, D5S818/D18S51, vWA/D18S51, D5S818/Penta_E, vWA/Penta_E, D5S818/D7S820, D5S818/Penta_D, D5S818/TPOX, D5S818/FGA, D13S317/D7S820, D13S317/Penta_D, D13S317/TPOX, D13S317/FGA, D16S539/D7S820, CSF1PO/D7S820, vWA/D7S820, D8S1179/D7S820, D16S539/TPOX, D16S539/FGA, CSF1PO/Penta_D, CSF1PO/TPOX, vWA/Penta_D, AMEL/TPOX, AMEL/FGA, vWA/D8S1179, vWA/TPOX, vWA/FGA, D8S1179/TPOX, D8S1179/FGA.

In another embodiment, the restriction loci amplicons are smaller than the smallest amplicons used in DNA profiling, which is about 100 bps in size.

In one aspect of the present inventive technology, quantification of amplicon signals is performed and not necessarily the quantification of the amount of DNA templates. By calculating ratios of amplicon signals, no standard curve or reference DNA is needed since it is unnecessary to calculate actual DNA concentrations. In this embodiment, the ratios of amplicon signals is calculated. The signals may be signals detected from any kind of labeling used for detecting PCR amplification products, such as, but not limited to, ethidium bromide staining, sybr green staining, silver staining, or by fluorescence.

With respect to the latter, in one embodiment, the PCR reaction is performed with fluorescently labeled primers, and determination of the intensity of the signals of the amplification products is achieved by separation of products by capillary electrophoresis and quantification of fluorescence signals.

In another embodiment, the PCR is real time PCR, and determination of the intensity of the signals of the amplification products is achieved by quantification of fluorescence signals.

In a particular embodiment, the methylation-sensitive restriction endonuclease is HhaI.

In a particular embodiment, the methylation-sensitive restriction endonuclease is HhaI and the first restriction locus is the TPOX locus.

In a particular embodiment, the methylation-sensitive restriction endonuclease is HhaI and the first restriction locus is the FGA locus.

In a particular embodiment in which categorization is according to semen or non-semen, the methylation-sensitive restriction endonuclease is HhaI, the restriction loci are SD1, SD2, SD3, and SD4 (SEQ ID NOs. 33-36), a digested control locus is SD5 (SEQ ID NO. 37), and an undigested control locus is SD6 (SEQ ID NO. 38).

In a particular embodiment in which categorization is according to blood, saliva, semen, or skin epidermis, the methylation-sensitive restriction endonuclease is HhaI and the restriction loci are SEQ ID NOs. 39-53.

In a particular embodiment in which categorization is performed together with DNA profiling, and is according to semen or non-semen, the methylation-sensitive restriction endonuclease is HhaI, and the restriction loci are SEQ ID NO. 40 and SEQ ID NO. 54.

In one embodiment, a pair of loci are selected from loci with known methylation levels in at least two DNA categories, such that one of the loci in the pair is known to have a higher methylation level than the second locus in that pair in one DNA category but not in the other.

In another embodiment, a pair of loci are selected without knowledge of methylation levels as follows: select a pair of random genomic loci; design fluorescently labeled primers for amplifying each locus; amplify both loci in DNA samples obtained from different categories; separate amplicons with capillary electrophoresis; calculate signal ratios in the different DNA samples; if ratios calculated from samples corresponding to at least one category are significantly different (e.g. as determined by the Kolmogorov-Smirnov test with a threshold of $p = 0.05$) than those obtained from samples corresponding to at least one other category, select the pair for use in assay. In one embodiment, the calculated ratios are compared to a set of ratios obtained from natural samples and to a set of ratios obtained from artificial samples.

With respect to the latter, in one embodiment, the respective mean signal ratios of natural and artificial DNA samples digested with HhaI for specific pairs of loci determined herein are shown below.

| | Natural DNA | Artificial DNA |
|---|---|---|
| FGA(PowerPlex16)/Hypo12 | 10.3 | 0.89 |
| FGA(PowerPlex16)/Hypo28 | 12.4 | 0.76 |
| TPOX(PowerPlex16)/Hypo12 | 9.7 | 1.12 |

In another embodiment, the calculated ratios are compared to a set of ratios obtained from DNA samples extracted from different tissue categories. See Tables 1-4 presented in Figures 7-10 for additional examples of loci sequences and illustrative signal ratios and results used and obtained in accordance with the inventive categorization methods disclosed herein.

In one embodiment this method, or any method described herein, further comprises simultaneously profiling a DNA sample and determining whether the category of the DNA. In one embodiment, the step of profiling a DNA sample is performed according to instructions provided in a commercially available DNA profiling kit. In another embodiment, the commercially available DNA profiling kit is the PowerPlex®16 profiling kit (Promega Corporation).

In one embodiment, with respect to the latter, categorization of DNA is according to tissue types. Any tissue type can be used such as, but not limited to blood, saliva, semen, epidermis, urine, plasma, and hair.

Accordingly, the method for categorizing a DNA sample, comprising:

(A) digesting a DNA sample with at least one of a methylation-sensitive restriction endonuclease and a methylation-dependent restriction endonuclease;
(B) amplifying at least two genomic loci from the digested DNA, wherein at least one of the loci is a restriction locus;
(C) determining the signal intensity of each amplification product;
(D) calculating signal ratios between the signal intensities of the amplification products; and
(E) comparing the signal ratios to reference values of different DNA categories,

wherein the approximation of the value of a signal ratio to the values of reference ratios of a particular DNA category indicates the categorical source of the DNA sample.

In one embodiment, the reference values against which the signal ratios are compared are from at least one DNA category selected from the group consisting of tissue type, cell type, physiological condition, pathological condition, age, ethnicity, sex, methylation level, species, cell lines, natural DNA and artificially-synthesized DNA, risk of developing a pathological condition, prenatal conditions, prognosis, propensity to respond to treatment, effects of cell line sub-culturing, response to medication.

In another embodiment, the tissue type or cell type is selected from the group consisting of blood, saliva, semen, epidermis, urine, plasma, hair, menstrual blood, vaginal cells and/or secretion, sweat, feces, brain, esophagus, lung, stomach, heart, duodenum, liver, gall bladder, intestine, kidney, adrenal gland, urinary bladder, urethra, colon, testicle, ovary, uterus, vagina, muscle, tendon, ligament, fat, cartilage, bone, endothelial cells, uterine cervix, lymph, thyroid, pituitary gland, cerebellum, and breast.

In a particular embodiment, the tissue type or cell type is blood, saliva, semen, or epidermis.

In another embodiment, the pathological condition is cancer, inflammation, autoimmune disorder, metabolic disorder, infection, degenerative disease, hormonal imbalances, a disorder characterized by abnormal DNA methylation, neurodevelopmental disorder of ICF (immunodeficiency, centromeric instability, and facial anomalies), Rett syndrome, and fragile X syndrome.

In one embodiment, the prenatal condition in Prader-Willi syndrome, Angelman syndrome, Beckwith-Wiedemann syndrome, fragile X syndrome, Russell-Silver syndrome, Transient neonatal diabetes mellitus, Albright hereditary osteodystrophy, McCune-Albright syndrome, Familial nonchromaffin paraganglioma, Maternal and paternal UPD14 syndromes.

In another embodiment, the reference values against which the signal ratios are compared represent categories that are mixtures of other categories.

In another embodiment, the reference values against which the signal ratios are compared are from mixtures of semen and non-semen at various ratios.

In one embodiment, the methylation-sensitive restriction endonuclease is selected from the group consisting of AatII, Acc65I, AccI, AciI, ACII, AfeI, AgeI, ApaI, ApaLI, AscI, AsiSI, AvaI, AvaII, BaeI, BanI, BbeI, BceAI, BcgI, BfuCI, BglI, BmgBI, BsaAI, BsaBI, BsaHI, BsaI, BseYI, BsiEI, BsiWI, BslI, BsmAI, BsmBI, BsmFI, BspDI, BsrBI, BsrFI, BssHII, BssKI, BstAPI, BstBI, BstUI, BstZ17I, Cac8I, ClaI, DpnI, DrdI, EaeI, EagI, EagI-HF, EciI, EcoRI, EcoRI-HF, FauI, Fnu4HI, FseI, FspI, HaeII, HgaI, HhaI, HincII, HincII, HinfI, HinP1I, HpaI, HpaII, Hpy166ii, Hpy188iii, Hpy99I, HpyCH4IV, KasI, MluI, MmeI, MspA1I, MwoI, NaeI, NarI, NgoNIV, Nhe-HFI, NheI, NlaIV, NotI, NotI-HF, NruI, Nt.BbvCI, Nt.BsmAI, Nt.CviPII, PaeR7I, PleI, PmeI, PmlI, PshAI, PspOMI, PvuI, RsaI, RsrII, SacII, SalI, SalI-HF, Sau3AI, Sau96I, ScrFI, SfiI, SfoI, SgrAI, SmaI, SnaBI, TfiI, TscI, TseI, TspMI, and ZraI.

In a particular embodiment, the methylation-sensitive restriction endonuclease is HhaI.

In another embodiment, the methylation-dependent restriction endonuclease is selected from the group consisting of McrBC, McrA, and MrrA.

In one embodiment, the reference DNA category is natural DNA or artificially-synthesized DNA, wherein at least one genomic locus in the natural DNA or artificially-synthesized DNA comprises a core short tandem repeat (STR) locus used for DNA profiling.

In another embodiment, the genomic locus comprises a human locus selected from the group consisting of D16S539 (SEQ ID NO. 1), D7S820 (SEQ ID NO. 2), D13S317 (SEQ ID NO. 3), D5S818 (SEQ ID NO. 4), CSF1PO (SEQ ID NO. 5), TPOX (SEQ ID NO. 6), TH01 (SEQ ID NO. 7), vWA (SEQ ID NO. 8), FGA (SEQ ID NO. 9), D21S11 (SEQ ID NO. 10), D8S1179 (SEQ ID NO. 11), D18S51 (SEQ ID NO. 12), and D3S1358 (SEQ ID NO. 13), Penta D (SEQ ID NO. 14), Penta E (SEQ ID NO. 15), and Amelogenin (SEQ ID NOs. 16 and 17), D2S1338 (SEQ ID No. 18), D19S433(SEQ ID No. 19), ACTBP2SE33 (SEQ ID No. 20), D10S1248 (SEQ ID No. 21), D1S1656 (SEQ ID No. 22), D22S1045 (SEQ ID No. 23), D2S441 (SEQ ID No. 24), and D12S391 (SEQ ID No. 25).

In one embodiment, the primers for amplification of at least one of the genomic loci are available in:

(1) a Promega Corporation commercial kit selected from the group consisting of PowerPlex® 16 HS (Cat.# DC2100, DC2101), PowerPlex® 16 (Cat.# DC6530, DC6531), PowerPlex® 2.1 (Cat.# DC6470, DC6471), PowerPlex® 16 BIO (Cat.# DC6540, DC6541), and PowerPlex® ES Systems (Cat.# DC6730, DC6731);

(2) an Applied Biosystems commercial kit selected from the group consisting of SGM, SGM+, AmpFlSTR Identifiler, AmpFlSTR Profiler, AmpFlSTR ProfilerPlus, AmpFlSTR ProfilerPlusID, AmpFlSTR SEfiler, AmpFlSTR SEfiler Plus, AmpFlSTR Cofiler, AmpFlSTR Identifiler Direct, AmpFlSTR Identifiler Plus, AmpFlSTR NGM, AmpFlSTR Y-filer, and AmpFlSTR Minifiler; or

(3) Investigator ESSPlex, Investigator ESSPlex SE, Investigator Nonaplex ESSPlex, Investigator Hexaplex ESSPlex, Investigator Triplex AFS QS, Investigator Triplex DSF, Investigator IDplex, Investigator Decaplex SE, Investigator HDplex, Investigator Argus X-12, Investigator Y-12 QS, Investigator DIPplex.

In another embodiment, one of the genomic loci is known to be unmethylated in all tissues of natural DNA. In one embodiment, one of the genomic loci is selected from the group consisting of Hypo23, Hypo28, and Hypo33.

In another embodiment, the method further comprises calculating at least one of the following ratios:

(1) D3S1358/D18S51,
(2) D3S1358/D7S820,
(3) D3S1358/Penta_D,
(4) D3S1358/TPOX,
(5) D3S1358/FGA,
(6) TH01/Penta_D,
(7) D21S11/D18S51,
(8) D21S11/D7S820,
(9) D21S11/Penta_D,
(10) D21S11/AMEL,
(11) D21S11/TPOX,
(12) D21S11/FGA,
(13) D5S818/D18S51,
(14) vWA/D18S51,
(15) D5S818/Penta_E,
(16) vWA/Penta_E,
(17) D5S818/D7S820,
(18) D5S818/Penta_D,
(19) D5S818/TPOX,
(20) D5S818/FGA,
(21) D13S317/D7S820,
(22) D13S317/Penta_D,
(23) D13S317/TPOX,
(24) D13S317/FGA,
(25) D16S539/D7S820,
(26) CSF1PO/D7S820,
(27) vWA/D7S820,
(28) D8S1179/D7S820,
(29) D16S539/TPOX,
(30) D16S539/FGA,
(31) CSF1PO/Penta_D,
(32) CSF1PO/TPOX,
(33) vWA/Penta_D,
(34) AMEL/TPOX,
(35) AMEL/FGA,
(36) vWA/D8S1179,
(37) vWA/TPOX,
(38) vWA/FGA,
(39) D8S1179/TPOX, and
(40) D8S1179/FGA.

In a particular embodiment, at least one of the genomic loci is TPOX (SEQ ID NO. 6) or FGA (SEQ ID NO. 9).

In another embodiment, the amplified loci are 90 bps or smaller in size.

In one embodiment, signal intensity is the amplification product's fluorescence level measured during capillary electrophoresis.

In one embodiment, steps (B) and (C) of the method disclosed above are performed by real-time PCR. That is, in one embodiment, all of the required reagents and primers and enzymes necessary for DNA digestion and amplification by PCR are present together in the same tube or vessel.

In one embodiment, fluorescently-labeled primers are used to PCR amplify the chosen loci of the digested DNA, wherein the signal intensity of each amplification product is the fluorescence level of each amplification product.

In another embodiment, the method further comprises hybridizing a fluorescently-labeled probe to an amplification product, wherein the signal intensity of the amplification product is the fluorescence level measured after hybridization of the probe to the product.

In another embodiment, the method further comprises assigning confidence levels to potential categories of a DNA sample, wherein the confidence level reflects the likelihood that a particular DNA category indicates the categorical source of the DNA sample; wherein the likelihood is calculated by:

(A) assigning a probability score to each comparison of a signal ratio to the reference values that correspond to the various categories of DNA, wherein the probability score is obtained by (i) assigning a gamma probability distribution function to the reference values of each potential category; wherein (ii) the probability score is equal to the value of the gamma probability distribution function assigned in step (A) at the observed signal ratio;

(B) for each category, calculating the category likelihood score, which is the product of the probability scores obtained in step (A); and

(C) normalizing likelihood scores by dividing the category likelihood score of each potential category by the sum of all category likelihood scores;

wherein the confidence level of each category is the normalized likelihood score of that category.

In another embodiment, the method further comprises simultaneously performing DNA profiling with the DNA categorization.

In one embodiment, the steps of DNA digestion and polymerase chain reaction are performed together in a single tube.

In one embodiment, the genomic loci that are amplified are chosen to produce distinct signal ratios for different potential categories.

Also disclosed is a kit for categorization of a DNA sample into at least two predetermined categories and for obtaining an associated categorization confidence level, comprising (1) primers for amplification of specific genomic loci; and at least one or more reagents selected from the group consisting of (2) a reaction buffer, (3) control DNA, (4) a methylation sensitive restriction endonuclease and/or a methylation dependent restriction endonuclease, (5) a written protocol for performing categorization. In one embodiment of the present invention, the written protocol may comprise instructions for performing any of the methods disclosed herein, including but not limited to, DNA digestion parameters, PCR cycling parameters, signal ratio analysis, and categorization analysis, as well as DNA profiling methods.

In another embodiment, the kit further comprises analysis software for performing categorization analyses.

Another aspect of the present invention is a kit for categorizing a DNA sample as semen or non-semen and for obtaining an associated categorization confidence level, comprising:

(a) primer mix, comprising the following primers:

SD1f (AAGAGCCCATCAGGCAGGTC);

SD1r (GTTTCTTGTCGAGCAGCACGTGGATGATG);

SD2f (CTCCAGAACTGGAACTTCCTG);

SD2r (GTTTCTTAACTTGGAGACGACGGCATC);

SD3f (TGGAGGACAATGCCCTGGTG);

SD3r (GTTTCTTGGCTTCACCTGCGACCGTCTC);

SD4f (CCCTCCGAGTGGCCAGCAG);

SD4r (GTTTCTGACCACTGCCGTGGGAATG);

SD5f (CTTCTCAGCCAATGGGAAGAG);

SD5r (ACGTAGAAGGACCCGAGGAC);

SD6f (TACAGACAAATCACTCAGCAGC); and

SD6r (GTTTCTTGTCTGACACTCGGTTGTAGGTATT);

(b) reaction buffer;

(c) HhaI restriction endonuclease;

(d) DNA polymerase

(d) a written protocol for performing categorization.

In another embodiment, the kit further comprises control DNA samples.

In another aspect of the present invention is a kit for categorizing a DNA sample as semen or non-semen and for obtaining an associated categorization confidence level, comprising at least one pair of forward (f) and reverse (r) primer pair combinations selected from the group consisting of:

(1) SD1f (AAGAGCCCATCAGGCAGGTC) and SD 1 r (GTTTCTTGTCGAGCAGCACGTGGATGATG);

(2) SD2f (CTCCAGAACTGGAACTTCCTG) and SD2r (GTTTCTTAACTTGGAGACGACGGCATC);

(3) SD3f (TGGAGGACAATGCCCTGGTG) and SD3r (GTTTCTTGGCTTCACCTGCGACCGTCTC);

(4) SD4f (CCCTCCGAGTGGCCAGCAG) and SD4r (GTTTCTGACCACTGCCGTGGGAATG);

(5) SD5f (CTTCTCAGCCAATGGGAAGAG) and SD5r (ACGTAGAAGGACCCGAGGAC);

(6) SD6f (TACAGACAAATCACTCAGCAGC) and SD6r (GTTTCTTGTCTGACACTCGGTTGTAGGTATT).

In one embodiment, the concentration of the primers in the primer mix are: 0.6μM SD1f, 0.6μM SD1r, 1.75μM SD2f, 1.75μM SD2r, 1.25μM SD3f, 1.25μM SD3r, 1.75μM SD4f, 1.75μM SD4r, 1.75μM SD5f, 1.75μM·SD5r, 0.9μM SD6f, and 0.9μM SD6r.

In one embodiment, the reaction buffer comprises 150mM TRIS-HCl, 15mM $MgCl_2$, 0.2mM each dntp, and 0.1μg/μl BSA.

In one embodiment, the kit further comprises a DNA ladder.

In one embodiment, the kit further comprises analysis software for performing categorization analyses.

Also disclosed is a kit for profiling, categorizing a DNA sample as semen or non-semen, and obtaining an associated categorization confidence level, comprising:

(a) primers for amplifying at least one semen-specific locus;
(b) primers for amplifying at least one locus used for DNA profiling;
(c) reaction buffer;
(d) HhaI restriction endonuclease,
(e) DNA polymerase
(f) a written protocol for performing categorization.

In one embodiment, the kit further comprises control DNA samples.

Another aspect of the present invention is a kit for profiling, categorizing a DNA sample as semen or non-semen, and obtaining an associated categorization confidence level, comprising:

(a) primers for amplifying at least one semen-specific locus; and

(b) primers for amplifying at least one locus used for DNA profiling;

at least one semen-specific locus amplified by a kit disclosed herein is the L68346 locus and wherein the primers for amplifying a 70 bp semen-specific amplification product from L68346 are a forward primer comprising the sequence of CAGCAACAGCACCCAGCTTG (FAM) and a reverse primer comprising the sequence of CACAGGCTCAGTCGCGGATC; or,

at least one semen-specific locus amplified by a kit disclosed herein is the L16264 locus and wherein the primers for amplifying a 95 bp semen-specific amplification product from L16264 are a forward primer comprising the sequence of GGACGAGTTAACTTCCTTAATTTC (FAM) and reverse primer comprising the sequence of GTTTCTTCGCGGAACCTGGTTTAACTTC.

In another embodiment, the reaction buffer comprises 150mM TRIS-HCl, 15mM $MgCl_2$, 0.2mM each dntp, and 0.1μg/μl BSA.

In another embodiment, the kit further comprises at least one of (a) a DNA ladder, (b) a Material Safety Data Sheet (MSDS), and (c) analysis software for performing categorization analyses.

Another aspect of the present invention is a kit for categorizing a DNA sample as blood, saliva, semen, or skin epidermis, and for obtaining an associated categorization confidence level, comprising:

(a) primer mix that comprises forward and reverse primers for amplifying the denoted loci as follows:

1. L91762 (forward GCAGCAGGCCGCGGAGAAG (FAM); reverse AGCAGCTGTGCCGGGCCAG)
2. L68346 (forward CAGCAACAGCACCCAGCTTG (JOE); reverse CACAGGCTCAGTCGCGGATC)
3. L50468 (forward AGGAAACCTCAGTAGCAAAATTG (JOE); reverse GCGAGACTTTAGGTGTGCATC)
4. L14432 (forward CGTAGGCTGCGGTGAGCTC (FAM); reverse GATCCATGCCCGCTGGGATG)
5. L4648 (forward CAGCCTAGACGTCAAGTTACAG (JOE); reverse ACGACCTCCGGATCCAACTG)
6. L39664 (forward CCCAGCTGGTTGGACATGTTG (FAM); reverse CACTTCCTTCGTGGACGCC)
7. L30139 (forward GAGAAGCGGGAGGATGAGAC (FAM); reverse CCGCATCTCCTCCGTCCTG)
8. L55429 (forward GCCTTCAGCAGGAAGTCCAC (JOE); reverse CCTGTGCCTCACACAGACTC)
9. L62086 (forward GTGCATGGTGTCTGGTACTTC (FAM); reverse GAAGCTCTCGTGGACTACTTG)
10. L76138 (forward CAGCCTGCTCTTCACTGCAG (JOE); reverse AGAGGCCGATGAAGCCGTAG)
11. L15952 (forward CTCCCTGATTTACGACAAGTTC (FAM); reverse GACAGTATGCTGATGCTTCTTG)
12. L36599 (forward AAGGGCAGAGTTCCGCTGTC (FAM); reverse CGGATGCAGGAGGATCCTAG)
13. L26688 (forward CGGACCAGATTGCTGGTCAC (JOE); reverse CGACCTTGCCAGATGTTTGAC)
14. L81528 (forward AGCCTCATCCACACTGACCAG (JOE); reverse TCAGAGCTCTCCTATCTGGAC)
15. L36556 (forward GCCAGGCCGTTGATGATGAC (JOE); reverse GAATATGGAGCCCTGGGCAG)

(b) reaction buffer;
(c) HhaI restriction endonuclease;
(d) DNA polymerase
(e) a written protocol for performing categorization.

In another embodiment, the kit further comprises control DNA samples.

In one embodiment, the reaction buffer comprises 150mM TRIS-HCl, 15mM $MgCl_2$, 0.2mM each dntp, and 0.1μg/μl BSA.

In another embodiment, the kit further comprises at least one of (a) a DNA ladder, (b) a Material Safety Data Sheet (MSDS), and (c) analysis software for performing categorization analyses.

In one embodiment in any of the methods disclosed herein, the DNA sample comprises a mixture of DNA samples.

Also disclosed is a method for calculating a distance measure of differential methylation between DNA samples, comprising:

(A) digesting each DNA sample with a methylation-sensitive and/or methylation-dependent restriction endonuclease;
(B) performing PCR on each digested DNA amplifying at least two genomic loci of which at least one is a restriction locus;
(C) for each DNA sample, determining the intensity of the signal of each amplification product;

(D) for each DNA sample, calculating "signal ratios" between the intensities of the signals produced by the loci;
(E) calculating the differential methylation measure between DNA samples by performing a quantitative comparison of their corresponding signal ratios.

In one embodiment of this method, the distance measure is sum of absolute differences between the signal ratios of the DNA samples.

In another embodiment of this method, the distance measure is the square-root of the sum of squared differences between the signal ratios of the DNA samples.

In one embodiment of this method, the distance measured between a subject sample and a reference healthy sample indicates the amount of medication required to treat a pathological state.

In another embodiment of this method, the distance measured between a DNA sample obtained from cultured cells to a reference sample indicates the number of sub-culturing procedures that the cells underwent.

Also disclosed is a primer selected from the group consisting of:

1. GCAGCAGGCCGCGGAGAAG;
2. AGCAGCTGTGCCGGGCCAG;
3. CAGCAACAGCACCCAGCTTG;
4. CACAGGCTCAGTCGCGGATC;
5. AGGAAACCTCAGTAGCAAAATTG;
6. GCGAGACTTTAGGTGTGCATC;
7. CGTAGGCTGCGGTGAGCTC;
8. GATCCATGCCCGCTGGGATG;
9. CAGCCTAGACGTCAAGTTACAG;
10. ACGACCTCCGGATCCAACTG;
11. CCCAGCTGGTTGGACATGTTG;
12. CACTTCCTTCGTGGACGCC;
13. GAGAAGCGGGAGGATGAGAC;
14. CCGCATCTCCTCCGTCCTG;
15. GCCTTCAGCAGGAAGTCCAC;
16. CCTGTGCCTCACACAGACTC;
17. GTGCATGGTGTCTGGTACTTC;
18. GAAGCTCTCGTGGACTACTTG;
19. CAGCCTGCTCTTCACTGCAG;
20. AGAGGCCGATGAAGCCGTAG;
21. CTCCCTGATTTACGACAAGTTC;
22. GACAGTATGCTGATGCTTCTTG;
23. AAGGGCAGAGTTCCGCTGTC;
24. CGGATGCAGGAGGATCCTAG;
25. CGGACCAGATTGCTGGTCAC;
26. CGACCTTGCCAGATGTTTGAC;
27. AGCCTCATCCACACTGACCAG;
28. TCAGAGCTCTCCTATCTGGAC;
29. GCCAGGCCGTTGATGATGAC; and
30. GAATATGGAGCCCTGGGCAG
31. AAGAGCCCATCAGGCAGGTC
32. GTTTCTTGTCGAGCAGCACGTGGATGATG
33. CTCCAGAACTGGAACTTCCTG
34. GTTTCTTAACTTGGAGACGACGGCATC
35. TGGAGGACAATGCCCTGGTG
36. GTTTCTTGGCTTCACCTGCGACCGTCTC
37. CCCTCCGAGTGGCCAGCAG
38. GTTTCTGACCACTGCCGTGGGAATG
39. CTTCTCAGCCAATGGGAAGAG
40. ACGTAGAAGGACCCGAGGAC
41. TACAGACAAATCACTCAGCAGC
42. GTTTCTTGTCTGACACTCGGTTGTAGGTATT
43. GGACGAGTTAACTTCCTTAATTTC
44. GTTTCTTCGCGGAACCTGGTTTAACTTC

Also disclosed is a primer pair for amplifying a specific human genomic locus selected from the group consisting of:

1. L91762 (forward GCAGCAGGCCGCGGAGAAG (FAM); reverse AGCAGCTGTGCCGGGCCAG);
2. L68346 (forward CAGCAACAGCACCCAGCTTG (JOE); reverse CACAGGCTCAGTCGCGGATC);
3. L50468 (forward AGGAAACCTCAGTAGCAAAATTG (JOE); reverse GCGAGACTTTAGGTGTGCATC);
4. L14432 (forward CGTAGGCTGCGGTGAGCTC (FAM); reverse GATCCATGCCCGCTGGGATG);
5. L4648 (forward CAGCCTAGACGTCAAGTTACAG (JOE); reverse ACGACCTCCGGATCCAACTG);
6. L39664 (forward CCCAGCTGGTTGGACATGTTG (FAM); reverse CACTTCCTTCGTGGACGCC);
7. L30139 (forward GAGAAGCGGGAGGATGAGAC (FAM); reverse CCGCATCTCCTCCGTCCTG);
8. L55429 (forward GCCTTCAGCAGGAAGTCCAC (JOE); reverse CCTGTGCCTCACACAGACTC);
9. L62086 (forward GTGCATGGTGTCTGGTACTTC (FAM); reverse GAAGCTCTCGTGGACTACTTG);
10. L76138 (forward CAGCCTGCTCTTCACTGCAG (JOE); reverse AGAGGCCGATGAAGCCGTAG);
11. L15952 (forward CTCCCTGATTTACGACAAGTTC (FAM); reverse GACAGTATGCTGATGCTTCTTG);
12. L36599 (forward AAGGGCAGAGTTCCGCTGTC (FAM); reverse CGGATGCAGGAGGATCCTAG);
13. L26688 (forward CGGACCAGATTGCTGGTCAC (JOE); reverse CGACCTTGCCAGATGTTTGAC);
14. L81528 (forward AGCCTCATCCACACTGACCAG (JOE); reverse TCAGAGCTCTCCTATCTGGAC);
15. L36556 (forward GCCAGGCCGTTGATGATGAC (JOE); reverse GAATATGGAGCCCTGGGCAG).
16. SD1 (forward AAGAGCCCATCAGGCAGGTC (FAM); reverse GTTTCTTGTCGAGCAGCACGTGGATGATG);
17. SD2 (forward CTCCAGAACTGGAACTTCCTG (FAM); reverse GTTTCTTAACTTGGAGACGACGGCATC);
18. SD3 (forward TGGAGGACAATGCCCTGGTG (FAM); reverse GTTTCTTGGCTTCACCTGCGACCGTCTC);
19. SD4 (forward CCCTCCGAGTGGCCAGCAG (FAM); reverse GTTTCTGACCACTGCCGTGGGAATG);
20. SD5 (forward CTTCTCAGCCAATGGGAAGAG (FAM); reverse ACGTAGAAGGACCCGAGGAC);
21. SD6 (forward TACAGACAAATCACTCAGCAGC (FAM); reverse GTTTCTTGTCTGACACTCGGTTGTAGGTATT); and
22. L16264 (forward GGACGAGTTAACTTCCTTAATTTC (FAM); reverse GTTTCTTCGCGGAACCTGGTTTAACTTC).

## BRIEF DESCRIPTION OF THE DRAWINGS

**Figures 1A-D.** Tissue identification assay. (A) Schematic overview of the assay. (B) Biochemical procedure - methylated loci remain intact during digestion and subsequently are amplified efficiently in the PCR, producing a strong signal (locus A) while unmethylated loci are digested and subsequently amplify inefficiently in the PCR, producing a weak signal (Locus B). The signal ratio (SR; rfu of locus A/rfu of locus B) reflects the differential methylation level between loci A and B. (C) Signal ratios between locus 1 and locus 2 are different in blood, saliva, skin, and semen, reflecting the differential methylation patterns in these tissues. (D) The observed differences in signal ratios between blood and semen (i.e. the signal) are more than a magnitude greater than the observed differences in signal ratios obtained from different PCRs and from different amounts of input DNA (i.e. the noise).

**Figure 2.** Tissue identification of different tissue types. Electropherograms of 8 samples (2 samples from each of 4 different tissues) are depicted demonstrating the distinct amplification pattern observed for each tissue type.

**Figures 3A-D.** Combined semen detection and DNA profiling. (A,B) Electropherograms of the combined assay performed on a semen (A) and urine (B) sample from the same individual. The ProfilerPlus profiles of the samples are identical, and the differences in tissue source are indicated by the differential amplification of two semen detection loci (arrows). (C) FAM channel data of the combined assay performed on samples from different tissue types. The signal ratio between the semen detection loci (arrows) is 25.04 in the semen sample, and less than 0.1 in all other tissue types. The top panel includes undigested DNA for reference. (D) FAM channel data of the combined assay performed on mixtures of semen and saliva (from different individuals) with various ratios. The signal ratio is correlated to the percentage of semen in the mixture.

**Figure 4.** Assessment of HhaI digestion. DNA samples of different sources were subjected to the same single digestion-amplification reaction used in the tissue identification experiments. In this experiment, the panel of loci consisted of two loci: an undigested control locus (L98328) that lacks an HhaI site and a digested control locus (SW14) thath is unmethylated in these tissues. All samples showed presence of the undigested control locus and complete absence of the digested control locus, indicating complete digestion by HhaI in contrast to the undigested sample which showed presence of both loci.

**Figure 5.** Aged samples. The stand-alone tissue identification assay was tested on DNA samples extracted from 20-month old stains of blood and semen. Both samples amplified well, similar to their fresh counterparts, and their source was correctly identified by the analysis software.

**Figure 6.** Simulation of degraded samples. Blood (top) and semen (bottom) samples were partially digested using DNAseI and analyzed with the combined semen detection and profiling assay. Although only partial profiles were obtained for both samples, the smaller tissue identification loci (the two loci in the top channel to the left of D3S1358) amplified successfully with the expected pattern, and the algorithm correctly identified the presence/absence of semen in these samples.

**Figure 7.** Table 1.

**Figure 8.** Table 2.

**Figure 9.** Table 3.

**Figure 10.** Table 4.

**Figure 11.** Table 5.

**Figure 12.** Stand alone semen detection assay. Sample plots of 6 DNA samples from different tissue types are depicted. Correct categorization (semen vs. non-semen) was achieved for all samples. For each sample the confidence level of the least likely category is shown (e.g. for a "semen" categorization, the p value of non-semen is shown)

## DETAILED DESCRIPTION

### 1. Introduction

The present invention relates to methods and assays that enable distinguishing between sources of DNA using loci-specific primers and commercially available enzymes. An underlying aspect of an inventive assay is the comparison of signals from at least two loci amplified from a particular source of DNA, which ultimately yields a numerical ratio that indicates whether that source of the DNA sample is, in one embodiment, natural or artificial. The inventive assays also can be used to distinguish between, for example, different physiological and pathological sources of DNA and between different tissues.

The signal ratios employed by the assay are correlated to methylation levels at specific genomic loci, but do not indicate actual methylation levels at any genomic locus. Therefore the invention setup eliminates the requirement for external DNA species and/or controls, thereby both simplifying and increasing the accuracy of the assay.

The inventive DNA categorization assays described herein are therefore powerful, multiplex, accurate, and inexpensive techniques applicable in any setting that calls for the categorization of a DNA sample. Thus, the inventive assays can be used, for example, by the police in a forensics capacity; the health care industry for diagnostic and therapeutic purposes; in the insurance industry to verify claims pursuant to anti-discrimination genetic laws, such as the Genetic Information Nondiscrimination Act (H.R. 493); by prosecutors and defense counsel for evidentiary purposes in criminal trials and civil proceedings and appeals; and the food and agriculture industry to verify the integrity of meats, crops, and plants such as grapevines and sources of coffee. The present invention is not limited to this non-exclusive, but representative, list of applications.

An underlying principle of a method disclosed herein therefore is the measurement of signal intensities between amplified genomic loci and their subsequent pairwise comparison against each other to produce ratios of signal intensities between the amplified loci. For example, if loci A, B, C, and D are all amplified according to the techniques described herein, then the signal intensity for each of A, B, C, and D is measured and recorded. Then, the signal intensities of A and B, A and C, and A and D are compared respectively and signal intensity ratios calculated for each of the A/B, A/C and A/D combinations. Then, the signal intensity ratios of B/C and B/D are calculated, and so on.

The value of each of those signal intensity ratios can then be compared against the values of known reference signal intensity ratios of DNAs whose source of origin is known. Thus, for instance, in the case of DNA whose source of origin is known to be human semen, the signal intensity ratios of loci, such as the pairwise ratios of the A, B, C, and D illustrative loci are known, or can be readily established afresh when analysing a test DNA sample. Thus the DNA category is "semen" and its respective signal intensity ratios for those loci are reference values against which the test DNA sample may be compared.

Accordingly, after establishing the various signal intensity ratios of loci A, B, C, and D, from the DNA sample, each ratio can be compared against one or more reference values of particular known categories of DNA, e.g., against the semen DNA ratio values. The ratio of any loci combination from the DNA sample that more closely approximates the signal intensity ratio reference value of a particular DNA category means it is highly likely that the tested DNA sample originated from that particular category of DNA to which it more or most closely approximates when compared. Thus, a signal intensity ratio from the tested DNA sample of the A/B loci that is the same as, or close to, the value of the signal intensity ratio of the A/B loci from semen indicates that the tested DNA sample likely originated from semen. By conducting the same comparative analysis across many different loci, and against many different reference categories, the likelihood that the tested DNA sample originated from a particular biological source, or that it did not originate from a particular biological source, increases.

Similarly, in a situation where a sample might comprise mixtures of DNA from different sources, such as saliva and blood, or saliva, blood, epidermis, and semen, it will be possible, by performing the methods disclosed herein, to identify the presence of different categories of DNAs within the sample. Thus, the identification of multiple as well as sole sources of DNA present in a sample can be achieved using the methods and reagents disclosed herein.

Accordingly, the present "categorization" methods can also be thought of as a tissue identification assay. In one example, as explained in the following passage, a tissue identification assay uses a panel of loci that are differentially methylated between tissues to determine the most probable source tissue of a DNA sample. See Table 1 (Figure 7). A scheme of the assay is presented in Figure 1A. DNA from a forensic sample is digested with, for example, the HhaI methylation-sensitive restriction enzyme, which cleaves DNA at its recognition sequence GCGC only if it is unmethylated (while leaving methylated targets intact).

A panel of tissue identification loci is then amplified by PCR from the digested DNA using fluorescently-labeled primers, and an aliquot of amplified products is separated by capillary electrophoresis. In the situation with Hha1-digested DNA, loci with higher methylation levels are amplified with higher efficiency because more DNA molecules are protected from digestion, producing a relatively strong signal in the electropherogram (Figure 1B, locus A). Conversely, loci with a lower methylation level are amplified with lower efficiency, yielding a relatively weak signal in the electropherogram (Figure 1B, locus B).

Automated signal analysis software disclosed herein enables analysis of an output electropherogram, and assigns heights (in rfu) to amplicons corresponding to tissue identification loci. Thus, the height of a single locus is correlated with its methylation level. Ratios of methylation levels between co-amplified loci can then be calculated as described generically above. For each pair of loci a signal ratio, calculated as the ratio between the heights of the first and second loci, and this ratio reflects the ratio between the methylation levels of the corresponding loci.

All calculated signal ratios can then be combined into a single numerical value. For example, 105 pairwise ratios can be calculated from a panel of 15 loci (*e.g.,* as between locus 1 and 2, locus 1 and 3, etc.). Those can then be compared to a database of reference values obtained from a data set of samples of known tissue origin. The tissue identification algorithm calculates for each potential tissue source a likelihood score, reflecting the likelihood that the DNA sample originated from that tissue, and the output of the algorithm is the most likely tissue. Disclosed elsewhere herein are algorithms and specific calculations for deriving likelihood and probability scores from these ratios and comparisons.

As shown in Example 1, for instance, such a tissue identification assay was performed on 50 samples: 14 blood, 14 saliva, 11 semen, and 11 skin epidermis using a panel of 15 tissue identification loci, ranging in amplicon size from 66 to 150 bps. Figure 2 depicts electropherograms of eight of these samples - two of each tissue type. Each tissue type had a distinct methylation profile. For example, the ratio of the L91762/L68346 loci in semen samples ranged from 0.04-0.53 and was higher in all other tissue samples (2.15-18.28; see Table 2 in Figure 8). Therefore a *low* L91762/L68346 ratio was found to be distinctive of semen samples. The ratio of the L76138/L26688 loci, however, was low in blood and saliva (0.08-1.54) and *higher* in semen and skin epidermis (2.04-19.56; Table 2 in Figure 8). Therefore a high ratio of L91762/L68346 concomitant with a high ratio of L76138/L26688 was distinctive of skin epidermis samples

The tissue identification algorithm disclosed herein correctly identified the true tissue source of all 50 samples. Further analysis of the data using subsets of tissue identification loci revealed that the full set of 15 loci was redundant and 100% identification was achieved by using only 7 out of the 15 loci (L91762, L68346, L50468, L14432, L30139, L15952, and L26688).

Accordingly, this illustrative example corroborates the generic inventive aspect of the present invention, namely that the comparative signal intensity ratio analyses provides exacting and correct DNA sample identification and categorization.

Furthermore, another significant aspect of the present invention is that it can readily complement and expand the usefulness of existing commercial DNA profiling kits to do more than profile a particular subject's DNA. The combination of the inventive assays disclosed herein, such as the signal ratio assay described in detail below, with Promega Corporation's PowerPlex® 16 kit, for example, enables a user to not only profile an individual's DNA composition but also to determine whether the profiled DNA is from a natural source or has been artificially synthesized. In another example, combining DNA profiling with DNA categorization enables a user to both profile DNA and determine the source tissue of the DNA sample.

For instance, one assay described herein may employ *both* conventional profiling of a DNA sample, for example with the PowerPlex16 kit (Promega), *and* categorization of DNA into categories of natural vs. artificial. See, for instance, Example 6 below.

For illustrative purposes only of how combined categorization and profiling may work together as disclosed herein, two samples of DNA are analyzed: (1) a natural DNA sample extracted from the blood of subject A, and (2) an artificial DNA sample synthesized by in vitro multiple displacement amplification using a commercial kit from a minute quantity of subject A's real DNA as template.

Furthermore, this illustration demonstrates how the present inventive method is far superior to any existing method because it is able to distinguish artificial DNA from natural DNA samples *even though* conventional profiling kits would identify both samples as identical to one another - because both the natural and artificial DNA are from subject A. Accordingly, notwithstanding the fact that the profiling kit would identify the samples as the same, the present inventive signal intensity ratio analyses can readily tell them apart and identify one profile as generated from artificial DNA, while the other profile was generated from natural DNA.

To explain further, the two samples are first profiled with PowerPlex16 and analyzed using a conventional profiling software, such as GeneMapperID-X. The scheme of this procedure can be briefly described as (1) performing multiplex PCR using the PowerPlex16 primer mix on a DNA sample; (2) separating the amplification products on a capillary electrophoresis machine; and then (3) analyzing the output data with, for instance, the GeneMapperID-X software. For each of the samples, the end product of this assay is a profile. The profiles of the two samples are identical. See Figures 9A-B.

Furthermore, the GeneMapperID-X software determined the profiles of both samples to be single contributor (i.e. from a single person) profiles, with no anomalies. This shows that a profile obtained from artificial DNA can be identical to a profile obtained from natural DNA. See D. Frumkin, et al., Authentication of forensic DNA samples, Forensic Sci. Int. Genet. (2009), doi:10.1016/j.fsigen.2009.06.009, which is incorporated herein by reference.

Both samples of DNA were subsequently also analyzed by a combined profiling and categorization assay based on the following scheme: digest a DNA sample with HhaI and perform multiplex PCR using the PowerPlex16 primer mix with the addition of primers for amplifying an additional locus, such as Hypo23; separating the amplification products on a capillary electrophoresis machine; and analyzing the output data with the capillary analyzer software, such as the software algorithm disclosed herein.

For each sample, the end product of this assay is a profile and assignment of category. As can be seen in Figure 9C and D, the natural DNA was assigned as "natural," in contrast to the artificial sample which was assigned as "artificial."

In this particular example, the assignment of category was performed by analysis of three parameters: (1) SR1 (signal ratio of TPOX/D8S1179), (2) SR2 (signal ratio of D3S1358/Hypo23), and (3) RR (representation ratio of OCA2/D3S1358).

The threshold values for these parameters are provided in Table 6:

**Table 6**

| | SR1 | SR2 | RR |
|---|---|---|---|
| Threshold for natural | >= 0.9 | >= 10 | >= 2 |
| Threshold for artificial | < 0.9 | < 10 | < 2 |

The observed values for these parameters, and the assigned category are provided in Table 7 (values that are above the threshold for natural DNA are shaded).

**Table 7**

| | SR1 | SR2 | RR | Category |
|---|---|---|---|---|
| Natural DNA sample | 1.23 | 17.54 | 3.31 | Natural |
| Artificial DNA sample | 0.06 | 2.06 | 2.47 | Artificial |

Assignment of the category was performed according to the following rule:

If all observed parameter values are above their respective thresholds for natural DNA, then assign a "natural" category, otherwise assign an "artificial" category. Thus, the present inventive signal intensity ratio analyses can readily identify sources of DNA, such as for example from natural vs. artificial sources, and in conjunction with DNA profiling techniques provides a powerful and sophisticated method for identifying genomic materials.

Specific compositions, methods, or embodiments discussed are intended to be only illustrative of the invention disclosed by this specification. Variations on these compositions, methods, or embodiments are readily apparent to a person of skill in the art based upon the teachings of this specification and are therefore intended to be included as part of the inventions disclosed herein.

In practicing the present invention, many conventional techniques in molecular biology and recombinant DNA are used. These techniques are explained in, *e.g.,* Current Protocols in Molecular Biology, Vols. I-III, Ausubel, Ed. (1997); Sambrook et al., Molecular Cloning: A Laboratory Manual, Second Ed. (Cold Spring Harbor Laboratory Press, Cold Spring Harbor, NY, 1989); DNA Cloning: A Practical Approach, Vols. I and II, Glover, Ed. (1985); Oligonucleotide Synthesis, Gait, Ed. (1984); Nucleic Acid Hybridization, Hames & Higgins, Eds. (1985); Transcription and Translation, Hames & Higgins, Eds. (1984); Perbal, A Practical Guide to Molecular Cloning; the series, Meth. Enzymol., (Academic Press, Inc., 1984); Gene Transfer Vectors for Mammalian Cells, Miller & Calos, Eds. (Cold Spring Harbor Laboratory, NY, 1987); and Meth. Enzymol., Vols. 154 and 155, Wu & Grossman, and Wu, Eds., respectively.

## 2. Definitions

The present technology is described herein using several definitions, as set forth throughout the specification. Unless defined otherwise, all technical and scientific terms used herein generally have the same meaning as commonly understood by one of ordinary skill in the art to which this invention belongs. As used herein, unless otherwise stated, the singular forms "a," "an," and "the" include plural reference. Thus, for example, a reference to "a nucleic acid" is a reference to one or more nucleic acids.

As used herein, the term "allele" is intended to be a genetic variation associated with a segment of DNA, *i.e.,* one of two or more alternate forms of a DNA sequence occupying the same locus.

The term "artificial DNA" or "artificially-synthesized DNA" or "artificial nucleic acid" as used herein refers to a nucleic acid which is synthesized by *various in vitro* methods. Herein disclosed are the characterizations of different methods for synthesizing DNA *in vitro. Such in vitro* generated nucleic acids include, but are not limited to:

1. Chemically synthesized oligonucleotides.

2. Products of PCR amplification of target sequences.

3. Products of Rolling circle amplification (RCA) of circular target sequences.

4. Products of molecular cloning (e.g. plasmids cloned in *E. coli*.)

5. DNA fragments assembled from other DNA fragments that were generated by any of methods 1-4, or a combination of them. Such assembly being achieved by any of the following methods (or a combination of them): annealing, ligation, polymerization. The process of assembly may also include steps of breaking DNA molecules (e.g. by restriction endonucleases, mechanical shearing etc.)

6. Products of PCR-based Whole genome amplification (WGA), and/or ligation mediated PCR (LMP)-based WGA methods, including primer extension preamplification (PEP)-PCR, degenerate oligonucleotide primed (DOP)-PCR, T7-based linear amplification of DNA (TLAD), Adaptor-Ligation PCR. The Genomeplex (Sigma) commercial kit utilizes Adaptor-Ligation PCR.

7. Products of WGA by Multiple displacement amplification (MDA) and Restriction and Circularization-Aided Rolling Circle Amplification (RCA-RCA). The Repli-G (Qiagen), and GenomiPhi (GE Healthcare) commercial kits utilize this method.

8. A mix of products from any of 1-7.

9. Products from any of 1-8 in which all or some products were methylated *in vitro* following their synthesis (e.g. by Sss1 Methylase).

10. Products from any of 1-8 mixed with natural DNA.

11. Products from 9 mixed with natural DNA.

The term "biological sample" or "test sample" as used herein, refers to, but is not limited to, any biological sample derived from, or obtained from, a subject. The sample may comprise nucleic acids, such as DNAs or RNAs. In some embodiments, samples are not directly retrieved from the subject, but are collected from the environment, *e.g.* a crime scene or a rape victim. Examples of such samples include but are not limited to fluids, tissues, cell samples, organs, biopsies, *etc.* Suitable samples include but are not limited to are blood, plasma, saliva, urine, sperm, hair, *etc.* The biological sample can also be blood drops, dried blood stains, dried saliva stains, dried underwear stains (*e.g.* stains on underwear, pads, tampons, diapers), clothing, dental floss, ear wax, electric razor clippings, gum, hair, licked envelope, nails, paraffin embedded tissue, post mortem tissue, razors, teeth, toothbrush, toothpick, dried umbilical cord. Genomic DNA can be extracted from such samples according to methods known in the art. (for example using a protocol from Sambrook et al., Molecular Cloning: A Laboratory Manual, Second Ed., Cold Spring Harbor Laboratory Press, Cold Spring Harbor, NY, 1989);
The term "capillary electrophoresis histogram" as used herein refers to a histogram obtained from capillary electrophoresis of PCR products wherein the products were amplified from genomic loci with fluorescent primers.

The term "methylated" as used herein means methylated at a level of at least 80% (i.e. at least 80% of the DNA molecules methylated) in DNA of cells of tissues including blood, saliva, semen, epidermis, nasal discharge, buccal cells, hair, nail clippings, menstrual excretion, vaginal cells, urine, and feces.

The term "partially-methylated" as used herein means methylated at a level between 20-80% (i.e. between 20-80% of the DNA molecules methylated) in DNA of cells of tissues including blood, saliva, semen, epidermis, nasal discharge, buccal cells, hair, nail clippings, menstrual excretion, vaginal cells, urine, and feces.

The term "unmethylated" as used herein means methylated at a level less than 20% (i.e. less than 20% of the DNA molecules methylated) in DNA of cells of tissues including blood, saliva, semen, epidermis, nasal discharge, buccal cells, hair, nail clippings, menstrual excretion, vaginal cells, urine, bone, and feces. The methods provided herein have been demonstrated to distinguish methylated and unmethylated forms of nucleic acid loci in various tissues and cell types including blood, saliva, semen, epidermis, nasal discharge, buccal cells, hair, nail clippings, menstrual excretion, vaginal cells, urine, bone, and feces.

The terms "determining," "measuring," "assessing," "assaying", and "evaluating" are used interchangeably to refer to any form of quantitative or qualitative measurement, and include determining if a characteristic, trait, or feature is present or not. Assessing may be relative or absolute. "Assessing the presence of" includes determining the amount of something present, as well as determining whether it is present or absent.

The term "forensics" or "forensic science" as used herein refers to the application of a broad spectrum of methods aimed to answer questions of identity being of interest to the legal system. For example, the identification of potential suspects whose DNA may match evidence left at crime scenes, the exoneration of persons wrongly accused of crimes, identification of crime and catastrophe victims, or establishment of paternity and other family relationships.

The term "locus" (plural - loci) refers to a position on a chromosome of a gene or other genetic element. Locus may also mean the DNA at that position. A variant of the DNA sequence at a given locus is called an allele. Alleles of a locus are located at identical sites on homologous chromosomes. A restriction locus is a locus that contains the recognition sequence of the restriction enzyme used in the assay. The term digested control locus as used herein refers to a genomic locus known to be unmethylated in all potential DNA categories. The term undigested control locus as used herein refers to a genomic locus that is either known to be methylated in all potential DNA categories, or is a genomic locus lacking the recognition sequence of the endonuclease used in the assay.

The term "natural DNA" or "natural nucleic acid" as used herein refers to, but is not limited to, nucleic acid which originates directly from the cells of a subject without modification or amplification.

The term "nucleic acid" as used herein refers to, but is not limited to, genomic DNA, cDNA, hnRNA, mRNA, rRNA, tRNA, fragmented nucleic acid, and nucleic acid obtained from subcellular organelles such as mitochondria. In addition, nucleic acids include, but are not limited to, synthetic nucleic acids or *in vitro* transcription products.

The term "nucleic-acid based analysis procedures" as used herein refers to any identification procedure which is based on the analysis of nucleic acids, *e.g.* DNA profiling.

The term "polymerase chain reaction (PCR) stutter" as used herein refers to PCR byproducts, obtained along with the main PCR product. These "stutter" byproducts are usually shorter by multiples of the repeated unit produced in the course of PCR amplification of STR sequences. The mechanism by which these artifacts are formed is understood, but it represents an intrinsic limitation of the PCR technology and therefore no effective remedy has been found to eliminate these spurious products (Olejniczak M, Krzyzosiak WJ., Electrophoresis. 2006 Oct; 27(19):3724-34). The term "-1 stutter" as used herein refers to a stutter byproduct that is one repeat unit smaller than its associated allele. Similarly, "+1 stutter" refers to a stutter byproduct that is one repeat unit larger than its associated allele. The term '-1 stutter fraction' refers to the height (or area) of the -1 stutter peak divided by the height (or area) of the true allele peak. Similarly, "+1 stutter fraction" refers to the height (or area) of the +1 stutter peak divided by the height (or area) of the true allele peak.

The term "Restriction and Circularization-Aided Rolling Circle Amplification (RCA-RCA)" refers to a whole genome amplification procedure which retains the allelic differences among degraded amplified genomes while achieving almost complete genome coverage. RCA-RCA utilizes restriction digestion and whole genome circularization to generate genomic sequences amenable to rolling circle amplification.

The term "STR primers" as used herein refers to any commercially available or made-in-the-lab nucleotide primers that can be used to amplify a target nucleic acid sequence from a biological sample by PCR. There are approximately 1.5 million non-CODIS STR loci. Non-limiting examples of the above are presented in the following website www.cstl.nist.gov/biotech/strbase/str_ref.htm that currently contains 3156 references for STRs employed in science, forensics and beyond. In addition to published primer sequences, STR primers may be obtained from commercial kits for amplification of hundreds of STR loci (for example, ABI Prism Linkage Mapping Set-MD10 -Applied Biosystems), and for amplification of thousands of SNP loci (for example, Illumina BeadArray linkage mapping panel). The term "CODIS STR primers" as used herein refers to STR primers that are designed to amplify any of the thirteen core STR loci designated by the FBI's "Combined DNA Index System", specifically, the repeated sequences of TH01, TPOX, CSF1PO, VWA, FGA, D3S1358, D5S818, D7S820, D13S317, D16S539, D8S1179, D18S51, and D21S11, and the Amelogenin locus.

The term "signal ratio" as used herein refers to the ratio between the intensities of the signals obtained from amplification of two loci.

### 3. Methods for distinguishing between natural and artificial DNA samples

Also disclosed is a method for distinguishing between natural and artificial DNA samples. A general scheme is as follows: the method accepts as input a DNA sample. The DNA undergoes a procedure including one or more biochemical steps followed by signal detection. In the last step of the procedure, the signal is analyzed to determine whether the DNA is natural or artificial. In another aspect, the present description provides a method for verifying that a DNA profile is of natural DNA. A general scheme is as follows: the method accepts as an input a DNA sample that underwent profiling (e.g. with Identifiler). The DNA sample undergoes a verification procedure which includes one or more biochemical steps followed by signal detection. In the last step of the entire procedure, data from both profiling and verification of the DNA sample are analyzed. The signal analysis determines whether the profile obtained from the DNA sample represents natural (*in vivo*) or artificial (*in vitro*) DNA.

In various aspects, the methods of the present description concern the verification that DNA profiles represent natural DNA. The methods are employed on a DNA sample in question, for example, DNA from a blood sample found at a crime scene. The isolation of nucleic acids (*e.g.* DNA) from a biological sample may be achieved by various methods known in the art (*e.g.* see Sambrook et al, (1989) Molecular Cloning: A Laboratory Manual, 2nd ed. Cold Spring Harbor, New York). Distinguishing between natural and artificial DNA, or the determination whether a DNA profile represents natural DNA, may be accomplished using various strategies, including those described in the following sections.

## 4. Methylation

Methylation in the human genome occurs in the form of 5-methyl cytosine and is confined to cytosine residues that are part of the sequence CG (cytosine residues that are part of other sequences are not methylated).

Some CG dinucleotides in the human genome are methylated, and others are not. In addition, methylation is cell and tissue specific, such that a specific CG dinucleotide can be methylated in a certain cell and at the same time unmethylated in a different cell, or methylated in a certain tissue and at the same time unmethylated in different tissues. Since methylation at a specific locus can vary from cell to cell, when analyzing the methylation status of DNA extracted from a plurality of cells (e.g. from a forensic sample), the signal can be mixed, showing both the methylated and unmethylated signals in varying ratios. The inventors discovered that in some genomic regions all CG loci are constitutively methylated. These regions are provided in Table 1 and in the section herein entitled Sequences. The inventors also discovered that in some genomic regions all CG loci are partially methylated. These regions are provided in Table 1 and in the section herein entitled Sequences. The inventors also discovered that in some genomic regions all CG loci are constitutively unmethylated. These regions are provided in Table 1 and in the section herein entitled Sequences. The inventors also discovered contiguous genomic regions containing constitutively methylated, partially methylated, and constitutively unmethylated CG loci. These regions are provided in Table 1 and in the section herein entitled Sequences. There are several different methods for determining the methylation level of genomic loci. Examples of methods that are commonly used are bisulfite sequencing, methylation-specific PCR, and methylation-sensitive endonuclease digestion. Further, various data sources are available for retrieving or storing DNA methylation data and making these data readily available to the public, for example MetDB (http://www.methdb.net).

Exemplary methods for determining the methylation level of nucleic acids include, but are not limited to the following methods:

*Bisulfite sequencing.* Bisulfite sequencing is the sequencing of bisulfite treated-DNA to determine its pattern of methylation. The method is based on the fact that treatment of DNA with sodium bisulfite results in conversion of non-methylated cytosine residues to uracil, while leaving the methylated cytosine residues unaffected. Following conversion by sodium bisulfite, specific regions of the DNA are amplified by PCR, and the PCR products are sequenced. Since in the polymerase chain reaction uracil residues are amplified as if they were thymine residues, unmethylated cytosine residues in the original DNA appear as thymine residues in the sequenced PCR product, whereas methylated cytosine residues in the original DNA appear as cytosine residues in the sequenced PCR product.

## 5. Methylation-specific PCR

Methylation specific PCR is a method of methylation analysis that, like bisulfite sequencing, is also performed on bisulfite-treated DNA, but avoids the need to sequence the genomic region of interest. Instead, the selected region in the bisulfite-treated DNA is amplified by PCR using two sets of primers that are designed to anneal to the same genomic targets. The primer pairs are designed to be "methylated-specific" by including sequences complementing only unconverted 5-methylcytosines, or conversely "unmethylated-specific", complementing thymines converted from unmethylated cytosines. Methylation is determined by the relative efficiency of the different primer pairs in achieving amplification.

It should be understood in the context of the present invention that methylation specific PCR determines the methylation level of CG dinucleotides in the primer sequences only, and not in the entire genomic region that is amplified by PCR. Therefore, CG dinucleotides that are found in the amplified sequence but are not in the primer sequences are not part of the CG locus.

## 6. Methylation-sensitive endonuclease digestion

Digestion of DNA with methylation-sensitive endonucleases represents a method for methylation analysis that can be applied directly to genomic DNA without the need to perform bisulfite conversion. The method is based on the fact that methylation-sensitive endonucleases digest only unmethylated DNA, while leaving methylated DNA intact. Following digestion, the DNA can be analyzed for methylation level by a variety of methods, including gel electrophoresis, and PCR amplification of specific loci.

In the procedure based on methylation-sensitive endonuclease digestion, each CG locus is comprised of one or more CG dinucleotides that are part of recognition sequence(s) of the methylation-sensitive restriction endonuclease(s) that are used in the procedure. CG dinucleotides that are found in the amplified genomic region, but are not in the recognition sequence(s) of the endonuclease(s) are not part of the CG locus.

In one embodiment, the one or more CG loci that are detected are partially methylated in natural DNA, but would be unmethylated in artificial DNA. Partial methylation would be expected to result in a mixture of T and C at the position being interrogated. Hybridization would be observed to both the T specific probes/primers and the C specific probes/primers, similar to detection of a heterozygous SNP. Relative amounts of hybridization may be used to determine the relative amount of methylation. Alternatively, both C and T would be observed upon bisulfite sequencing. Alternatively, fluorescent signals corresponding to amplification products of methylated or partially methylated CG loci can be detected.

**7. Overview of the Present Signal ratio Assay**

As mentioned above, one particular assay of the present invention involves the quantitative comparison of intensity of the signals from a pair of locus-specific amplification products produced by performing a Polymerase Chain Reaction on restriction-digested DNA. See Figure 1A,B. The numerical ratio of intensities corresponds to the category of the template DNA, for example whether the DNA sample was extracted from semen or from a non-semen tissue. For example, in one embodiment, locus 1 and locus 2 can be amplified using fluorescently labeled primers, separated by electrophoresis, and the intensity of the signals is the relative fluorescence units (rfu) of peaks corresponding to the loci. See Figure 1B,C. The intensity of the signals will correspond to the successfulness of amplification of locus 1 and locus 2 from the source DNA template. By comparing rfu between the two amplification products one can calculate a signal ratio - a ratio that reflects the ratio of the quantities of the amplification products.

In addition, however, one aspect of this assay includes the predetermination of the expected signal ratios from various types of DNA categories. Thus, the template DNA that is subject to analysis is first digested with a methylation-sensitive restriction endonuclease before it is cycled through the quantitative-styled PCR amplification protocol. If the level of methylation at the site/s of the restriction recognition sequence/s in the pair of the two loci in the various categories of DNA is known, the skilled person can predict the expected signal ratios of amplification products of the pair of loci in the respective categories. Alternatively, if the levels of methylation are unknown, the skilled person can nonetheless empirically determine the signal ratios between a pair of genomic loci in the various categories of DNA by performing the assay on samples from known categories.

With this premise, the present inventive assays comprise digesting a DNA sample with a methylation-sensitive and/or methylation dependent enzyme, performing a PCR amplification reaction on the digested DNA, and determining the intensity of the signals from locus-specific amplification products. As mentioned, the intensity of signals can be quantified or measured by using fluorescent PCR. Determination of the category of the DNA sample is performed by comparing the observed signal ratios to reference ratios obtained from DNA samples of known categories, and determining which category corresponds best to the observed ratios.

This particular signal ratio assay is not reliant upon identifying or obtaining measurements of the absolute methylation fraction or level of selected loci. In addition, this particular signal ratio assay is not reliant upon the efficiencies of the primer pairs used, does not necessitate that both primer pairs have similar efficiencies, is not reliant upon amount of input template DNA, is not reliant upon specific thermocycler machine and reaction conditions. Rather, the assay determines the ratio between two signals which correspond to the ratio of methylation levels in the different loci. By this manner, the quantity or concentration of starting DNA material in the sample is irrelevant to the analysis and does not skew the output results. That is, the ratio of signal levels between a first locus and a second locus will remain constant regardless of how much DNA is used as a template for PCR and regardless of the number of amplification cycles that are run on the thermocycler. For example, a signal ratio of 10 between loci 1 and 2 will remain the same whether the input DNA represents methylation levels of 0.9 and 0.09 (90% methylation in locus 1 and 9% in 2), or 0.5 and 0.05 (50% methylation in locus 1 and 5% in 2), etc. In another example, a signal ratio of 10 between loci 1 and 2 will remain the same regardless of the amount of DNA template used in the reaction.

The fact that the inventive assay does not depend on determination of methylation levels at any genomic locus, and does not require any external control, allows the assay to be more accurate, cost effective, and applicable to samples with low amounts of DNA.

In the context of forensic tissue identification, the inventive assay represents a standardized procedure which is identical regardless of the specific tissue identification task that is being performed (except for the use of panel-specific primers), that requires only standard forensic procedures and equipment, thus enabling the assay to be automated and easily integrated into forensic labs.

Furthermore, the setup of the inventive assay makes possible combining tissue identification and DNA profiling into a single assay.

The fact that the inventive assay performs digestion and amplification in the same reaction minimizes the complexity (the number of required tubes and pipetting steps of the assay), thereby minimizing the risk of contamination and/or error.

This inventive assay therefore provides a useful biochemical marker in the form of, in one example, a numerical ratio, that can be used to differentiate between natural DNA and artificial DNA that was synthesized *in vitro* such as by PCR, chemical synthesis, or multiple displacement amplification (MDA). More particular details of this exemplary assay follow.

### 8. Characteristics of Primers for Locus-Specific Amplification

Accordingly, an aspect of the present invention concerns obtaining a "signal ratio" in which the fluorescence intensities of multiple DNA loci amplification products produced from fluorescent PCR are compared to one another in order to calculate ratios between successive pairs of loci, e.g., Loci #1 vs. Loci #2; Loci #1 vs. Loci #3; Loci #1 vs. Loci #4; Loci #2 vs. Loci #3, Loci #2 vs. Loci #4, and so on. When this technique is used to test the natural vs. artificial nature of a DNA sample, the primers that are used in the signal ratio amplification reactions are chosen so as to amplify a pair of loci that are differentially methylated in natural DNA (e.g. the first is 90% methylated and the second is 30% methylated in natural DNA).

One consideration for selecting which two pairs of primers (a first pair and a second pair) to use to amplify two loci (1) and (2) is the degree to which the two loci are differentially methylated in *in vivo* DNA. Thus, a pair of loci in which locus (1) is more methylated in comparison to locus (2), are examples of loci that can be used to design primers for the signal ratio amplification assay.

### 9. Selection of Genomic Loci

1. Selection of putative loci can be performed in any of three modes:

   (i) Select a locus closest to the transcription start site of a gene that has been shown in the literature to be differentially expressed (mRNA data) in the relevant categories (e.g. different tissues).

   (ii) Select a locus that has been shown in the literature to be differentially methylated between different categories (e.g. tissues).

   (iii) Select a random genomic locus.

2. Design primers for putative loci.

3. Empirically test "informativeness" of putative loci. For each putative locus calculate the signal ratio between the putative locus and another putative locus, or alternatively, between the putative locus and an undigested control locus.

4. Choose "informative" loci (i.e. loci whose signal ratios are significantly different in the different DNA categories) for use in the assay.

### 10. Methylation-Sensitive Restriction Endonucleases

A second consideration of the present inventive method is the selection of loci that are or are not cut or digested by a methylation-sensitive and/or methylation-dependent restriction endonuclease, depending on their methylation status. The endonuclease is selected if, for instance, it is unable to cut the DNA strand if its recognition sequence in that locus is methylated. Thus, in the context of locus (1), which is methylated, and locus (2), which is not methylated, an endonuclease like HhaI or HpaII will not digest locus (1) but will digest locus (2). Accordingly, the selection of loci for amplification in the signal ratio assay may also take into account the presence of methylation-sensitive restriction endonuclease recognition sequences within each locus.

Forward and reverse primers can then be designed to anneal to a region of DNA that flanks the recognition sequence of the loci.

Accordingly, in the case of a methylation-sensitive enzyme, if a locus is methylated it will (A) not be digested but (B) it will be amplified. Conversely, if a locus is unmethylated, it will (A) be digested but (B) not amplified. In the case of a methylation-dependent enzyme, the situation is vice versa.

### 11. Advantages of the Signal Ratio Assay

The signal ratio assay of the present invention has several advantages and benefits over other approaches for analyzing methylation, and therefore the skilled person would appreciate the various advantageous uses to which this signal ratio assay can be put. For instance, this assay is insensitive to various "noise" factors inherent when relying on the absolute intensity level of an amplicon signal that fluctuates as a consequence of changes in template DNA concentration, thermocycler manufacturer, and PCR conditions, and presence of inhibitors. Instead, the presently-calculated methylation ratio does not depend on such factors, since the two analyzed loci are co-amplified in the same reaction and are therefore subject to the effects of such disparities. Thus, the present inventive method does not require absolute quantification of genomic targets or amplicons; and the assay is a single stand-alone reaction with no need for a standard curve or any external controls.

The signal ratio can be performed on very small quantities of DNA in a single biochemical reaction and is therefore an inexpensive, rapid, and powerful method for establishing, for example, whether a sample contains natural or artificial DNA. An important feature of the design of the inventive methods is that it can be combined with other PCR-based procedures, such as DNA profiling, in a single biochemical reaction.

### 12. Capillary Electrophoresis and Multiplexing

The rapidity of the analysis is evident in consideration of the use of, for instance, capillary electrophoresis to separate numerous amplification products produced from the amplification of multiple pairs of target loci. As described above the present signal ratio assay can be performed on multiple loci, and in each case a signal ratio is calculated for each pair of loci separately. For example, if four loci (A,B,C,D) are co-amplified in the reaction, six different signal ratios can be calculated, *i.e.:* A/B, A/C, A/D, B/C, B/D, C/D.

Accordingly, if "n" loci are co-amplified, then $(n^2-n)/2$ different ratios can be calculated. Therefore, the amount of information that is provided by the present methylation assay rises exponentially with the number of analyzed loci. Capillary electrophoresis, as opposed to real-time PCR amplification methods, can distinguish between a large number of loci in a single run. For example, for DNA profiling, 17 genomic loci are routinely co-amplified from a particular DNA sample, and analyzed together. As a consequence, the performance of the present signal ratio assay on all 17 loci yields 136 independent signal ratios. Real-time PCR cannot simultaneously distinguish in a single reaction those numbers of discrete amplification products necessary to produce 136 ratios. About four loci can by distinguished by real time PCR, which corresponds to the calculation of only six ratios.

By contrast, capillary electrophoresis can readily separate out amplification products from all paired permutations of 17 loci and can therefore readily produce data to simultaneously calculate all 136 signal ratios in a single reaction. Theoretically, hundreds of loci can be run together and separated in a single capillary electrophoresis run. In order to facilitate analysis of multiple ratios, it can be advantageous to calculate a single score, herein termed "Combined Signal Score" (CSS), from these ratios. The CSS can then be used, for example, in order to ascertain whether the source DNA sample is natural or artificial.

### 13. Sequences

The sequences provided herein for the various CODIS, PowerPlex® 16, and other loci commonly used for profiling, *i.e.,* SEQ ID NOs. 1-25 are of genomic loci commonly used for STR profiling., have been analyzed herein to determine (1) the position of every cytosine-guanine (CG) dinucleotide, (2)the methylation level of all CG dinucleotides in the locus in natural DNA, and (3) the methylation sensitive and methylation dependent restriction enzyme profile for that particular locus. The sequence listing included within the text of this application therefore provides guidance to the skilled person in the identification of particular methylation-sensitive and methylation-dependent restriction endonucleases that can be used in accordance with the inventive ratio-generating assay methods. For example, SEQ ID NO. 8, which is the 751-long nucleotide sequence for the "vWA" CODIS and PowerPlex® 16-amplified locus, comprises only one CG dinucleotide at position 571. That CG and the nucleotides that immediately flank it, provide the recognition sequence for enzymes BssKI, HpaII, Nt.CviPII, and SEQ ID NOs 26-32 are genomic loci that have been found by the inventors to produce signal ratios with significantly different values between natural and artificial DNA samples. SEQ ID NOs 33-54 are genomic loci that have been found by the inventors to produce signal ratios with significantly different values (based on the Kolomogorov-Smirnov test using a threshold of $p = 0.05$) between samples of DNA extracted from different tissues.

The sequence information provided herein also permits the skilled artisan to design forward and reverse amplification primers that anneal to regions of a selected locus that flank the CG and restriction site. Thus, the present invention is not limited to the amplification of, for instance, CODIS loci, using only those commercially available primers, although the use and availability of commercially available primers can be a more convenient and cost-effective option for performing the present inventive authentication assays.

### 14. Algorithm and Software

In one embodiment, calculation of signal ratios and/or representation ratios (for example in order to determine whether a DNA profile represents natural DNA) is performed based on analysis of the signal intensities of amplification products of fluorescent PCR that are run on a capillary electrophoresis machine. The output of the capillary electrophoresis machine is a binary computer file (for example, an FSA file in the case of capillary electrophoresis machines of Applied Biosystems). This file includes information regarding the capillary electrophoresis run, including the channel data, which is the relative fluorescent units (rfus) of each fluorophore as a function of each sampling time point (called datapoint).

The present description also describes a software program that accepts as input a file that is the output a capillary electrophoresis machine run, and calculates the fluorescence intensities of a set of loci whose amplification products were run on the capillary electrophoresis machine. Based on these intensities, the software calculates signal ratios and/or representation ratios (or combined scores based on these ratios), based on a set of predefined pairs of loci for which the ratios are defined to be calculated. Finally, the software outputs a decision based on the comparison of the calculated ratios to predefined thresholds.

Following is a scheme of this analysis performed by the software program:

1. Subtract from the signal of each dye channel the baseline level, defined as the mean rfu level of that channel in the range 400-500 bps, and remove spectral overlap between fluorophores based on the matrix obtained from the spectral calibration procedure of the capillary electrophoresis machine.

2. Obtain the rfu level of each analyzed locus, defined as the maximal rfu level in the range -0.5bp to +0.5bp of the expected size of the locus.

3. Calculate signal ratios between a set of pairs of loci, defined as the ratio between the rfu levels of the first and second loci in the pair (to avoid division by zero assign a level of 50 rfus to all loci with rfu levels below 50).

4. For each ratio calculated in step 3 and for each tissue type calculate a Probability Score, defined as the value of the reference probability function (see below) at the observed ratio.

5. For each tissue type, calculate a Combined Probability Score, defined as the product of the separate Probability Scores for that tissue obtained in step 4 (where n is the number of signal ratios).

6. For each tissue type, calculate a Likelihood Score (representing the likelihood that the DNA sample originated from that tissue), defined as the ratio between the Combined Probability Score of the tissue and the sum of the Combined Probability Scores of all tissues. The tissue with the highest Likelihood Score is considered the source tissue of the sample.

### 15. Reference probability functions

The reference probability function of a certain pair of loci in a certain tissue is the gamma distribution function fitted to the set of signal ratios observed in samples of natural DNA of that tissue type.

### 16. Correction for incomplete digestion

Since incomplete digestion of template DNA might occur as a result of the presence of inhibitors in the sample, it is useful to incorporate into the assay an analysis of actual digestion level. This can be achieved by incorporation of at least one "digested control locus" and at least one "undigested control locus". A digested control locus is a genomic locus known to be unmethylated in natural DNA in all potential tissues. An undigested control locus is a locus that is either known to be methylated in natural DNA in all potential tissues, or is a locus lacking the recognition sequence of the endonuclease used in the assay. The signal ratio between a digested control locus and an undigested control locus is correlated to the actual digestion level in the assay. The algorithm can make use of such loci by checking whether adequate digestion occurred (and if not - aborting analysis), and by modifying actual rfu levels of loci to values representing rfu values that would have been obtained had complete digestion occurred. If this modification is performed, then signal ratios are calculated from the modified rfu values.

## EXAMPLES

## MATERIALS AND METHODS

### A. Collection of biological tissues

Blood (venous/menstrual), saliva, semen, skin epidermis, urine, and vaginal secretion were collected from volunteers. Informed consent was obtained from all participants recruited into the study.

### B. DNA extraction and quantification

DNA was extracted from all samples using by organic extraction. The quantity of recovered DNA was determined using the Quantifiler® Human DNA quantification kit (Applied Biosystems) and the 7300 Real-Time PCR system (Applied Biosystems).

### C. Selection of genetic loci and primer design

Random CpG islands (defined as a region >= 200 bp and with a GC content >= 0.5 and with at least 8 CGs; corresponding to observed/expected CpG ratio > 0.6) were searched using a software program developed for this task. From these, CpG islands that contained a HhaI recognition sequence were selected for initial screening. Primers (18-28 bps) flanking the HhaI recognition sequence were designed with a Tm of 64-66°C and for amplicon sizes of 66-150 bps. Fluorescently-labeled forward primers (FAM or JOE) were purchased from Integrated DNA Technologies. Loci were screened in pairs for differential methylation by comparing signals obtained from amplification of pooled DNA samples (each containing DNA from 10 individuals) of each tissue type. Loci that showed significant differential amplification patterns (defined as the largest signal ratio observed in all tissue types being greater that three times the smallest ratio observed) were selected for multiplexing. In total, 205 genomic loci were screened of which 38 showed significant differential amplification patterns. From these, 16 loci were used in the experiments. For the stand-alone tissue identification assay, 15 loci that could be co-amplified without significant noise were used. For the combined semen detection and profiling assay two loci (L68346 which was also used for the stand-alone assay and L16264) were used.

### D. Endonuclease digestion, PCR, and capillary electrophoresis

In stand-alone assay experiments, each DNA sample was digested by HhaI and subjected to PCR amplification in the same reaction, consisting of 30 U HhaI (New England Biolabs), 2.5 U AmpliTaq Gold (Applied Biosystems), 2.5 μg BSA (New England Biolabs), 0.2 mM each dNTP, 0.1-0.3 μM each primer, 2.5μl reaction buffer (150mM Tris-Hcl, 15mM $MgCl_2$) and DDW to a total volume of 25μl. The reaction composition in the combined tissue identification-profiling experiments was the same as in the stand-alone assay except for the primers, which were 0.2μM each semen identification primer together with 5μl Primer Set of ProfilerPlus (Applied Biosystems). Reactions (for both stand-alone and combined assay) were performed in a GeneAmp® PCR System 9700 (Applied Biosystems), and the thermocycling program used was: 37°C for 15 min, 95°C for 11 min, followed by 28 cycles of 94°C for 1 min, 59°C for 1 min, 72°C for 1 min, and followed by a final extension step of 60°C for 45 min. A mix containing 1.5μl of amplification products, 24.5μl HiDi formamide (Applied Biosystems) and 0.5μl ROX size standard (Applied Biosystems) was denatured (95°C for 3 min immediately followed by 3 min on ice), and run on an ABI 310 Genetic Analyzer (Applied Biosystems) according to the manufacturer's instructions. All resulting electropherograms were analyzed using in-house developed software, and electropherograms resulting from combined tissue identification-profiling experiments were also analyzed using GeneMapper ID-X analysis software (Applied Biosystems).

### E. Tissue identification assay -data analysis

Data analysis for tissue identification was performed by an in-house developed software that accepts as input fsa files and outputs the most likely source tissue. Derivation of semen percentage in mixture samples was performed based on profiling loci and tissue identification loci. Derivation based on profiling loci was performed by dividing the sum of rfu values of alleles corresponding to the contributor of the semen by the total sum of rfu values of all alleles in STR loci that had 4 genotyped alleles. Derivation based on tissue identification loci was performed first by calculating the expected signal ratio for all possible percentages of mixtures (from 0 to 100% with 1% increments, based on the signal ratio of undigested DNA), and the algorithm provided the percentage of semen for which the expected signal ratio is closest to the observed signal ratio.

### F. Simulation of degraded samples

Twenty nanograms of each sample were digested for 10min at 37°C with 0.01U DNAseI (Ambion), 2μl 10X DNAseI buffer (Ambion) in total reaction volume of 20μl, followed by heat-inactivation at 75°C for 10 min. One nanogram of each digested DNA sample was then subjected to the combined tissue identification-profiling assay.

### Example 1

### Categorization of DNA according to tissue type - stand alone assay

In this example, the categorization procedure is termed "tissue identification assay." The tissue identification assay uses a panel of loci that are differentially methylated between tissues to determine the most probable source tissue of a DNA sample as shown in Table 1 (Figure 7). A scheme of the assay is presented in Figure 1A. One nanogram of DNA from a forensic sample in question is digested with the HhaI methylation-sensitive restriction enzyme, which cleaves DNA at its recognition sequence GCGC only if it is unmethylated (while leaving methylated targets intact). A panel of tissue identification loci is then amplified by PCR from the digested DNA using fluorescently-labeled primers, and an aliquot of amplified products is separated by capillary electrophoresis. Loci with higher methylation levels are amplified with higher efficiency (because more DNA molecules are protected from digestion), producing a relatively strong signal in the electropherogram (Figure 1B, locus A). Conversely, loci with a lower methylation level are amplified with lower efficiency, yielding a relatively weak signal in the electropherogram (Figure 1B, locus B).

Automated signal analysis software enables analysis of an output electropherogram, and assigns heights (in rfu) to amplicons corresponding to tissue identification loci. Although the height of a single locus is correlated with its methylation level, the actual methylation level cannot be derived, because that level also depends on the precise template concentration and specific PCR conditions. Instead, ratios of methylation levels between co-amplified loci can be calculated, since all the loci are subjected to the same template concentration and specific reaction conditions. Therefore, for each pair of loci a signal ratio, defined as the ratio between the heights of the first and second loci, is calculated, and this ratio reflects the ratio between the methylation levels of the corresponding loci. All calculated signal ratios are combined into a single vector (if, for example, in a 15-loci panel the vector contains 105 ratios - between locus 1 and 2, locus 1 and 3, etc.), which is then compared to a database of reference vectors obtained from a data set of samples of known tissue origin. The tissue identification algorithm calculates for each potential tissue source a likelihood score, reflecting the likelihood that the DNA sample originated from that tissue, and the output of the algorithm is the most likely tissue.

A stand-alone tissue identification assay was performed on 50 samples: 14 blood, 14 saliva, 11 semen, and 11 skin epidermis using a panel of 15 tissue identification loci, ranging in amplicon size from 66 to 150 bps. Figure 2 depicts electropherograms of eight of these samples - two of each tissue type. Although samples of the same tissue type from different individuals were not completely identical (due to natural variability of methylation levels and stochastic PCR effects), it was nevertheless evident that each tissue type had a distinct methylation profile. For example, the ratio of L91762/L68346 in semen samples ranged from 0.04-0.53 and was higher in all other tissue samples (2.15-18.28; Table 2 in Figure 8), and therefore a low L91762/L68346 ratio was distinctive of semen samples. The ratio of L76138/L26688 was low in blood and saliva (0.08-1.54) and higher in semen and skin epidermis (2.04-19.56; Table 2 in Figure 8); therefore a high ratio of L91762/L68346 concomitant with a high ratio of L76138/L26688 was distinctive of skin epidermis samples. The tissue identification algorithm correctly identified the true tissue source of all 50 samples. Further analysis of the data using subsets of tissue identification loci revealed that the full set of 15 loci was redundant and 100% identification was achieved by using only 7 out of the 15 loci (L91762, L68346, L50468, L14432, L30139, L15952, and L26688). Because tissue identification relies on efficient digestion, complete digestion of template DNA by HhaI was assessed in a single digestion-amplification reaction setup. The 50 DNA samples were subjected to the same procedure but using a panel of two different loci as shown in Table 3 (Figure 9) - a control locus without a HhaI site (L98328) and a locus which was previously shown to be completely unmethylated in these tissues (SW14; see D. Frumkin et al., Authentication of forensic DNA samples, Forensic Sci. Int. Genet. 2009, doi: 10.1016/j.fsigen.2009.06.009, which is incorporated herein by reference). All samples showed presence of the reference locus and complete absence of SW14, indicating complete digestion by HhaI in contrast to the undigested sample which showed presence of both loci (example runs are shown in Figure 4).

### Example 2

### Analysis of noise factors affecting signal ratios

The assay described in example 1 is based on utilizing loci that are differentially methylated between tissues, for which signal ratio values are generally tissue-specific (Figure 1C). However, even in differentially methylated tissues, there potentially could be some overlap in the observed signal ratios as a result of natural variability in methylation levels among different individuals and/or artifacts associated with the PCR such as differences in DNA template concentration (e.g. as a result of pipetting errors and stochastic effects). The effect of each of these "noise" factors was analyzed in relation to the "signal", which is the average signal ratio difference between tissues. Two loci with an average 10-fold difference in signal ratio values between semen and blood were selected. Different PCRs were performed on the same semen sample using 1 ng and 2 ng DNA as template. The ratios obtained from these amplifications were compared to each other and to ratios obtained from amplification of different blood samples. The differences between the ratios obtained in different PCRs and using different amounts of input DNA were more than one order of magnitude smaller than the differences between the ratios obtained from the different tissues (Figure 1D).

**Example 3**

**Combined semen detection and DNA profiling**

In this example categorization of the DNA is into one of two categories: semen and non-semen where non-semen refers to all tissues except semen. The categorization is performed together with DNA profiling in the same reaction.

The tissue identification procedure utilizes the same platforms used in standard STR profiling (i.e. thermocycler and capillary electrophoresis instruments) and is therefore readily amenable to integration with DNA profiling in a single reaction. This capability was demonstrated by generating an integrated STR profiling and semen detection assay. A STR profiling kit (Profiler Plus) PCR was supplemented with primers specific for two semen identification loci (Table 4 in Figure 10). These loci consist of a 70 bps amplicon which is efficiently amplified only in semen and a 95 bps amplicon which is efficiently amplified in all tissues except semen. The assay was tested on pure samples of semen, urine (male), venous blood, menstrual blood, vaginal secretion, and saliva. The correct pattern of semen identification amplification was observed and the tissue identification algorithm correctly identified presence/absence of semen in all samples. Figures 3A and B depict complete electropherograms of semen and urine samples (obtained from the same individual), and Figure 3C depicts FAM channel data from all samples including the two semen identification loci and three profiling loci. The observed signal ratio was 25.04 in the semen sample, and 0.04 - 0.089 in the other samples. The combined assay was also tested on mixtures of semen and saliva at various ratios (Figure 3D). For each of these mixtures the percentage of the semen in the sample was derived from the signal ratio of the semen identification loci and compared with the corresponding percentage derived from the profiling loci (the semen and saliva samples were obtained from different individuals and could therefore be differentiated based on STR loci). The percentages derived were comparable, with a maximum difference of 10% (Table 5 in Figure 11). Moreover, the presence vs. absence of semen was correctly determined in all samples (including a sample that contained only 13% semen; Figure 3D). The STR profiles obtained from the samples using the integrated profiling-semen detection assay were identical to profiles obtained from the same samples using ProfilerPlus (without semen detection).

**Example 4**

**Categorization of Aged and degraded DNA samples**

The stand-alone tissue identification assay (described in example 1) was tested on two DNA samples extracted from 20-month old stains (blood and semen on cotton) as a proof-of-concept for application to forensic-type samples. Both samples amplified well, similarly to fresh samples (Figure 5), and the algorithm correctly identified the tissue source of both samples. In order to simulate degraded DNA samples, two additional DNA samples (blood and semen) were subjected to digestion with DNAseI under conditions that yield partial digestion. These samples were then analyzed with the combined semen detection and profiling assay. DNAseI digestion resulted in dropout of alleles in the larger STRs in both samples such that their profiles were partial. In the blood sample only D3S1358 and Amelogenin alleles were typed, and in the semen sample dropout of alleles was observed in FGA, D21S11, D18S51, D13S317 and D7S820 (Figure 6). The smaller tissue identification loci amplified successfully with the expected pattern, and the algorithm correctly identified the presence/absence of semen in these samples.

**Example 5**

**Combined profiling and categorization of DNA**

This particular assay employs both conventional profiling of a DNA sample (with the Promega PowerPlex 16 kit), and categorization of DNA into categories of natural vs. artificial.

One aspect of this assay, therefore, is to check the natural nature of the DNA against several or all types of artificial DNA (see the Definitions section), and therefore contains an analysis of several different ratios. The combination of ratios makes it possible to therefore compare a single parameter various DNAs synthesized from various methods. A single parameter, for example representation ratio, can distinguish between a sample that contains the entire genome (natural DNA but also MDA synthesized DNA - as in this example) and a sample that contains only a subset (for example, only CODIS loci).

Two samples of DNA were analyzed - a natural DNA sample extracted from the blood of subject A, and an artificial DNA sample synthesized by in vitro multiple displacement amplification using a commercial kit (from a minute amount of subject A's real DNA as template).

The two samples were first profiled with a conventional kit (PowerPlex16) and analyzed using a conventional profiling software (GeneMapperID-X). The scheme of this procedure can be briefly described as performing multiplex PCR using the PowerPlex16 primer mix on a DNA sample; separating the amplification products on a capillary electrophoresis machine; and then analyzing the output data with, for instance, the GeneMapperID-X software.

For each sample, the end product of this assay is a profile. The profiles of the two samples are identical, as can be seen in Figure 9A,B. Furthermore, the GeneMapperID-X software determined the profiles of both samples to be single contributor (i.e. from a single person) profiles, with no anomalies. This shows that a profile obtained from artificial DNA can be identical to a profile obtained from natural DNA. See D. Frumkin, et al., Authentication of forensic DNA samples, Forensic Sci. Int. Genet. (2009), doi:10.1016/j.fsigen.2009.06.009 , which is incorporated herein by reference.

Both samples of DNA were subsequently also analyzed by a combined profiling and categorization assay based on the following scheme: digest a DNA sample with HhaI and perform multiplex PCR using the PowerPlex16 primer mix with the addition of primers for amplifying an additional locus, such as Hypo23; separating the amplification products on a capillary electrophoresis machine; and analyzing the output data with the capillary analyzer software, such as the software algorithm disclosed herein.

For each sample, the end product of this assay is a profile and assignment of category. As can be seen in Figure 9C and D, the natural DNA was assigned as "natural", in contrast to the artificial sample which was assigned as "artificial".

In this example, the assignment of category was performed by analysis of three parameters:

SR1 (signal ratio of TPOX/D8S1179), SR2 (signal ratio of D3S1358/Hypo23), and RR (representation ratio of OCA2/D3S 1358).

The threshold values for these parameters are provided in Table 6:

**Table 6**

| | SR1 | SR2 | RR |
|---|---|---|---|
| Threshold for natural | >= 0.9 | >= 10 | >= 2 |
| Threshold for artificial | < 0.9 | <10 | <2 |

The observed values for these parameters, and the assigned category are provided in Table 7 (values that are above the threshold for natural DNA are shaded).

**Table 7**

| | SR1 | SR2 | RR | Category |
|---|---|---|---|---|
| Natural DNA sample | 1.23 | 17.54 | 3.31 | Natural |
| Artificial DNA sample | 0.06 | 2.06 | 2.47 | Artificial |

Assignment of the category was performed according to the following rule:

If all observed parameter values are above their respective thresholds for natural DNA, then assign a "natural" category, otherwise assign an "artificial" category.

**Example 6**

**Combined profiling and categorization of DNA (MDA vs. non-MDA)**

This particular assay employs both conventional profiling of a DNA sample (with the Promega PowerPlex16 kit), and categorization of DNA into categories of MDA (artificial DNA that was synthesized by Multiple Displacement Amplification vs. other (non-MDA) types of DNA.

Two samples of DNA were analyzed - a natural DNA sample extracted from the blood of subject A, and an artificial DNA sample synthesized by in vitro multiple displacement amplification using a commercial kit (from a minute amount of subject A's real DNA as template).

The two samples were first profiled with a conventional kit (PowerPlex16) and analyzed using a conventional profiling software (GeneMapperID-X). The scheme of this procedure is as follows:

DNA → multiplex PCR using the PowerPlex16 primer mix →separation of amplification products on a capillary electrophoresis machine → analysis of data with the GeneMapperID-X software

For each sample, the end product of this assay is a profile. The profiles of the two samples are identical, as can be seen in Figure 15A-B. Furthermore, the GeneMapperID-X software determined the profiles of both samples to be single contributor (i.e. from a single person) profiles, with no anomalies.

Both samples of DNA were subsequently also analyzed by the Capillary Analyzer software. For each sample, the end product of this assay is a profile and assignment of category. As can be seen in Figure 15C-D, the natural DNA was assigned as "non-MDA", in contrast to the MDA sample which was assigned as "MDA".

In this example, the assignment of category was performed by analysis of three parameters:

RR1 (representation ratio of vWA/D18S51), RR2 (representation ratio of CSF1PO/Penta_D), and RR3 (representation ratio of D21S11/D7S820).

A Combined Representation Score was calculated based on these three ratios as follows:

$$CRS = \sqrt[3]{R1*R2*R3}$$

The threshold value that was determined based on analysis of multiple MDA-samples and non-MDA-samples was 1.5, hence an unknown sample with a CRS value of above 1.5 is categorized as "MDA", while an unknown sample with a CRS value of below 1.5 is categorized as "non-MDA".

The CRS values of the MDA sample and non-MDA sample were calculated, and are as follows:

CRS value of MDA sample = 2.04

CRS value of non-MDA sample = 0.91

Based on the threshold value, both samples were correctly categorized.

**Example 7**

**Stand alone semen detector kit**

The assay employed by the kit accepts as input a DNA sample which then undergoes a biochemical procedure followed by signal analysis by a dedicated software. The output of the assay is the category of the DNA sample (semen or non-semen), and a statistical confidence level representing the likelihood that the outputted category is the true category of the DNA sample.

A semen detection kit of the present invention may comprise one or more of the following components:

1. A box that may comprise one or more of the following components:

**Tube 1** comprising a 5X primer mix of:

0.6μM SD1f (AAGAGCCCATCAGGCAGGTC);

0.6μM SD1r (GTTTCTTGTCGAGCAGCACGTGGATGATG);

1.75μM SD2f (CTCCAGAACTGGAACTTCCTG);

1.75μM SD2r (GTTTCTTAACTTGGAGACGACGGCATC);

1.25μM SD3f (TGGAGGACAATGCCCTGGTG);

1.25μM SD3r (GTTTCTTGGCTTCACCTGCGACCGTCTC);

1.75μM SD4f (CCCTCCGAGTGGCCAGCAG);

1.75μM SD4r (GTTTCTGACCACTGCCGTGGGAATG);

1.75μM SD5f (CTTCTCAGCCAATGGGAAGAG);

1.75μM SD5r (ACGTAGAAGGACCCGAGGAC);

0.9μM SD6f(TACAGACAAATCACTCAGCAGC); and

0.9μM SD6r (GTTTCTTGTCTGACACTCGGTTGTAGGTATT)

The forward primers (*e.g.*, SD_f) are fluorescently labeled;

**Tube 2** comprising a 10X reaction buffer (150mM TRIS-HCl, 15mM $MgCl_2$, 0.2mM each dntp, 2.5μg BSA);

**Tube 3** comprising HhaI restriction endonuclease;

**Tube 4** comprising a control semen DNA sampl;

**Tube 5** comprising a control non-semen DNA sampl;

**Tube 6** comprising a DNA ladder; and

a Material Safety Data Sheet (MSDS).

2. A paper detailing the following protocol, or similar instructions:

(a) For each reaction, combine in a 0.2 ml tube (not provided) the following ingredients: 5μl 5X primer mix, 2.5μl 10X reaction mix, 0.5μl HhaI endonuclease, 0.5μl DNA polymerase, 0.5 ng DNA, and DDW (distilled water) to a total volume of 25μl.

(b) In addition to the tested DNA, set up a positive control semen reaction using the supplied semen DNA, a positive control non-semen reaction using the supplied blood DNA, a negative control reaction using DDW instead of DNA, and a digestion control reaction using DDW instead of HhaI.

(c) Place the reaction tubes in the thermal cycler and run the following program: 37°C for 15 min, 95°C for 11 min, followed by 30 cycles of 94°C for 1 min, 59°C for 1 min, 72°C for 1 min, followed by a final extension step of 60°C for 45 min, and hold at 25°C.

(d) For each post-amplification reaction, mix 1.5μl product with 24.5μl formamide and 0.5μl fluorescent size standard in a new 0.2ml tube.

(e) Denature samples at 95°C for 3 minutes, then immediately chill on ice for 3 minutes.

(f) Run denatured samples in the capillary electrophoresis machine using the following parameters:

Module = GS STR POP4 (1 mL) F;

Inj. secs = 5;

Inj. kV = 15;

Run kV = 15;

Run C = 60;

Run time = 24 min

(g) Analyze output .fsa files in the TissueIdentifier analysis software.

3. "TissueIdentifier" analysis software implementing the tissue identification algorithm as described in the "Algorithm and Software" section above, with the following modifications:

In step 3 of the algorithm, the set of pairs of loci used are: {SD1,SD6}, {SD2,SD6), {SD3,SD6}, {SD4,SD6}, and {SD5,SD6}.

A correction for incomplete digestion was performed using the signal ratio of {SD5,SD6}. SD5 acted as a digested control locus and SD6 acted as an undigested control locus.

Reference probability functions of semen and non-semen were obtained for {SD1,SD6},{SD2,SD6},{SD3,SD6},{SD4,SD6} using a set of 27 reference semen samples obtained from 27 different individuals and 86 reference non-semen samples (blood, saliva, urine, vaginal swab, menstrual blood) obtained from different individuals.

The assay is based on analysis of 6 genomic loci. Of these, 4 loci were found by the inventors to be differentially methylated in semen vs. non-semen tissues. Two of these four loci are methylated in all tissues except semen, and the two other loci are unmethylated in all tissues except semen. The assay also includes an undigested control locus that does not contain a HhaI recognition sequence (SD6) and is therefore expected to be amplified successfully regardless of the tissue type, and a digested control locus (SD5) which was found by the inventors to be unmethylated in all potential tissues.

The kit was tested on 27 semen and 86 non-semen (blood, saliva, urine, vaginal swab, menstrual blood) DNA samples from 95 different donors of different sexes, ages, and ethnicities. The algorithm correctly identified the source of all samples with a typical confidence level > 99.999999%. Figure 12 shows sample plots of 6 samples. The tested samples were the same samples used for obtaining reference probability functions. However, in order to avoid bias, in each specific analysis, the analyzed sample was not used for obtaining the reference probability functions.

**SEQUENCES**

Sequence 1: D16S539

```
  1 CTCTTCTCAT TCCACAAGCT CTCCCCAAAA GACCCCATTC CTCCCCACCT TCAACCATCT
 61 CTGGCAGGGA GGAGGGGGAA CTGAGAGGCT ACTTTCTGAC CCAGGACCCT AAGCCTGTGT
121 ACGGAGAGAG CATGAGCTGG GTGAGCTGCT TGCCAAGGAG TGGCATCTGC CCTCATCAGT
181 GGACACAAAA AGCCCCAGGG GTTAAGTGGC CATGGCTGCC CTCATGGCTG CACCGGGAGG
241 ATGACTGTGT TCCCACTCTC AGTCCTGCCG AGGTGCCTGA CAGCCCTGCA CCCAGGAGCT
301 GGGGGGTCTA AGAGCTTGTA AAAAGTGTAC AAGTGCCAGA TGCTCGTTGT GCACAAATCT
361 AAATGCAGAA AAGCACTGAA AGAAGAATCC AGAAAACCAC AGTTCCCATT TTTATATGGG
421 AGCAAACAAA GGCAGATCCC AAGCTCTTCC TCTTCCCTAG ATCAATACAG ACAGACAGAC
481 AGGTGGATAG ATAGATAGAT AGATAGATAG ATAGATAGAT AGATAGATAT CATTGAAAGA
541 CAAAACAGAG ATGGATGATA GATACATGCT TACAGATGCA CACACAAACG CTAAATGGTA
601 TAAAAATGGA ATCACTCTGT AGGCTGTTTT ACCACCTACT TTACTAAATT AATGAGTTAT
661 TGAGTATAAT TTAATTTTAT ATACTAATTT GAAACTGTGT CATTAGGTTT TTAAGTCTAT
721 GGCATCACTT TCGCTTGTAT TTTTCTATTG ATTTCTTTTC TTTTCTTTTC TTTTTTGAGA
781 CAGAGTCTCA CTCTCACCCA GGCTGGAGTA CCGTGGCACG ATCTTGGCTC ATTGCAACCA
841 CCACCTCCCG GGCTCAAGTG ATTATCCTGC CTCAGCCTCC CAAATAGCT
```

Sequence 2: D7S820

```
  1 ATATGCTAAC TGGATGTGAA CAATTGTGTT CTAATGAGCT TAATATGAGT TTCATAATTT
 61 GTGCATTTTG CTGTTAAAAA GCCAGAAAAC AAAACAAAAC AAAATACTGA AACCAGTGTG
121 AACAAGAGTT ACACGATGGA AGGCATCAGT TTTCACACCA GAAGGAATAA AAACAGGCAA
181 AAATACCATA AGTTGATCCT CAAAATATGA TTGATTTTAA GCCTTATGAG ATAATTGTGA
241 GGTCTTAAAA TCTGAGGTAT CAAAAACTCA GAGGGAATAT ATATTCTTAA GAATTATAAC
301 GATTCCACAT TTATCCTCAT TGACAGAATT GCACCAAATA TTGGTAATTA AATGTTTACT
361 ATAGACTATT TAGTGAGATT AAAAAAAACT ATCAATCTGT CTATCTATCT ATCTATCTAT
421 CTATCTATCT ATCTATCTAT CTATCTATCT ATCGTTAGTT CGTTCTAAAC TATGACAAGT
481 GTTCTATCAT ACCCTTTATA TATATTAACC TTAAAATAAC TCCATAGTCA GCCTGACCAA
541 CATGGTGAAA CCCCGTCTCT AAAAAAAATA CAAAAATTAG CTGGATGCAG TAGCACATGC
601 CTGTAGTCCC AGCTACTCAG GAGGCTGGGG CAGGAGAACC ACTTGACCCA AGAAGCGGAG
661 GTTGCAGTGA GCCGAGATCG CACCACTGCA CTCCAGCCTG GGTGACAGAG TGAGACTCCA
721 TCTCAAGATA AAGAAATAAA TAAAAACAAA CAAACAAAAA AATTCCATAG GGGGTCAGGT
781 GCGGTGGCTC ATGCCTGTAA TCCCAGCACT TTGGGAGGCC GAAGCAGGTG GATCACTTGA
841 GGT
```

Sequence 3: D13S317

```
  1 AATATGAATT CAATGTATAC AGAGAGAGAG AGAGAGAGAG AGAGAGAGAG AGACTTCTAC
 61 AGAGCTCTAA GCATAATTGT GTAACTCCAA GCTCACAGTG CCTAAGACCA GTACCAGGCT
121 GACTCATTGG AAAGCTGCCA TAGTAAGACT CTTCTGTTCA CTGCATTATT TATTGATGTA
181 TTGCAAGCAC TTAGTTACAT TTCTAGCATA TAACACATGA TCAATAAATA TTTTGACATG
241 AACAAATGGT AATTCTGCCT ACAGCCAATG TGAATATTGG GATGGGTTGC TGGACATGGT
301 ATCACAGAAG TCTGGGATGT GGAGGAGAGT TCATTTCTTT AGTGGGCATC CGTGACTCTC
361 TGGACTCTGA CCCATCTAAC GCCTATCTGT ATTTACAAAT ACATTATCTA TCTATCTATC
421 TATCTATCTA TCTATCTATC TATCTATCAA TCAATCATCT ATCTATCTTT CTGTCTGTCT
481 TTTTGGGCTG CCTATGGCTC AACCCAAGTT GAAGGAGGAG ATTTGACCAA CAATTCAAGC
541 TCTCTGAATA TGTTTTGAAA ATAATGTATA TTAATGAATG TACAAATTTC CCCACTTGTA
601 CTTTCAGACT GTTATCTGTG AGTTAAAACT CCTCCACTCT TTTTCCTACC CAAATAATAG
661 CATACTTTTT TCTGAGTATA TTTTGGGAAG AAGAGTTATT CAGTTATTGT TATATTTTAA
721 AAAATTCCTT ATACCAAACT CTACTTGATC TAAGGCTATT CATTGAAACT TTCAGCATGC
781 TTAATAGCAG TC
```

Sequence 4: D5S818

```
  1 CCCTCTGTGT AGCCTGGCTA TGTGCCACAT TGTGAGGTTC TCTCCCTCTA GCTACTTCTT
 61 CCAGTTTCCT CTTTCAGGAT CCCAATTCCT TTCTCAAAGC ACTGGTGAAT AACTCCAAAT
121 ACTCCATCAT TTCATTATAC AGAGTAATAT AAGTCTTCTT TTTCTAAACC TCTCCCATCT
181 GGATAGTGGA CCTCATATTT CAGATGCTAA TAGGCTGTTG AGGTAGTTTC CTAAGCAAAA

241 AAGTAATTGT CTCTCTCAGA GGAATGCTTT AGTGCTTTTT AGCCAAGTGA TTCCAATCAT
301 AGCCACAGTT TACAACATTT GTATCTTTAT CTGTATCCTT ATTTATACCT CTATCTATCT
361 ATCTATCTAT CTATCTATCT ATCTATCTAT CTATCTTCAA AATATTACAT AAGGATACCA
421 AAGAGGAAAA TCACCCTTGT CACATACTTG CTATTAAAAT ATACTTTTAT TAGTACAGAT
481 TATCTGGGAC ACCACTTTAA TTAGAAGCTT TAAAAGCATA TGCATGTCTC AGTATTTAAT
541 TTTAAAATTA TTACATAATT ATATACTCCT TTGAATTAGA AAATTACAAA TGTGGCTATG
601 TATTATTTTC TCCCATGTAT TTTCAAAATG AGGGGGTAAG CCAGACCCTC TCCCTCTCCC
661 ATGCCTAATA GCTCAAAGTT AAAGGTAAAG AAACAAGAAA ATATGGTGAA AGTTAACCAG
721 CTTCACTTCA GAGGA
```

Sequence 5: CSF1PO

```
  1 ATTCAACACA TGAGGCACGG CCAGACCTAA ATGTCTCAGA GCCTGCTCCC ACTCCGATGA
 61 GCTGCTGCCT TGCTTCAGGG TCTGAGTCCA GTGACTGCCA CTGCCTGCAC CCAATCACCA
121 TAGCCAGAGA CCTGGAGGTC ATCCTTATCT CCTTTCTCTT CCTCATCCCT GCATCTCAGA
181 CTCTTCCACA CACCACTGGC CATCTTCAGC CCATTCTCCA GCCTCCAGGT TCCCACCCAA
241 CCCACATGGT GCCAGACTGA GCCTTCTCAG ATACTATCTC CTGGTGCACA CTTGGACAGC
301 ATTTCCTGTG TCAGACCCTG TTCTAAGTAC TTCCTATCTA TCTATCTATC TATCTATCTA
361 TCTATCTATC TATCTATCTA TCTATCTAAT CTATCTATCT TCTATCTATG AAGGCAGTTA
421 CTGTTAATAT CTTCATTTTA CAGGTAGGAA AACTGAGACA CAGGGTGGTT AGCAACCTGC
481 TAGTCCTTGG CAGACTCAGG TTGGAACACT GCCCTGGAGT GTGTGCTCCT GACCACCACG
541 AAGTGCCTCC TCTGTACAAT CTGACCCCAT CACTCTCCTC TTTACAATGA CCTCCCAATA
601 GGTTAAGATG CAGTTATTCT TTCTCACTTT AAGACACCTT TACCTCCGGC TTCTGCCACC
661 TCCTCTGCTC CCCTGTGGCC ACTCCTCACA CCACTCCACA TCCCAGCTGT TGTCAAGTTC
721 TTTCAGTGTT CCAAATGATC TATGTTCTCT TTGCCTTTGA GCCTTGCATA TGTTCCTCCC
781 TCTGCCAGAA GCGCTGTTCC CCTTCCTTTC CCACCCTTCT GCCCGGCCAA CTCACCTTCA
841 TTCTTCCCAT CCCAGTTTAG AGGCCACCTT CTCGAAGCCT GGGTTGGGGG GACTCTTCAG
901 TGTTCCCAGG ACACCTTGTG CTTCCCCCAT AATCACTGGG TGATCATTG
```

Sequence 6: TPOX

```
  1 CCCAGCACAC ACCTTGCCTC TGGCTGGGAC CCCCTTTGCT GCTGGCCCTG CTCAGGCCCC
 61 ACAGCTTGAT CTCCTCATGT TCCCACTGCT GACTTCCCCA AGCTAACTGT GCCACAGAGT
121 GGGGGACCCC CTCCCGGCTC TCACAACCCC CACCTTCCTC TGCTTCACTT TTCACCAACT
181 GAAATATGGC CAAAGGCAAA AACCCATGTT CCCACTGGCC TGTGGGTCCC CCCATAGATC
241 GTAAGCCCAG GAGGAAGGGC TGTGTTTCAG GGCTGTGATC ACTAGCACCC AGAACCGTCG
301 ACTGGCACAG AACAGGCACT TAGGGAACCC TCACTGAATG AATGAATGAA TGAATGAATG
361 AATGAATGTT TGGGCAAATA AACGCTGACA AGGACAGAAG GGCCTAGCGG GAAGGGAACA
421 GGAGTAAGAC CAGCGCACAG CCCGACTTGT GTTCAGAAGA CCTGGGATTG GACCTGAGGA
481 GTTCAATTTT GGATGAATCT CTTAATTAAC CTGTGGGGTT CCCAGTTCCT CCCCTGAGCG
541 CCCAGGACAG TAGAGTCAAC CTCACGTTTG AGCGTTGGGG ACGCAAACAC GAGAGTGCTT
601 GGTGTGAGCA CACAGGAGGA GTCACGACAG AGCAGTGTAA GAGCCGCCAC GTGGGTCCCA
661 CACAGGGGGA GTCACGACAC AGCAGTGTAA GAGCCGCCAC GAGGGTCCCA CACAGGGGGA
721 GTCGCGACAC AGCAGTGTAA GAGCCGCCAC GAGGGTCCCA CACAGGGGGA GTCACGACAC
781 AGCAGTGTAA GAGCCGCCAC GAGGGTCCCA CACAGGGGGA GTCACGACAC AG
```

Sequence 7: TH01

```
  1 TTACCCTTGG GGTGGGGGTG TAGGATGCAG CTGGGGCTGC AGTTCCAGGC CACGGAGAGC
 61 CTGTGAGGCT GGGCCCCGGG GCGCCCTGGG GAGGGGATGC CTGATGGGGA GCCTGGTGGG
121 GGAGGGTAGG GGAGGGCGGG GGAGGACGGG GGAGGGCGCC CTGTGTCCCT GAGAAGGTAC
181 CTGGAAATGA CACTGCTACA ACTCACACCA CATTTCAATC AAGGTCCATA AATAAAAACC
241 CATTTTAAAT GTGCCAGGGA GCCCAAGGTT CTGAGTGCCC AAGGAGGCAC CGAAGACCCC
301 TCCTGTGGGC TGAAAAGCTC CCGATTATCC AGCCTGGCCC ACACAGTCCC CTGTACACAG
361 GGCTTCCGAG TGCAGGTCAC AGGGAACACA GACTCCATGG TGAATGAATG AATGAATGAA
421 TGAATGAATG AGGGAAATAA GGGAGGAACA GGCCAATGGG AATCACCCCA GAGCCCAGAT
481 ACCCTTTGAA TTTTGCCCCC TATTTGCCCA GGACCCCCCA CCATGAGCTG CTGCTAGAGC
541 CTGGGAAGGG CCTTGGGGCT GCCTCCCCAA GCAGGCAGGC TGGTTGGGGT GCTGACTAGG
601 GCAGCTGGGG CAGAGGGAGG CAGGGGCAGG TGGGAGTAGG GTGGGGGCTG GGTGCAGCAG
661 CCGGGGACCT CTGGCCATCT TGGATTTTTT GGATGGATTT GTTTCCACAT TCCGATCGTT
721 AAGATTCAAG ATGAAACAAG ACACAGAGAC CCACACGACC CCCGAG
```

Sequence 8: vWA

```
  1 AGATGATAGA TACATATGTT AGACAGAGAT AGGATAGATG ATAGATACAT AGGTTAGATA
 61 GAGATAGGAT AGATTATAAA TAGATACACA GGTTAGATAG ATTAGACAGA CAGATAGATA
121 CATACATAGA TATAGGATAG ATAACTAGAT ACAATAGAGA TAGATAGATA GATAGATAGA
181 TGATAGAGGA TAGATGATAA ATAGATATAT AGCTTAGATA GAGATAGGAT AGATGATAGA
241 TACATAGGAT AGATAGAGAC AGGATAGATG ATAAATAGAT ACATAGGTTA GATAGAGATA
301 GGACAGATGA TAAATACATA GGATGGATGG ATAGATGGAT AGATAGATAG ATAGATAGAT
361 AGATAGATAG ATAGATAGAT AGACAGACAG ACAGACAGAC AGATAGATCA ATCCAAGTCA
421 CATACTGATT ATTCTTATCA TCCACTAGGG CTTTCACATC TCAGCCAAGT CAACTTGGAT
481 CCTCTAGACC TGTTTCTTCT TCTGGAAGGT GGGAACTCTA CCTTATAGGA TCAGTCTGAG
541 GAGTTCACAA AATAATAAGG GCAAAGTGCC CGGCACATTG TAGGAGACTA GTAATGTCTA
601 TAAAATGAGG GGCTTGAAGT AAATGATCCC TCTAGTTCTC TCTACTGCTA ACATTCTAAG
661 ACCTCCTTTA CATTAATTGT TCTCAAGCCA CATCTCCCTC CCCTACAGGA CTTCTATTTA
721 TTTCTGATCA ATTTCATGAG TACAAATAAG T
```

Sequence 9: FGA

```
  1 ACTGAACATT TGCTTTTGAA ATTTACTATC TATGTACCGT TGGAAAATTT ACTTAATATC
 61 TCTGAATTTT TTTTCTTCAA CTGTGGAGTG AGGAAAATAA TACCTACTTT TAGGTAGATG
121 ATGGATATAA CACTTTTCTC TGCATATAGT AGACACTCAG TGCATAACTA TCGCCTTCCT
181 TTTCCCTCTA CTCAGAAACA AGGACATCTG GGACCACAGC CACATACTTA CCTCCAGTCG
241 TTTCATATCA ACCAACTGAG CTCTAACATT TTTCTGCAGA AGCTGGATAT GCTGTACTTT
301 TTCTATGACT TTGCGCTTCA GGACTTCAAT TCTGCTTCTC AGATCCTCTG ACACTCGGTT
361 GTAGGTATTA TCACGGTCTG AAATCGAAAA TATGGTTATT GAAGTAGCTG CTGAGTGATT
421 TGTCTGTAAT TGCCAGCAAA AAAGAAAGGA AGAAAGGAAG GAAGGAGAAA GAAAGAAAGA
481 AAGAAAGAAA GAAAGAAAGA AAGAAAGAAA GAAAGAAAGA AAGAGAAAAA AGAAAGAAAG
541 AAACTAGCTT GTAAATATGC CTAATTTTAT TTTGGTTACA GTTTAATCTG TGAGTTCAAA
601 ACCTATGGGG CATTTGACTT TTGGATAATG TTATGCCCTG CAGCCTTCCA TGAATGCCAG
661 TTAAGATGTC CTAATAGCAA TTAGTAATCC CAAAGAAATA TAGAAGAAGA ACTTTCTTTG
721 GAATTTTAAA GGTGTAATTT GGAGTTAAAA TAGTTGGTTT GATTGCATTT CAATTATTTT
781 ATAACATCCT TAATCAAGGG ACTTGAACAT ATTGGATTTT CTTACTGATG AGCTTTTCTT
841 TTTAATCTAT AGATTTGAAA TGGTTCCTAA GCTGTTTTGG GTCAACAGGA TCACTCACTT
901 GCCAGCTAGT GTTGCATCAC TGATTTTAAA TGTCAAGTGT TTGTG
```

Sequence 10: D21S11

```
  1 GTTGGCTGGG GCTCAGAGAG AACAAAAAGG CAGAGGAAAA ACAAATTTCC CCTCTCACTT
 61 CTGGAGATGG AACACTTTTC TTCTGCTTTT GGACATCAGA AATCCAAGTT CTCTGGCCTT
121 TGGACTTTGG GACTTGTGCC AGCACCCTCC TGGGTTCCCT GGCCTTTGGC CTCAAACTGA
181 AGGTTACACT ATCAGCTTCC GTTGTTCTAA GGGCTTCAGA CTTGGACAGC CACACTGCCA
241 GCTTCCCTGA TTCTTCAGCT TGTAGATGGT CTGTTATGGG ACTTTTCTCA GTCTCCATAA
301 ATATGTGAGT CAATTCCCCA AGTGAATTGC CTTCTATCTA TCTATCTATC TGTCTGTCTG
361 TCTGTCTGTC TGTCTATCTA TCTATATCTA TCTATCTATC ATCTATCTAT CCATATCTAT
421 CTATCTATCT ATCTATCTAT CTATCTATCT ATCTATCTAT CGTCTATCTA TCCAGTCTAT
481 CTACCTCCTA TTAGTCTGTC TCTGGAGAAC ATTGACTAAT ACAACATCTT TAATATATCA
541 CAGTTTAATT TCAAGTTATA TCATACCACT TCATACATTA TATAAAACCT TACAGTGTTT
601 CTCCCTTCTC AGTGTTTATG GCTAGTAATT TTTTACTGGG TGCCAGACAC TAATTTTTAT
661 TTTGCTAAGT GGTGAATATT TTTTATATCC TTAAAAATAT TTTTGAGTGT TGATCTGGGT
721 AAAGTTAAGT TCAATATTGG AAAAATATTG ATTCTTTTGA GGATAGTTAT CTTCTAATTA
781 GTCTACCTGT TGCCCCATAA ATGGCATGAT TTTCCACTCT GTG
```

Sequence 11: D8S1179

```
  1 TACTACAGCA AGAGCGCTTG AACCAGATGT AGGGGAGATA GCAGCTGGAG AGCATAACAG
 61 AGGCACTGAC ATGTGAGCAG CTAACGAGGC CTTTTACAAG ACATCTGTGA CCACACGGCC
121 AAGTAGAAGA AAGCCGTTAA AAGCATCAAG GTAGTTAGGT AAAGCTGAGT CTGAAGTAAG
181 TAAAACATTG TTACAGGATC CTTGGGGTGT CGCTTTTCTG GCCAGAAACC TCTGTAGCCA
241 GTGGCGCCTT TGCCTGAGTT TTGCTCAGGC CCACTGGGCT CTTTCTGCCC ACACGGCCTG
301 GCAACTTATA TGTATTTTTG TATTTCATGT GTACATTCGT ATCTATCTGT CTATCTATCT

361 ATCTATCTAT CTATCTATCT ATCTATCTAT CTATTCCCCA CAGTGAAAAT AATCTACAGG
421 ATAGGTAAAT AAATTAAGGC ATATTCACGC AATGGGATAC GATACAGTGA TGAAAATGAA
481 CTAATTATAG CTACGTGAAA CTATACTCAT GAACACAATT TGGTAAAAGA AACTGGAAAC
541 AAGAATACAT ACGGTTTTTG ACAGCTGTAC TATTTTACAT TCCCAACAAC AATGCACAGG
601 GTTTCAGTTT CTCCACATCC TTGTCAACAT TTGTTATTTT CTGGGTTTTT GATAATAGCT
661 GTGAAAGGAA AATAAAAACT TGGGCCGGGC GCGGTGGCTC ACGCCTGTAA TCCCAGCACT
721 TTGGGAGGCC AAGGCGGGCA GATCTCAAGG TCGGGAGATT GAGACCATCC TGGCTAACAT
781 GGTGAAAACC CATCTCTACT AAAAATACAA AAACAAAAAA TTAG
```

Sequence 12: D18S51

```
  1 CATGCCACTA AGCTGTACAC TGAAAAACGG TTAACATGAT AAATTTTATG TTACATACAT
 61 TTTACCACAA TTTAAAAAAA TTATTAAAAA ATACTAACAA TAGGCCAAGC GTGATGGCTC
121 ACACCTGTAA TCCCAGCACT TTGGGAGGCT GAGACAGGTG GATCAATTGA GCTCAGGAGT
181 TTGAGACCAG CCTGGGTAAC ACAGTGAGAC CCCTGTCTCT ACAAAAAAAT ACAAAAATTA
241 GTTGGGCATG GTGGCACGTG CCTGTAGTCT CAGCTACTTG CAGGGCTGAG GCAGGAGGAG
301 TTCTTGAGCC CAGAAGGTTA AGGCTGCAGT GAGCCATGTT CATGCCACTG CACTTCACTC
361 TGAGTGACAA ATTGAGACCT TGTCTCAGAA AGAAAGAAAG AAAGAAAGAA AGAAAGAAAG
421 AAAGAAAGAA AGAAAGAAAG AAAGAAAGAA AGAAAGAAAA AGAGAGAGGA AAGAAAGAGA
481 AAAAGAAAAG AAATAGTAGC AACTGTTATT GTAAGACATC TCCACACACC AGAGAAGTTA
541 ATTTTAATTT TAACATGTTA AGAACAGAGA GAAGCCAACA TGTCCACCTT AGGCTGACGG
601 TTTGTTTATT TGTGTTGTTG CTGGTAGTCG GGTTTGTTAT TTTTAAAGTA GCTTATCCAA
661 TACTTCATTA ACAATTTCAG TAAGTTATTT CATCTTTCAA CATAAATACG CACAAGGATT
721 TCTTCTGGTC AAGACCAAAC TAATATTAGT CCATAGTAGG AGCTAATACT ATCACATTTA
781 CTAAGTATTC TATTTGCAAT TTGACTGTAG CCCATAGCCT TTTGTCGGCT AAAGTGAGCT
841 TAATGCTGAT CAGGTAAATT AAAAATTATA GTTAATTAAA AGGGCATAAA TGTTACCTGA
901 CTCAATAAGT CATTTCAATT AGGTCTG
```

Sequence 13: D3S1358

```
  1 CTGGTTTTGG TGGAATTGAC TCCCTCTGTC ACAAACTCAG CTTCAGCCCA TACCCTGAGC
 61 CATAGACCTA TCCCTCTAAT GCATTGTACT AGTCTCAGGG CTAATAACAA GGGAGAGGTG
121 TCAAAGGGCC AGTTCCACCT CCACCACCAG TGGAAAAGCT ATTCCCAGGT GAGGACTGCA
181 GCTGCCAGGG CACTGCTCCA GAATGGGCAT GCTGGCCATA TTCACTTGCC CACTTCTGCC
241 CAGGGATCTA TTTTTCTGTG GTGTGTATTC CCTGTGCCTT TGGGGGCATC TCTTATACTC
301 ATGAAATCAA CAGAGGCTTG CATGTATCTA TCTGTCTATC TATCTATCTA TCTATCTATC
361 TATCTATCTA TCTATCTATC TATCTATCTA TGAGACAGGG TCTTGCTCTG TCACCCAGAT
421 TGGACTGCAG TGGGGGAATC ATAGCTCACT ACAGCCTCAA ACTCCTGGGC TCAAGCAGTC
481 CTCCTGCCTC AGCCTCCCAA GTACCTGGGA TTATAGGCAT GAGCCACCAT GTCCGGCTAA
541 TTTTTTTTTT TAAGAGATGG GGTCTCGCTG TGTTCCCCAG CCTTGTCTTA AACTCCTGGC
601 CTCAAGTGAT CCTCCCATCT CAGCCTTCCA AAGTGCTGAG ATTACAGCAG AGGCTTTTAA
661 GTCAAAGCTT TCCCTGCTAG GACAAGCCCT AGTTAAAGTC CTGGAGCACT GGCCACTGCA
721 GCTGCACTTG G
```

Sequence 14: Penta D

```
   1 CCTACTCGGG AGGCTGAGGC AGGAGAATCG CTTGAACCCA GGAGGGGGCG ACTGCAGTGA
  61 GCCGAGATCG TGCCACTGCA CTCCAGCCTG GGTGACAGAG CGAGACTCCA TCTCAAAAAA
 121 AAAAAAAAAA AAACAGAATC ATAGGCCAGG CACAGTGGCT AATTGTACCT TGGGAGGCTG
 181 AGACGGGAGG ATCGAGACCA TCCTGGGCAC CATAGTGAGA CCCCATCTCT ACAAAAAAAA
 241 AAAAAAATTT TTTTTAAATA GCCAGGCATG GTGAGGCTGA AGTAGGATCA CTTGAGCCTG
 301 GAAGGTCGAA GCTGAAGTGA GCCATGATCA CACCACTACA CTCCAGCCTA GGTGACAGAG
 361 CAAGACACCA TCTCAAGAAA GAAAAAAAAG AAAGAAAAGA AAAGAAAAGA AAAGAAAAGA
 421 AAAGAAAAGA AAAGAAAAGA AAAGAAAAGA AAAGAAAAAA CGAAGGGGAA AAAAAGAGAA
 481 TCATAAACAT AAATGTAAAA TTTCTCAAAA AAATCGTTAT GACCATAGGT TAGGCAAATA
 541 TTTCTTAGAT ATCACAAAAT CATGACCTAT TAAAAAATAA TAATAAAGTA AGTTTCATCA
 601 AAACTTAAAA GTTCTACTCT TCAAAAGATA CCTTATAAAG AAAGTAAAAA GACACGCCAC
 661 AGGCTAAGAG AAAGTACTTC TAATCACATA TCTAAAAAAG GACTTGTGTC CAGATTAAAG
 721 AATTCTTACA CATCAATAAG ACAACCCAAT TAAAAATGGG CAAAAGATTT GAAGAGATAT
 781 TTAACCAAAG AAAACATATA AATGTGTCCG GGCGCGATGG TAATCCCAGC ACTTTGAGAG
 841 GCCGAGGCAG GCGGATCACT TGAGGTCAGG AGTTTAGGAC CAGTCTGGCC AACATGGTGA
 901 AACCCTGTCT CTAATAAAAA TACAAAAATT AGCTGGGTGT GGTGGCGTAA GCCTGTAATC
 961 CCAGCTGCTC AGGAGGCTGA GGCAGAAGAA TTGCTTGAAC CTGGGAGGTG GAGGCTGCAG
1021 TAAGCG
```

Sequence 15: Penta E

```
  1 CACATGTGGA CATTTCTTAT TTTCTCATAT TGGTGGTATG GCTCATTTAT GAAGTTAATA
 61 CTGGACATTG TGGGGAGGCT GTGTAAGAAG TGTTAAAGGG GATCAGGGAT ACATTCACTT
121 CTCTTTTCCT TTGCTAGTTC TGTGGTCTTA AGCAAAGTAG CCTCAAACAT CAGTTTCCTC
181 TTTTATAAAA TGAGGAAAAT AATACTCATT ACCTTGCATG CATGATATAA TGATTACATA
241 ACATACATGT GTGTAAAGTG CTTAGTATCA TGATTGATAC ATGGAAAGAA TTCTCTTATT
301 TGGGTTATTA ATTGAGAAAA CTCCTTACAA TTTTCTTTTC TTTTCTTTTC TTTTCTTTGA
361 GACTGAGTCT TGCTCAGTCG CCCAGGCTGG AGTGCAATGG CGTGATCTCG GCTCACTTCA
421 ATCTCCACCT CCTGGGTTCA AGTGATTCTC CTGTTTCAGC CTCCAGAGTA GCTGGGATTA
481 CAGGTGCCTA CCACCACACC CAGCTAATTT TTTGTATTTT AGTAGAGACG GGGTTTCACC
541 ATGTTGCCCA GGCTGGTCTT GATCTCCTGA GCTCAGGTAA TACACCTGCA TCGGCCTCCC
601 AAAGTGCTAG GATTGCAGGC GTGAATCACC GCACCTGTCC ACAATTTTCT TGTTATTGGT
661 ACCCTTTCAT GTTGGTAAAA TGTATTTTAT TTTCTCTTAT CAAATAATTT TCAATGCAAT
721 GAGACGTCAA CTTTAAGCCC AAAGTAGACC AGTAGTAAAA CTAAGGCTGA AACCATTGAT
781 TGATTATTAC CATATATTGT CCTAAAATAT TCGGCTTTTA AAACATTTGG TTTCATTTTT
841 CATGATAAAA ATATGTAGCA TTTTTGCACT TTTAATTCAC TTTGTAGAGT TCTCAATCAT
901 TTCTAACACA TGCTTGGCAA TGACAAGCCA TTTGTGAAAG AGTTTTGCTG GCTTTAAAAT
961 ATATGCAAAT GTAATAT
```

Sequence 16: AMEL X

```
  1 AGGTCTCCTC TTCTATACAG CACATTTGTT CAAACTAAAA ACAGACCTCA AGTATATTCT
 61 GCACTATATA GATTTTTTTA AAGTAGCTTC AGTCTCCTTT AATGTGAACA ATTGCATACT
121 GACTTAATCT CTTCCTCTCT CTTCTCTTCC TTCACTCTCT CCCTTCCTCT CTCTTTCTAT
181 TCTCCTCCCC TCCTCCCTGT AAAAGCTACC ACCTCATCCT GGGCACCCTG GTTATATCAA
241 CTTCAGCTAT GAGGTAATTT TTCTCTTTAC TAATTTTGAC CATTGTTTGC GTTAACAATG
301 CCCTGGGCTC TGTAAAGAAT AGTGTGTTGA TTCTTTATCC CAGATGTTTC TCAAGTGGTC
361 CTGATTTTAC AGTTCCTACC ACCAGCTTCC CAGTTTAAGC TCTGATGGTT GGCCTCAAGC
421 CTGTGTCGTC CCAGCAGCCT CCCGCCTGGC CACTCTGACT CAGTCTGTCC TCCTAAATAT
481 GGCCGTAAGC TTACCCATCA TGAACCACTA CTCAGGGAGG CTCCATGATA GGGCAAAAAG
541 TAAACTCTGA CCAGCTTGGT TCTAACCCAG CTAGTAAAAT GTAAGGATTA GGTAAGATGT
601 TATTTAAAAC TCTTTCCAGC TCAAAAAACT CCTGATTCTA AGATAGTCAC ACTCTATGTG
661 TGTCTCTTGC TTGCCTCTGC TGAAATATTA GTGACTAAGT GGTATA
```

Sequence 17: AMEL Y

```
  1 TTATTCTCCA ATATTTTGAA ATGTGAATAT TACAGTAATT TCCCTTGTCC AAATGAGAAA
 61 ACCAGGGTTC CAAAGAGAGG AAATTATTTG CCCAAAGTTA GTAATTTTAC CTAATCTTTA
121 CATTTTACCG GATGGGATAG AACCAAGCTG GTCAGTCAGA GTTGACTTTT TGCCCTTTCA
181 TGGAACCTTC CTGAGCAGTG GTTCATGAAT GAATAAACTT ACAGCCATAT TTAGGAGGAA
241 AGAGTCAATC CGAATGGTCA GGCAGGAGGG TGCTGGAGCA ACACAGGCTT GAGGCCAACC
301 ATCAGAGCTT AAACTGGGAA GCTGATGGTA GGAACTGTAA AATTGGGACC ACTTGAGAAA
361 CCACTTTATT TGGGATGAAG AATCCACCCA CTATTCTTTA CAGAGCCCAG GGGACTGCTA
421 ATGCAAACAG TGATCAAAAT TAGTAAAGAG AAAAATTACC TCATAGCTGA AGTTGATATA
481 ACCAGGGTGC CCAGGATGAG GTGGTAGCTT TTATAGGGAG GAGGGGAGGA GAAGAGAAAG
541 AGAGAGGAAG GGAGAGTGTG AAGGAAGGGA AGAGAGAGTA AGAGATTAAG TCAATATGCA
601 ATTGTTAACA TTAAGAGAGA CTAAAATTAC TTTTAAAAAA TCTATATAGT ACAGAATATA
661 TTTGAGGTCT GTTTTTCGTT AAAACAAGTG TGCTATGTAG GAGAGGAGAC TT
```

Sequence 18: D2S1338

```
  1 ACAAGGCACG GAACTCACAC CCAGCCTCTC TCCATACAAC AGAATATGGG TTCTTGCGGA
 61 GCTGGACTCT GCAGGAGTCT ATCTAATATG GACTCTGTGT CAATGACTCC TGGGCCTCCT
121 CTGATCACCC CATTAAAGTC CTTCGATTGC TTTGAGCCTC AAATCTATGT GACATCAATA
181 CGTTCATTTC TTCCTAGCAC TTAGAACTGT TTCTTGTTGA TACATTTGCT GGCTTCTTCC
241 CTGTCTCACC CCTTTTCCTA CCAGAATGCC AGTCCCAGAG GCCCTTGTCA GTGTTCATGC
301 CTACATCCCT AGTACCTAGC ATGGTACCTG CAGGTGGCCC ATAATCATGA GTTATTCAGT
361 AAGTTAAAGG ATTGCAGGAG GGAAGGAAGG ACGGAAGGAA GGAAGGAAGG AAGGAAGGAA

421 GGAAGGAAGG AAGGAAGGAA GGAAGGCAGG CAGGCAGGCA GGCAGGCAGG CAAGGCCAAG
481 CCATTTCTGT TTCCAAATCC ACTGGCTCCC TCCCACAGCT GGATTATGGG CCAGTAGGAA
541 TTGCCATTTT CAGGGTTTTG CTGTCACTGT AGTCAGGACC ATGAAGTCTT TAGGCACCTC
601 CACTCCACAC ACCCCCTGGT GAGAGCTCCC ATCTCCCTGT TCTGAAACAG CTCCCCAATA
661 TAGTACTGAT TCCGGTTAAA CTTGAACCCC TGCCCCTGCC CCTGCCCCTG ATTTACATGA
721 GGACACTGAG GCCCAGAGGG GTAAAGTGAC TGCCAGGGGT CACACAGCTA GAAAGTGGCG
781 GTGCCAGAAC TGGAAGGAGG CCCTCATTCC TGAGTCACGG CTTTTCCATA GCACAGCCTT
```

Sequence 19: D19S433

```
  1 ATGAAACTGG ACACAGAAAC CAGACCCCAG AGCACATACC GTATGAGTCC ATTGGTATGA
 61 AGTTTAAAAA CAGATGGCAC TAGTCCAAAG GATTGGAAGT TGGAATAGTG GTTACCAGGA
121 CTGGGGGGAG GAAGGGATGG TGGATGGTGA ACAAAAGGAC CTTGGAGGGC TCCTGGGGTT
181 CTAGGAATCA ATCTTCCTTC TTTCCTTCCT TCCTTCCTTC CTCTTTCTCT CTTTCTTTCT
241 GTTTTTATTT CAATAGGTTT TTAAGGAACA GGTGGTGTTG GTTACATGAA TAAGTTCTTT
301 AGCAGTGATT TCTGATATTT TGGTGCACCC ATTACCCGAA TAAAAATCTT CTCTCTTTCT
361 TCCTCTCTCC TTCCTTCCTT CCTTCCTTCC TTCCTTCCTT CCTTCCTTCC TTCCTTCCTA
421 CCTTCTTTCC TTCAACAGAA TCTTATTCTG TTGCCCAGGC TGGAGTGCAG TGGTACAATT
481 ATAGCTTTTT GCAGCCTCAA CCTCCTGGGC TCAAGTGATC TTCCTGCCCC AGCCTCCTGA
541 GTAGCCAGGA CTACAGGAAT GTGCCAACAT GCCTGGCTAA TTTTAAAAAA TTTTTTATAG
601 AGAAGAGGTC TCACTATGTT GCCCAGACTA GACTTGAACT CCTTCCCTCA AGTGATCTTT
661 CTGCATCAGT CTTCCAAAGT GCTGGGATTG CAGGCATGAG CCACCTCACC CAGCCTTAGA
721 AATGTTCTGT TTCTTGACCT GAGAGCTGGA TATACAGGAT TGCTCACTTT GTGAAAATTC
```

Sequence 20: ACTBP2SE33

```
  1 GTACTTCAGA GTCAGGATGC CTCTCTTGCT CTGGGCCTCC TTGCCCACAT AGGAGTCCTT
 61 CTGACCCATG CCCACCATCA CTCCCTGGTG CCTAGGGTGC CCCACAATGG AGGGGAAGAC
121 GGCCTGGGGA GCCTTGCGCA TGCTGGAGCA GTTGTCGACG ACGACGAGCG CGGTGATAGC
181 ATCATCCATG GTGAGCTGGC GGCGGGTGCG GACGCAAGGC GCAGCGGCAA GGACAAGGTT
241 CTGTGCTCGC TGGGCTGACG CGGTCTCCGC GGTGTAAGGA GGTTTATATA TATTTCTACA
301 ACATCTCCCC TACCGCTATA GTAACTTGCT CTTTCTTTCC TTCCTTTCTT TCTTTCTTTC
361 TTTCTTTCTT TCTTTCTTTC TTTCTTTCTT TCTTTCTTTC TTTCTTTTTC TTTCTTTCTT
421 TCTTTCTTTC TTTCTTTCTT TCTTTCTCTT TCTTTCTTTC TCTTTCTTTC TTTTTCTTTC
481 TTTTTCTTCC TTCCTTCCTT TCTCTCTCTC TCTCTTTCTT TCTTTCTAAC TCTCTTTGTC
541 TCTTTCTTTC TTTCTTTTGA CGGAGTTTCA CTCTTGTCGC CCAGATTGGA GTGCAATGGC
601 ATGACCTCGG CTCACTGTAG CCTCCACCTC CCAGGTTCAA GCGATTATCC TGCCTCAGCC
661 TCCCTAGGAG CTGGAATTAC AGACGTGCAC CACCAAGCCT GGCTAATTTT TGTATTATTA
721 GTAGAGACGG GGTTTCACCT TGTTGGCCAG GCTGGTCTCG AACTCCTGAC CTCAGGTGAC
781 CCACCTGCCT TAGGCTCCCA AAGTCCTGGG ATTATAGGCA TGAGCCACAG TGCCCAGCCT
841 TCTTTTCATT TAATACTATA GTAGTGTGAT CCTCTCTACC TATTACA
```

Sequence 21: D10S1248

```
  1 TTCTGTTTTG CGGTGGTTCC TAGTATGGTA CCTGGCCAAG GGCACACTAG ATCTTTGTCA
 61 AGGTAATGAC TACTTTTTAT TAAATGCTTT CCATGTATCA AGTTCTGTGC CAAGCACTTG
121 ACATATATCA TTTTATTTTA TCCCGTGAAG TAGTTATTGG TATCTTCATT TACAAATAAA
181 AAAACAAGCT TAGTACTTAA CTCACTGCCT TGAACATAAT TATTGCTTTA AAGGTAGCTA
241 GGATTCTTAA TAGCTATTAT TACCAAAGCA TGAACAATCA GTAAAAAGCA AACCTGAGCA
301 TTAGCCCCAG GACCAATCTG GTCACAAACA TATTAATGAA TTGAACAAAT GAGTGAGTGG
361 AAGGAAGGAA GGAAGGAAGG AAGGAAGGAA GGAAGGAAGG AAGGAAGGAA ATGAAGACAA
421 TACAACCAGA GTTGTTCCTT TAATAACAAG ACAAGGGAAA AAGAGAACTG TCAGAATAAG
481 TGTTAATTAT AATATCCAGG GGTGGGATAC AGAGGTTTTA GCATCTGCTC TTTGCCAAGC
541 ACTGCACTTA TTCCTGAGGA ATACCTGAGG GAAAAAGTAT GGTTTCTCAC AGGATCTAGT
601 TGGACTGGAA ATATGACATT CATATTGGAA TCCAGTGTCT TTTTCTGAAA AAGAGAGTTC
661 GTTCCAAGCT TAGCTCACAT GCAAGCTAAG ACAACCACTA GAAATTACTC TCCCCAGGGC
```

Sequence 22: D1S1656

```
  1 GTCATGCCTA CAGTGTAACG GGAATTGACC AGGTAGGCGA CTTGAACTCC AACTGCAGGC
 61 TATGGGGAGA CATGTGACAA TGCTAATCCC TTAGGCATTT ATTCAGTGCA TTGCAGTTTA

121 AATGTCTGCC TTTCAGGCAT TTCAGAGATT ATGTCACCTA AAGAGGCAGG CTGGAATTCA
181 AAACGGCAAG CCAGGAAAGA GAGAAACCAT GTGATTCCAC CGCAGCACAA AACTCGTTTA
241 GCAGCTGTAA GCGCCTGGTC TTTGTTTATT TTTAATTTCC TTTCTTTCCC AATTCTCCTT
301 CAGTCCTGTG TTAGTCAGGA TTCTTCAGAG AAATAGAATC ACTAGGGAAC CAAATATATA
361 TACATACAAT TAAACACACA CACACCTATC TATCTATCTA TCTATCTATC TATCTATCTA
421 TCTATCTATC TATCTATCTA TCTATCTATC TACATCACAC AGTTGACCCT TGAGCAACAC
481 AGGCTTGAAC TTATATGGGG ATTTTCTTCC ATCTCTACCA CCCCTGAGAC AGCAAGACCA
541 ACTCCTCCTC CTCCTTCTCA GCCTACTCAA CATGAAGATA ATAAGGATGA AGACCTTTAC
601 AATGACCCAG TTCCACTTAA TAAATAGTAA ATGTATTTCC TCTTCCCTAT GATTTTCTTG
661 ATAACATTTC TTTTCTCTGG CTTATTTATT GTAAGAATAC AGTATATAAT ATAAATAATT
721 ATAAAACATG TTAATTGGTT CTTTACGTTA TCGATAAGAC TTCTGGTCAA TGGTAGGCTA
```

TscI McrBC (half site)
752: Partially methylated BspDI ClaI

Sequence 23: D22S1045

```
  1 GAGCCCAAGT TTAAACCCAG GCCCTCTGTG TCCCCCTACA GGGTGACTGC ATCTCCGAGT
 61 CCTGGCTTGT CATGCCTGAC AGAGGGCTGC CGAGTGAGCA GCTTAAGGCA TCCTGCCACT
121 GTGCAGCTGC CAACCCTACA GCCCGGCAGC CCTGCGGGAG GAAGCTCTAG TGCAGGCCTC
181 TTAGGATCTG GGGTCCAGGA TGCTGATTTC AGGGCCGGGA CCTTGGGCAC CGTCCCTCTG
241 GTCTGCATAA GACCCACTAT GGGCAAACCT TAAACCTGAT CGTTGGAATT CCCCAAACTG
301 GCCAGTTCCT CTCCACCCTA TAGACCCTGT CCTAGCCTTC TTATAGCTGC TATGGGGGCT
361 AGATTTTCCC CGATGATAGT AGTCTCATTA TTATTATTAT TATTATTATT ATTATTATTA
421 TTATTATTAC TATTATTGTT ATAAAAATAT TGCCAATCAT ACATTCGCGT GATCACTCAC
481 ACTGTGCCGG GCACTCTTGA GAGCACTTTA CATATATTGT CTCATTTAAT TCTCTCAACT
541 TGGGCACAGG CACTGTCACT ATTTCCATTC TACAGCTGAG GAGACTGAAG CACAGAGAGC
601 CTTAGGGACT TGCCTGAGGT CACACAGCTA AGAAATGGTG GAGCCAGGAT CAGAAACCAG
661 GCCACCTACA GAGCTCCCTG CAAGGGGAAC AGCATCCGGT TCCAGAGGCT GTGATTTTAT
721 CAGCTACACT GTGTGACTCC ATCTTCACAC TCTCCTGCCC CTCAAGAAGA CATATAACCT
```

Sequence 24: D2S441

```
  1 ATGAAGAGAT GGTCAGGCGA GGTATGGGGG AAGGGGCGTG GAGCTTCCAT GTCCTCCCTG
 61 GGCGCCACCC TCCAGGAACC TCCACGTGTT CAGCTATACA GAAGCTTCCT GAACCCAGTC
121 CTCTTGGGGT TTGAGGGAAG CTTCATGACA TCAGCATTCC TTCCTCCAGG GTATTAATGG
181 GACCCTCTCT GAAGAGATTC TTAAGACCCA CGGCCAGAAA GTTGGGTAAA GACTAGAGTC
241 CTGCCTTGGG GCAGGTGAAA GGAGTGCAAG AGAAGGTAAG AGAGATTCTG TTCCTGAGCC
301 CTAATGCACC CAACATTCTA ACAAAAGGCT GTAACAAGGG CTACAGGAAT CATGAGCCAG
361 GAACTGTGGC TCATCTATGA AAACTTCTAT CTATCTATCT ATCTATCTAT CTATCTATCT
421 ATCTATCTAT CTATATCATA ACACCACAGC CACTTAGCTC CAATTTAAAA GATTAATCAT
481 AAACATTTGG GAAGGAGAGT GAAGATTTTT GTGATGTTAA ATAAGAATGA TTATACTAAA
541 AACCAAAATA ATATGTTATT TATGGCTGGG TGTGGTGGCT TAAGCCTGTA ATCCCAGAAC
601 TTTGGGAGGC CAAGGCTTGT GGATCACTTG AGCCCAGAAG TTCAAGACCA GCCTGGGCAA
661 CATAGGGAGA CCCTGTCTCT ACAAAAAATT TTAAAATTAG CTGGACATGA TGGCACGCAC
721 CCGTAGTCTC AGCTACTCAG GAGGCTCACG CCACTGCATT CCAGTCTGGG TAACGCACAC
```

Sequence 25: D12S391

```
  1 GTCAGGAGTT CGAGACCAGC CTGGCCAACA TGGCGAAACC CTGTCTCTAC TAAAAATACA
 61 AAAAAATTAG CTGGGCATGG TGGTGTGTTC CTGTAACCCC AGCTACTCAG GAGGCTGAGG
121 CAAGAGAATC GCTGGAACCC AGGAGGTGGA AGTTGCAGTG AGCTGAGATT GCACCACTGC
181 ACTCCAGTGT GGGCAACAGA GCGAGACTCT GTCTCAGAAA AAAAAAAGAA TACATGAAAT
241 CAGAGAAACT CAAATTGTGA TAGTAGTTTC TTCTGGTGAA GGAAGAAAAG AGAATGATAT
301 CAGGGAAGAT GAAAAAAGAG ACTGTATTAG TAAGGCTTCT CCAGAGAGAA AGAATCAACA
361 GGATCAATGG ATGCATAGGT AGATAGATAG ATAGATAGAT AGATAGATAG ATAGATAGAT
421 AGATAGACAG ACAGACAGAC AGACAGACAG ACAGATGAGA GGGGATTTAT TAGAGGAATT
481 AGCTCAAGTG ATATGGAGGC TGAAAAATCT CATGACAGTC CATCTGCAAG CTGGAGACCC
541 AGGGACACTA GGAGCATGGC TCAGTCCAGG TCTAAAAGCC AAAAAACCAG GGAAACTGAT
601 GGTGTAATTA TCCATCCCAG GTGGAAGGCC TGAGAACCTG GAGTGCCCCT GGTATAAGTC
661 CCAGAGTACA AAGACAGGAG AGCCTGGAGT TCTGACTTCC AAGGGCAGAA GAATGTGTCG
721 CAGCTCCAGG AGAGAGAGAG AAAGAATTTC TTTCCTCCGC CTTTTGATTC TATCTGGGGG
```

Sequence 26: ADD6 (from ncbi accession NT_022135)

```
16664701 cttgaacctg agaggcagag gttgcagtga gccgagacca tgccattgca ctccagcctg
16664761 ggcaatagag taaaactcca tcctcccgct ccaaaaaagt agacaacgtc catgaggtga
16664821 tgaggaaggg gttatcgtgt gttgcttgct gagaacagga cccccagact caccgtgtcg
16664881 acgccggcca gcagcatctc agtcacgttg gcgtagatct cctgcagcgt cagagcctgg
16664941 ctaaggaaga ggtatgtgag aagtcccccg ctcaccctcc ggcctcggtc catttggtac
16665001 tgtatgtccc tcaacttgtt gtcaacatga atttggcctg tttgaaaaca gtatttcttt
16665061 tgaaaggagt ttgggttgag aatcatcttt tcagtctcaa agccctctgt cctcccagta
16665121 gcttaactaa accagtggca ggtgacagag ggtaaggaaa cccaatttat ctaacgtcaa
16665181 cctgggagtt tcactcatac acttgcttat gtaaatgaat gaaaagttaa aagacaagct
```

Sequence 27: ADD10 (from chromosome 17:3477839-3478292)

```
  1 AGAGAGCCCA GGAGACAGGC AGAAAGGAAG GCATGTGACC GGATCACAAT CATCAGCTCT
 61 CTGCTGTCCT CTTTGGGAAG GGTTTTAGTA TTAAAAGGAC ATTTATTCTC ATTAATGCAA
121 AATTAAGGAG TTTTAAAAGC TTTTACAACC TAGACTCCCT CTGAGAGGTT AGCCTTGACA
181 CCCTAATCGC CTTCTGCTCC CGCCACTGCT CGGTGCCAAG CAGCTCCCAC GGCCCCGGCG
241 GGTCTGATGA TAGCCGGACA GGAGGGAGGA AGGGGAGGAG GAAGAGCCTG CATCAGCTCC
301 TACGATTGCC CAGCCCCATC CTGGGAGTGA TTAAACGGTG CATCACCAAA TGCCAGTCCC
361 ACTGACAGGC AGGTCACCGT GCACTTCAGG GCACTCTAAA TTGCCGACTC TCCATGTAGA
421 GAGGGATGAA TCCAATATTG AAATCCTCAT AACTACAGCC CCCCAAAGTA GCCGTCCATC
481 TTCTGCTTAA AATGTTGATC TGTAGTAAAA TGTTGATTTT GTTGAAGCTG AGTGATG
```

Sequence 28: ADD17 (from chromosome 1: 50149332-50149574)

```
  1 TTGAACCTAG GAGATGGAGG TTGCAGTGAG CTGCGATCAT GCCACTTCCC TCCAGGCTGG
 61 GCAACAGAGC GAGACTCCAT CTCAAAAAAA CAAAAAAGAA AACCAACCTT TTGAATGTAG
121 GGGAAACTTT TCAAAGGATA TCTAGTTTTC AATTACAGTA AACTTGTGGA AGGGAGGTTC
181 AGAGTTGAGA TTGAGATTAT AGATTTTGCT GATGATAAAC CATGAGTTCC AGAGGACATA
241 GTAGACTATT CTGGGCAGTT ATACAGGGGT GGATGGAATG TGGGAGTGGG GTTGTATAGT
301 GCCATAAAGA AATGAGAGTC CGGATTAAAA ATAATGAGCT GGACTCGCGA GCCTTTTGTA
361 ACTGAAATAA ATAGAAAAAT AAGAAATACA TTATTTCTGT GATTGTTGAG AGGAAGAAAT
421 GGTGGAAATC TTGTGAGAAG CACACTGAGC TCTAGCACCA CCTCTTCACT CCTACAGATG
481 GTGGAATAAA CGGCAGGCAA GTTCAAAATC ACATATAGTC ATTATTGCAA GATAGTTCTA
541 TGGATATAGA TACTACATAC AATATAAATC ATGCTCATTG AATGGTTCAG TGGAAACTAC
601 TCTGAACTT
```

Sequence 29: Hypo23(from ncbi accession NM_004907)

```
123161 GAGTTTGGGA AGGGTATTTT AGGGGGGAAT AACTTTTGAG TTCCCAGCGT GCGGGGGAAG
123221 GGCGGGACGG GAGGGTGTCC CAAGGCCTGA GAAGATCAGT GTGGGGCAGG GGTCAGGAAT
123281 AACCTGGGAG GGGGCCTTGT ATGGGGGAAA TAATTGGGAA GAGGAGAGAT GGGATGAAGG
123341 GGGCCTCAGC GGGTCGTCTC CTGTGTATGC AGGGTCGTTC TGCAGCGTCT CTGGGAGATG
123401 GCGTCCCTGG GAGCCCTCAG GTCGCCCCTA CCCGCTGCGG GGTGCTTTCC TGGCGTCACG
123461 CCTTCCTGGC CCCTGGAGGG AAGGAAGTGA AACTCTCCTC TTCCCCCACC CGGCTGGAAT
123521 GCGAGTCAGG AAGCCTGGGG CTCCAGCCTG CTCCGGCTGC CCGGGTCGGG GATGGGGAGG
123581 GGCGTGGCCG GAGCGCAAAG CCCCGCCCCT CCGCGCCCCC CCCCCGGAAG CCCCGCCGCC
123641 GGCCGCTAAG GCGATCACGG GCCCTGTCCT AATATGGGCA ACCGGAAGCG GCCCGCGCGA
123701 CTGCCCTACG TCACTCCGTC CAAATTTAGT TGTGGAAGTC AGCGGGCGCT GGTGGCGGGA
123761 AGGCGCCCGCG AGCCAGTGCG GGCGGAAAGG GGGCGGGGGG CGCACCACCC CTTAAAGGGC
123821 CCGCACCAGG AATGAATGGA GCCATTCGAA CAATTCTGCA TCCTATTTTT GGAGGAAGTG
123881 GAATTAGTAT TT
```

Sequence 30: Hypo28 (from chromosome 5: 85949232-85949719)

```
949232 TTTATTTTTA AAAAAGAAAG AAAGAAGAGA AAAGGGATGG GTTTATTGTC CTTTTCAACA
949292 GACTAGAGTA TACGGGGTGA AACTGCTTCA CTTGATTCAA TAAAATCGTT TCCGGTAACA
949352 GGCCCCAGGA ATCCTAGACC TAAGCCTGGC GCGAAACTAC ATTTCCCACA ATCCTTCGGG
949412 GGCTGATAAG GCTCCGCAAT GGTCTGAACT ACAATTCCCA CAATCCAGGG CGATTTCCGC
949472 TTTGTCGCGT TTCCTCAAGG CTCCGCCCCA TTTCCCATCT TTCTTTTCAG TCCTTGCGCA
949532 CCGGGGAACA AGGTCGTGAA AAAAAAGGTC TTGGTGAGGT GCCGCCATTT CATCTGTCCT
949592 CATTCTCTGC GCCTTTCGCA GAGCTTCCAG CAGCGGTATG TTGGGCCAGA GCATCCGGAG
949652 GTTCACAACC TCTGTGGTCC GTAGGAGCCA CTATGAGGAG GGCCCTGGGA AGGTTAGTGT
949712 GTAAGGGG
```

Sequence 31: Hypo33 (from chromosome 6: 34211067-34211314)

```
211067 ACCCTCATTT CACATTTCAC CCCTTCCTCA AAATGCTCCC TTCATATTAC CTCCTCAGAA

211127 ACCAAGAATA TGGCTACTAA TTCTCCCTGG CCCCATGCTG CAGGTGAACC GGTAGCCCAG
211187 AGGTATCACA TAATTCTCCC AAAGTCACAC AGCAAATCAA GATGCATCCA GGACTAGAAG
211247 CCATGTCAGC CACACTGGGA AGCCCCAGCG AAGCTGACAG AAAGTTTCAT AATACCACCC
211307 TCTCCCCT
```

Sequence 32: OCA2 sequence: (from chromosome 15: 25276967-25277446)

```
6967 aaacacccca gtctgaaaat aaccatagtt tgttgctctt acgagtgaaa atgctatttc
7027 atacacgaag ctttgtcctt cagcacccaa gatttaagga taattatgga tgaatattat
7087 ggattcattt taaatccttt ggcaaatctg ctctgggggc ttctctgtca gaaggtctct
7147 ccttcccaac tctaagaaac gttattccta tgcaaatgct gctgagtcaa gacggggagg
7207 gaagtgcaga gagaagggct ggtggcatgg tcagtaagtc atgagggtga gattaggggt
7267 gacacactgc ttgccaacgt aggagaaggc tctgccctca cctagcaggt ctgatggaag
7327 ccccttattc cgtccttcct gccgggttcc accgagatcc aaaaaggaat gctgtgtagg
7387 agcacatgat atgtgataaa tgagagaaag gtcaaacatt taaggaacgc ccagagaaag
```

Sequence 33: SD1 (chromosome 11: 72085699 - 72086161)

```
72085699 CGCTGGGCGC GAGTGAAGAG CCCATCAGGC AGGTCGCGCA GGAAGCGCTT GAGCGCCGAG
72085759 GAAACATCAT CCACGTGCTG CTCGCCCTCC TTGAGGTGCA CAGAGCGCGC ATCCTGCCGC
72085819 AGGCTCTCCA GCAGCCGCTG TGTCTTCGAT GTCTGCCCAC ACTTGCGGTA GATGCCCTCG
72085879 GAGGTCAGGC CTAGGGAGGG GCGGGGCCAA GCGTTCGGGG CCTGAGGCAT AGAGTCATGG
72085939 GGCGGGGCCG CGCAGCTCTG GGGCGGGAGG CGGCTCTCCG GGAGGGGCGG GGCTGGCACC
72085999 CTAGGGGCAG GGCTCACCGC ACTGCGTGAT GTAGTCCACA CAGCGGTACA CGATCACCGG
72086059 GATATCCGAG TCCCCAAGCT GCTGCTCCGA CAGCGTGTCC CCCATGCTGG CGGCTGCTTT
72086119 CTGGATGGCC CCCAGCCAAC CCATGAAGTC CAGCCGCCGC TCG
```

Sequence 34: SD2 (chromosome 4: 25286907 - 25287230)

```
25286907 CGATCCCGTT CACTCGCCTG CCCATCCGCA TGGCCAAGGG GCTGGGCAAC ATCTCTGCCA
25286967 AGTATCGCTG GTTCGCCGTC TTCTACCTGA TCATCTTCTT CTTCCTGATC CCGCTGACGG
25287027 TGTTTGGCCT CTCGCTGGCC GGCTGGCGGG TGCTGGTTGG TGTCGGGGTT CCCGTCGTCT
25287087 TCATCATCAT CCTGGTACTG TGCCTCCGAC TCCTGCAGTC TCGCTGCCCA CGCGTCCTGC
25287147 CGAAGAAACT CCAGAACTGG AACTTCCTGC CGCTGTGGAT GCGCTCGCTG AAGCCCTGGG
25287207 ATGCCGTCGT CTCCAAGTTC ACCG
```

Sequence 35: SD3 (chromosome 11: 57170977 - 57171240)

```
57170977 CGCGAGAGGA GGGGAATGGC AGGTCCCCGC TTCGCGTACC TTGGAAATAA GCTCATCGTG
57171037 TTTGGCCAGG TGTGCACGGC AGTGGACACA GCTGTAAGTG CGGTGACAGC GGGGCAGATA
57171097 GCTGCGGAAA GTCTTGGTGG GGAGGCAGGC AGGGCCCGGA CCGGGGTCAC AGCTGGGCAT
57171157 GACGGGCTGG AGGACAATGC CCTGGTGGGC TGGAGGGGCT GGCGCCGTGC AGCCCCCGCA
57171217 GAGACGGTCG CAGGTGAAGC AGCG
```

Sequence 36: SD4 (chromosome 11: 1493306 - 1493605)

```
1493306 CGGTGCTCAC CGGCTCGACC CCCCAGCAAC ACGAGAGACC TCACAGAGGG AGTCACACTA
1493366 ACGTGGTCGG GGCTCCAGAG CGAAACCCCA ACCACTATGC TCACAGCCAG GACCGAGCAG
1493426 GCTGGGCCAA CGGCAGTCCC TGCCCAGCGC CCGGCTCCCT CCGAGTGGCC AGCAGCGCCC
1493486 TCTGGTGGAG ACTGGCTCGG CCTCCGCGGC ACTGCATTCC CACGGCAGTG GTCCATCTAG
1493546 TCCCCAAGTC CTAGAGGAGG CCCCTCTCTC TCCCTCAGCC CTGGCAGGGT CCTTGGCGCC
```

Sequence 37: SD5 (chromosome 22: 16491391 - 16491872)

```
16491391 CTGAGAGCCA TGGCGAGAGC AATGCTAGGC CGGTGAACAG TAGGCTCGAG TTTAGGTTTG
16491451 AAAGGTGAGG TGAGAGAAAT CGGCAAAGGG AACCCCTGCG CAGATCTCGG GTTCCTTTAC
16491511 TTTATAACCG CGGGTTCCGG TTCCTGCCAG GTGACTGCAC AGTTCATCCT CATGACCCTT
16491571 CTCAGCCAAT GGGAAGAGAG CGACGCCCAG GAAGTCCCGC CCTGTCCCGG CCTGTGGGCG
16491631 CGTCCTCGGG TCCTTCTACG TCGCTGACTC GTGACCTGAC CGGTATTTTT TTCCTAAACT
16491691 GGGATCTTGG GTAGGAGGAA GAAAAGATAA GGAGTTCCTC TATCTGAAAT TAGTCGGGCT
16491751 GTTTTGAGGA GTACTGGTTA GGTATATTGG AGAATGTGCC TTTATTGGAA TTTTTCGCAT
16491811 AATTACACAG GGTTATGGGC TTAGGGGTAC ATGATTGTTG GCCGGGCGTG GTGGCTCACG
16491871 CC
```

Sequence 38: SD6 (chromosome 4: 155727706 - 155729006)

155727706 TTGCAGGGCT AGCCAGGATA AGCAATGGAT GCTGTTTAAT TCATCTTAGC CTCAGAATGG
155727766 ATCAAGAGTA AGGTGCTATT CCCTCTCAAA GGAAACGCTA ATTAAATGAT CCTTGGTGAA
155727826 ACTCAGGCTG AGGGCTCAGA GGTGTGGTGA TGTAATGGGC TTTGGAATTA GACAGGGACT
155727886 GAACATTTGC TTTTGAAATT TACTATCTAT GTACCGTTGG AAAATTTACT TAATATCTCT
155727946 GAATTTTTTT TCTTCAACTG TGGAGTGAGG AAAATAATAC CTACTTTTAG GTAGATGATG
155728006 GATATAACAC TTTTCTCTGC ATATAGTAGA CACTCAGTGC ATAACTATCG CCTTCCTTTT
155728066 CCCTCTACTC AGAAACAAGG ACATCTGGGA CCACAGCCAC ATACTTACCT CCAGTCGTTT
155728126 CATATCAACC AACTGAGCTC TAACATTTTT CTGCAGAAGC TGGATATGCT GTACTTTTTC
155728186 TATGACTTTG CGCTTCAGGA CTTCAATTCT GCTTCTCAGA TCCTCTGACA CTCGGTTGTA
155728246 GGTATTATCA CGGTCTGAAA TCGAAAATAT GGTTATTGAA GTAGCTGCTG AGTGATTTGT

155728306 CTGTAATTGC CAGCAAAAAA GAAAGGAAGA AAGGAAGGAA GGAGAAAGAA AGAAAGAAAG
155728366 AAAGAAAGAA AGAAAGAAAG AAAGAAAGAA AGAAAGAAAG AGAAAAAAGA AAGAAAGAAA
155728426 CTAGCTTGTA AATATGCCTA ATTTTATTTT GGTTACAGTT TAATCTGTGA GTTCAAAACC
155728486 TATGGGGCAT TTGACTTTTG GATAATGTTA TGCCCTGCAG CCTTCCATGA ATGCCAGTTA
155728546 AGATGTCCTA ATAGCAATTA GTAATCCCAA AGAAATATAG AAGAAGAACT TTCTTTGGAA
155728606 TTTTAAAGGT GTAATTTGGA GTTAAAATAG TTGGTTTGAT TGCATTTCAA TTATTTTATA
155728666 ACATCCTTAA TCAAGGGACT TGAACATATT GGATTTTCTT ACTGATGAGC TTTTCTTTTT
155728726 AATCTATAGA TTTGAAATGG TTCCTAAGCT GTTTTGGGTC AACAGGATCA CTCACTTGCC
155728786 AGCTAGTGTT GCATCACTGA TTTTAAATGT CAAGTGTTTG TGAAGGTGTA AAAAGGCAAA
155728846 GCAAAACTTG AGAAACTGAG GACTCCCTAG ACTCGCTGTC CATGCCCAGA GTGAATGCAA
155728906 TGTTTCCTAA CCCTAATGAG TAGACTGTGA GAATGACGTA GCTTGACCCT ATATTTTAAA
155728966 TTTAAAAATC TACCTAATCA GCTCAGTGGA GTCTGGGAGT T

Sequence 39: L91762 (chromosome 12: 73985133 - 73985792)

73985133 GAACTGAAAT TTCGGAGGGA GCTAAGAGCT CCATGTTTTT ATTTTACTAT TCTTTAAACA
73985193 AGCAGACACA GTGCTGGTTT CTGATCATGT GGGAGGTGTG TAGGAGAGAA CACAGGTAAA
73985253 GTAAAATAAA AATATAAAGC CATAAATGCC ACAACAGAAA TTCAATGAAC AAACATAGAA
73985313 GAGAATACCT TAATTCCTAG AAGACAGAGA GAAGAGTTTA TGAAAGGCAT CCTAGAAGAG
73985373 GAGATATTTG CACTGATTCT TAAAAGGTGA GAGTACAAGA CTTCCTCTCC AGGAGTCGGC
73985433 GGAGGGAGGG CAGACTCAAA GTGCTCCTGG ACGCAGAGGC TCTTGTCAGA GGGCACAGAC
73985493 CGGTTGGAAC AAAGTAAGGA TCTGAGCGAC CCCAACTTTG CAGCCGAGGC CTCCAGCTCC
73985553 GAGGTGCATA GCAACCCTAG GGTTCCGGTA GGCGTTCCTC CGTCCGCGAC CCTGGCAGAG
73985613 CCGCAGAGCC CTCCTCCAGA CCCGACCACT GCCCCAGGCC CCGAGCGACC GGAAGGCTCA
73985673 GCAGCAGGCC GCGGAGAAGG GCGGCGCCCA CCAGGCCCCT AGCACTGCCC AGTCTGGCCC
73985733 GGCACAGCTG CTGCAGAAGG CGCACGACGA GCTCGTGTGG TACATGCGGG TCAAGGACCA

Sequence 40: L68346 (chromosome 3: 12175184 - 12175532)

12175184 CGCGGACCTC GGACTCACCA AGTGCCGAGT GGCAGATGTG CTGCTGAGGG TGCGCCGGGG
12175244 CGCAGCTGCA TGCCTCACCC AGCCCCGGGG GCCGCAGCAA CGCCAGCAGC CGCAGCAACA
12175304 GCACCCAGCT TGGCGCGGGC CGAGGGCTCC CAGGCATGAC ACTGCAGATC CGCGACTGAG
12175364 CCTGTGAGGT CTGGGGGACT GGACGGCCCC AGCAGGGCTC CTTCCCAAGG CCGTTGTGCC
12175424 CCTCGCCCCA GGCTTTATGA GGTGGCCCTA AGGGCCAATC CCGCCCCGAC GGGCTCCGCC
12175484 TTCCTTTGGC TCTAGGCCAC CCGCGGAGGC AGGACCCGAG GCTCTTCCG

Sequence 41: L50468 (chromosome 3: 55492699 - 55493120)

55492699 CGCGTGCCAC GGGGAGGAGG ACTGGGGGCG TTTGAGGGGC TCAGCGCACC AGAGGAGTGA
55492759 GGTGGAGGAG GGCGTTCCCG CGTCCTCCTC TTCAATCCAG AGCAGCTCAA CGACGTGGCT
55492819 CCCTTTCTAT GTATCCCTCA AAGCCTTCGC GTCGGATTAA AGGTGTTCTT GATCCTTCTT
55492879 TACCAACCAC GGTGCGGGCC AGGCGTGATG AGGGATGAGG GAGAGGAAAC CTCAGTAGCA
55492939 AAATTGTTCA GAGGACGTTC GGAGGGCGCG GGGAGCAGCC GGATGCACAC CTAAAGTCTC
55492999 GCAACACCCA ACTCCTCCTC CGCAGGCAGT CCCTTAAGAG AATAGATAAA AAGGCCAAGC
55493059 AAAGATCCTC TCCCCTCGGC CCCGGGGCAC TGCCACATTC AGTCTAGGCA TCCCCTTCTG
55493119 CT

Sequence 42: L14432 (chromosome 22: 29866031 - 29866650)

29866031 GCTCGGGTCC GGGGGTGTGG ATGAAGGAGC CCCAGGCAGT CTCATGAGCA CAGAGAGCAC
29866091 CGTAGGCTGC GGTGAGCTCC GGCTCATCCT CCCAGCTACA TGACTGCAGC CTCCTATGCG
29866151 CATCCCAGCG GGCATGGATC AGGGCCAGTC CCTGAGCATC CTTGGCCAGG AAGCTCAGGT
29866211 ACCCCAGTGG GTTGCCAGGG ACGGCCTTGG TCAAGTGGCA GGAGGTCCTG TACCAGCGGA
29866271 GGGCAGGGGA GCCCCCCAGG GCCACCCCCA GGAAGCCCAG CATCCCAAAC AGCCCTGCCT
29866331 GAACCCCCAT TCTGCACTGG CCCAGTCCAG TCAGACAAAG CCCCTGGGAT GCCTGCCCTT
29866391 GGTGGCCCCA CCAGGCGGCA GCTGAGCAGT GGAACGGAAG CGGAGCCCAG CAGGCCCGGT
29866451 GCGGCGGGAC CAATGAATGG AGCTGCGGGA GGAGGAGGAA AGAGGCTGGA GTATGGGGTG
29866511 ATCTTGGGCT TGTAACCGAA TCCACCAGCC GGGCAGGACG CTGATTGGCT GAGGAGTGCA
29866571 CTTGCCAGGG CCCACGCCCC CATTCTGCCT GCCTTCTCAG CACCATCCAG TCACCTGCTG
29866631 CCAGCCCTGC CTAGATTGGG

Sequence 43: L4648 (chromosome 1: 35815020 - 35816031)

35815020 CGGCGCGCTG GCTCCCTGGA GCGGGGCGGG ACGCGGCCGC GCGGACTCAC GTGCACAACC
35815080 GCGCGGGACG GGGCCACGCG GACTCACGTG CACAACCGCG GGACCCCAGC GCCAGCGGGA
35815140 CCCCAGCGCC AGCGGGACCC CAGCGCCAGC GGGACCCCAG CGCCAGCGGG ACCCCAGCGC
35815200 CAGCGGGACC CCAGCGCCAG CGGGACCCCA GCGCCAGCGG GTCTGTGGCC CAGTGGAGCG
35815260 AGTGGAGCGC TGGCGACCTG AGCGGAGACT GCGCCCTGGA CGCCCCAGCC TAGACGTCAA
35815320 GTTACAGCCC GCGCAGCAGC AGCAAAGGGG AAGGGGCAGG AGCCGGGCAC AGTTGGATCC
35815380 GGAGGTCGTG ACCCAGGGGA AAGCGTGGGC GGTCGACCCA GGGCAGCTGC GGCGGCGAGG
35815440 CAGGTGGGCT CCTTGCTCCC TGGAGCCGCC CCTCCCCACA CCTGCCCTCG GCGCCCCCAG
35815500 CAGTTTTCAC CTTGGCCCTC CGCGGTCACT GCGGGATTCG GCGTTGCCGC CAGCCCAGTG
35815560 GGGAGTGAAT TAGCGCCCTC CTTCGTCCTC GGCCCTTCCG ACGGCACGAG GAACTCCTGT
35815620 CCTGCCCCAC AGACCTTCGG CCTCCGCCGA GTGCGGTACT GGAGCCTGCC CCGCCAGGGC
35815680 CCTGGAATCA GAGAAAGTCG CTCTTTGGCC ACCTGAAGCG TCGGATCCCT ACAGTGCCTC
35815740 CCAGCCTGGG CGGGAGCGGC GGCTGCGTCG CTGAAGGTTG GGGTCCTTGG TGCGAAAGGG

35815800 AGGCAGCTGC AGCCTCAGCC CCACCCCAGA AGCGGCCTTC GCATCGCTGC GGTGGGCGTT
35815860 CTCGGGCTTC GACTTCGCCA GCGCCGCGGG GCAGAGGCAC CTGGAGCTCG CAGGGCCCAG
35815920 ACCTGGGTTG GAAAAGCTTC GCTGACTGCA GGCAAGCGTC CGGGAGGGGC GGCCAGGCGA
35815980 AGCCCCGGCG CTTTACCACA CACTTCCGGG TCCCATGCCA GTTGCATCCG CG

Sequence 44: L39664 (chromosome 17: 53710207 - 53710501)

53710207 CGCGCTGAGC GGTTGGTGAG GAGACAGGGG TCATCGTGCA GGTTGTCAAA GGGCAGCAGG
53710267 GCCCGGCCGT TGTCTTGGAA GCGCTGGTTG ACGGCCAGCA GCCCCAGCTG GTTGGACATG
53710327 TTGCGCAGGT TCCTGGCCAG GGGCTCCTCG CTGCCGTACA CCATGCTGGC GTCCACGAAG
53710387 GAAGTGAGCG CGTTGATCTG GTTGCGGATG GTGATGTTGC TCCCGGGGCA AGCCGGGCAG
53710447 GAGCGGAAGA ACGGGATGCA GTCGGCTTGG TTCTTGATGC GGGGGTCATT GGGCG

Sequence 45: L30139 (chromosome 17: 36996273 - 36997073)

36996273 TGCCCACCAG AAGCCCATCA CCACCAGCAA AGCCACCACC AAAGCCACCA CCCAAGCCAG
36996333 CACCAAGGCC ACCACCATAT CCTCCCCCAA AGCCACTACC AAAGCTGCTG CTGCTGCTGC
36996393 TGAAGCCACC GCCATAGCCG CCCCCCAGCC CGCAGGCTCC CCCAGAGGAG AAGCGGGAGG
36996453 ATGAGACAGA CAGGCCGCCC CCGTAGGTGC TGGGGGCGCG GCAGGACCCT CCGGCCAGGA
36996513 CGGAGGAGAT GCGGCTGGAG CCGCCCCCGA TGCCGCCCCC GATGCCGCAG GAGCCCTTCA
36996573 TGGAGCTGGA GGAGGTGAAC TGGCGGCTGC AGGTGGTCAT GGTGCAGAGG AGGGAGGTGA
36996633 GCGAGCGAGC AGTTGGCTGA GTGAAGAGAA GGTGCTCGGG TAAATTGGAA AGGGATGCGA
36996693 GTGCTTTATA CTCGTGGGTA GGGGGCGGGT CTGGCACTTT CCATTCCCCT TGGCTTTCAT
36996753 CACCCACAGG CTAGCGCCAA CTCCCAGCCA GGTCCCTCCT CTCATCCGCC TCATCATGTC
36996813 TGTCATATTT TACTGGAAAC TCATTGTTTG GAGTGTTTTG GGCTTTCTTG TCCCGCCAGG
36996873 CGTGATTCAC AGGGGGAGGT GTGGGCCTGC AGGCTACACT TTCCCATCGG ACCCTGGGAG
36996933 TCCCAGCCCT CAGGAACCCG CGCACTGGGC TTAGCCAGGG TGACAGAGAG CAGGGCCTCT
36996993 GCACCTTAAA CCTAGTTACC TGCTAGCTCT CCATGAACTG AATGGGCCTT TAACATCCAC
36997053 GTAGAGGAAG CCTGCTGCTG C

Sequence 46: L55429 (chromosome 5: 1547854 - 1548287)

1547854 CGATGGAGGA CATCGTCATG GTCACAGGGA TGGCGTCGAA GTAGGACGAG TGAGGCGCGA
1547914 GCGTGAGGAT GGCCGCCTCG GTGGGCAGCG CCTGCCGCCC CTTCACGGCC ACCCGGTGGA
1547974 AGCCGCCGGC GAACCACATG GTGCGCATGA TGGCCTTCAG CAGGAAGTCC ACAACCCTGC
1548034 AAAAGAGGGC GCTGCGTCAC GCGGGCACAC GTCCGCAGTC TCGGAGTCTG TGTGAGGCAC
1548094 AGGGGCGGTC CCACGGGAGA GCCCTCCAGG GCGCAGTCCA GGCCACGGGC TTCCTGTGGT
1548154 CGCCGCCGCT GGGACATCTG CTTGAGGGAA GAAAAGACGC CGCGCCTTCC TGGCCTCCGC
1548214 CTGTGTCCTC GCTGGCTGCG CTCTCACCTA CGAAGGAGGC CGCGGGGCCC TGTGTGGTGG
1548274 TCACGGGCCA CGCG

Sequence 47: L62086 (chromosome 19: 40478392 - 40478802)

40478392 CGTCCATCTC GGCCTTCGAA GGCACGTGCG TCTCCATCCC CTGCCGCTTT GACTTCCCGG
40478452 ATGAGCTGCG GCCCGCTGTG GTGCATGGTG TCTGGTACTT CAATAGCCCC TACCCCAAGA
40478512 ACTACCCCCC GGTGGTCTTC AAGTCGCGCA CCCAAGTAGT CCACGAGAGC TTCCAGGGCC
40478572 GCAGCCGCCT CCTGGGGGAC CTGGGCCTGC GAAACTGCAC CCTCCTGCTC AGCAACGTCA
40478632 GCCCCGAGCT GGGCGGGAAG TACTACTTCC GTGGGGACCT GGGCGGCTAC AACCAGTACA
40478692 CCTTCTCAGA GCACAGCGTC CTGGATATCG TCAGTGAGTC CCCAGCGGTT GTGCAGGCAC
40478752 CGGGAGCTGG GGCAGCGGGG CGGGAAGGAG TGTGGCCGGA AGGCCTCCCC G

Sequence 48: L76138 (chromosome 19: 3129742 - 3130341)

3129742 CGGGTGGCTC CACCCTGCGT CGGGCCTCAG TCAGCCCCCG GGGGAGGCCA TGAACGCCAC
3129802 GGGGACCCCG GTGGCCCCCG AGTCCTGCCA ACAGCTGGCG GCCGGCGGGC ACAGCCGGCT
3129862 CATTGTTCTG CACTACAACC ACTCGGGCCG GCTGGCCGGG CGCGGGGGGC CGGAGGATGG
3129922 CGGCCTGGGG GCCCTGCGGG GGCTGTCGGT GGCCGCCAGC TGCCTGGTGG TGCTGGAGAA
3129982 CTTGCTGGTG CTGGCGGCCA TCACCAGCCA CATGCGGTCG CGACGCTGGG TCTACTATTG
3130042 CCTGGTGAAC ATCACGCTGA GTGACCTGCT CACGGGCGCG GCCTACCTGG CCAACGTGCT
3130102 GCTGTCGGGG GCCCGCACCT TCCGTCTGGC GCCCGCCCAG TGGTTCCTAC GGGAGGGCCT
3130162 GCTCTTCACC GCCCTGGCCG CCTCCACCTT CAGCCTGCTC TTCACTGCAG GGGAGCGCTT
3130222 TGCCACCATG GTGCGGCCGG TGGCCGAGAG CGGGGCCACC AAGACCAGCC GCGTCTACGG
3130282 CTTCATCGGC CTCTGCTGGC TGCTGGCCGC GCTGCTGGGG ATGCTGCCTT TGCTGGGCTG

Sequence 49: L15952 (chromosome 7: 2740971 - 2741510)

2740971 CGGGGCGAGC ACGTGCACCT CGTGACCACC GGCTCTCTAG AGCCCCAGGC AGAGGCCCAG
2741031 CTCTGCAGTA GGGAAGGCAT CGGGAGTGCC AGGGTGAACG TACCCCAAGG GCCCGGCACG
2741091 ACTGACCTCT CGTGCCTGCT CTCTCCCTTC CTCGCCAAGC CCCCGTGATG TGGGAAGCCA
2741151 GCGTGAGGCC GGTGGGGCAG CCGCCTTCCC GTGGCTGTGC CAAGTCCCCC CGGTCCTCTG
2741211 CACATCATGC CTCCTTCCAC ACCCTGACAG GAAGCAGCTG GGAGAAGCCG TTGGGTGCAC
2741271 TCACTCCCTG ATTTACGACA AGTTCCTTCC TCAGCGCCTC TCTCTCCTGC CTCCTCCTGC
2741331 TCTCCTGCCC TCCCCTGGGC CTCGGGAGGT GCCACGCAAG CCCAAGAAGC ATCAGCATAC
2741391 TGTCCCTCCC TCTCCTGTGG CCACGGGCTC CCCCAGGGAG CTGAGAGTAG CAGCAGCTCA
2741451 CAGCCCAAGC CACCCTTGCC CGTTTCTAGG CAGGTGGTGG CACCAGGCAC GAAGGAAGCA

Sequence 50: L36599 (chromosome 19: 4867561 - 4867784)

4867561 CGGTGACCCG GCCGGGTCGA AGGGCAGAGT TCCGCTGTCA CTAGCCCTCC ACCCGTCCTG
4867621 TGTGCTGGGA TGCCCTCGCG GCGCCGTCCA CGCCACCGCC GCCCCCTCTT GTGGGTTCTG
4867681 TCTCCTCCGT GTCTAGGATC CTCCTGCATC CGTTTTTCCT TCCTCCCTTC TCTCCCTCCG
4867741 TCTGTCTTGC CCGCACCTGA GGTTGTCGCA GAGGCGCTGA GACG

Sequence 51: L26688 (chromosome 17: 77844647 - 77844962)

77844647 CGCGGGCTGC GGGGCTGCGG GGAGGGAGGG GCGGGTTCAG GGCTGTGGGC CCCGCCCGGG
77844707 GCACCGCCTG GATCAGGCCT GAGGCCGGCT GCAGAGAACT TGGGCACCGC GGACCAGATT
77844767 GCTGGTCACC AGGATGAAAA ATAAAAAAGA CCCAAACAGC TCCCCCGACC CCGCCTTCGC
77844827 GCAGGCCCCT GGCGTTCTGC TCAGGGGTTT TTGTTTAATG AAGAACGTCA AACATCTGGC
77844887 AAGGTCGGAA TGATTTTGCG ATGAGCACCG CCTGGATTCT GCGGTGAAAG CGACTGTGTG
77844947 CGCGCTCACG GCCTCG

Sequence 52: L81528 (chromosome 19: 47395445 - 47395816)

```
47395445 CGGGCGCCGC CCCCCTTCCT CCTCCATCAG CAACAGGCGG CGCCGGCCAG CCTCATAGTC
47395505 AGCCTCATCC ACACTGACCA GCAGGCGAAC AGCCTCCCGG CCCACAGCCT CTCGCAGGGC
47395565 CTCAGTCAGG AACACGCCCC GCAGGGCCTG CAGCAGGGCG CCACTCAGGT AGTCGCCCCA
47395625 GAAGGCGTCC AGATAGGAGA GCTCTGAGAA CTTGATGTCA CAAACCACAG AGCCCAGGTC
47395685 CCTTGAGCGC AGCACTGCGG TGGCCTGCCC AAACACGTCC AGCTGCCGCG CCAGCGCCTG
47395745 GGGCCGCCGG GATGCCACGC CCTGCTCCAA GGCTGGCCCA TGCTCGCAGT ACTCTGCTCG
47395805 AACCCGGAGC CG
```

Sequence 53: L36556 (chromosome 19: 50962003 - 50962260)

```
50962003 CGCCCAGGGC CAGGCCGTTG ATGATGACGG GGCCCCCGTT GAGGAGCACT GCTGGGGAGC
50962063 CGCTGGCTGC CAGGAAGCTC CCGTTCACCA GGATGGAGGA GGAAGCCGGG CAAGGCGCGG
50962123 GAGGGCCGGT CCCTGCCAGG AATATGGAGC CCTGGGCAGC GGCCTCGGCG GACACTGGGG
50962183 CCGCCCCTCT CTCCAGGTCC TCAGGACTTC GGCTGGACTC GTCCTCAGTC GTGGGATTCC
50962243 CATCAGACTC GCTGGCCG
```

Sequence 54: L16264 (chromosome 2: 2733750 - 2734147)

```
2733750 CGCCTTTTCC TTCGGTCACT CAGACCGCGC CCGAGGTCCC TGGTGCCCCC GCGGGGGAGC
2733810 TGAGGTTTGC GGTCCCTCCT GGGGTTCGCT TCCCGCGGGG CTAAACCGCG GCGACCAGGG
2733870 CCCCTTTCTC CACTGGTGCC TTTTCCGGGA AACGCTGCTC CTTAGATGGA CGAATACGTA
2733930 CTCGGTACCC AGCACATCCT GGACGAGTTA ACTTCCTTAA TTTCCATATT TGCCGGAGAA
2733990 CCAGCTGGTT CAGAGCGCAC AGCAAACGGG AGAAGTTAAA CCAGGTTCCG CGACCCAGAG
2734050 CCCAGGGGTG GCCCCGGGGA CACCCACCTG ACTCCGCACC CCCCACGAGA GGGGAGGATC
2734110 CTTGCAGACC TCACCTTTGC TGGCAACGCT GCGGCCCG
```

## Claims

**1.** A method for categorizing a DNA sample by determining signal ratios between genomic loci co-amplified in the same reaction, comprising:

(A) digesting a DNA sample with a methylation-sensitive restriction endonuclease or a methylation-dependent restriction endonuclease;
(B) amplifying at least two genomic loci from the digested DNA, wherein at least one of the loci is a restriction locus;
(C) determining the signal intensity of the amplification product of each locus;
(D) calculating signal ratios between the signal intensities of the amplification products; and
(E) comparing the signal ratios to reference values of different DNA categories,

wherein the approximation of the value of a signal ratio to the values of reference ratios of a particular DNA category indicates the categorical source of the DNA sample wherein the genomic loci that are amplified are chosen to produce distinct signal ratios for different potential categories, wherein no standard curve or reference DNA is needed and wherein no actual methylation level at any genomic locus is indicated by said method.

**2.** The method of claim 1, wherein the reference values against which the signal ratios are compared are from at least one DNA category selected from the group consisting of tissue type, cell type, physiological condition, pathological condition, age, ethnicity, sex, methylation level, species, cell lines, natural DNA and artificially-synthesized DNA, risk of developing a pathological condition, prenatal conditions, prognosis, propensity to respond to treatment, effects of cell line sub-culturing, response to medication.

**3.** The method of claim2, wherein the tissue type or cell type is selected from the group consisting of blood, saliva, semen, epidermis, urine, plasma, hair, menstrual blood, vaginal cells and/or secretion, sweat, feces, brain, esophagus, lung, stomach, heart, duodenum, liver, gall bladder, intestine, kidney, adrenal gland, urinary bladder, urethra, colon, testicle, ovary, uterus, vagina, muscle, tendon, ligament, fat, cartilage, bone, endothelial cells, uterine cervix, lymph, thyroid, pituitary gland, cerebellum, and breast.

**4.** The method of claim 2, in which the pathological condition is cancer, inflammation, auto-immune disorder, metabolic disorder, infection, degenerative disease, hormonal imbalances, a disorder **characterized by** abnormal DNA methylation, neurodevelopmental disorder of ICF (immunodeficiency, centromeric instability, and facial anomalies), Rett syndrome, and fragile X syndrome; and wherein the prenatal condition is Prader-Willi syndrome, Angelman syndrome, Beckwith-Wiedemann syndrome, fragile X syndrome, Russell-Silver syndrome, Transient neonatal diabetes mellitus, Albright hereditary osteodystrophy, McCune-Albright syndrome, Familial nonchromaffin paraganglioma, Maternal and paternal UPD14 syndromes.

5. The method of claim 1, wherein the reference values against which the signal ratios are compared represent categories that are mixtures of other categories.

6. The method of claim 5, wherein the reference values against which the signal ratios are compared are from mixtures of semen and non-semen at various ratios.

7. The method of claim 1, wherein the methylation-sensitive restriction endonuclease is selected from the group consisting of AatII, Acc65I, AccI, AciI, ACII, AfeI, AgeI, ApaI, ApaLI, AscI, AsiSI, AvaI, AvaII, BaeI, BanI, BbeI, BceAI, BcgI, BfuCI, BglI, BmgBI, BsaAI, BsaBI, BsaHI, BsaI, BseYI, BsiEI, BsiWI, BslI, BsmAI, BsmBI, BsmFI, BspDI, BsrBI, BsrFI, BssHII, BssKI, BstAPI, BstBI, BstUI, BstZ17I, Cac8I, ClaI, DpnI, DrdI, EaeI, EagI, EagI-HF, EciI, EcoRI, EcoRI-HF, FauI, Fnu4HI, FseI, FspI, HaeII, HgaI, HhaI, HincII, HincII, HinfI, HinP1I, HpaI, HpaII, Hpy166ii, Hpy188iii, Hpy99I, HpyCH4IV, KasI, MluI, MmeI, MspAII, MwoI, NaeI, NarI, NgoNIV, Nhe-HFI, NheI, NlaIV, NotI, NotI-HF, NruI, Nt.BbvCI, Nt.BsmAI, Nt.CviPII, PaeR7I, PleI, PmeI, PmlI, PshAI, PspOMI, PvuI, RsaI, RsrII, SacII, SalI, SalI-HF, Sau3AI, Sau96I, ScrFI, SfiI, SfoI, SgrAI, SmaI, SnaBI, TfiI, TscI, TseI, TspMI, and ZraI; and wherein the methylation-dependent restriction endonuclease is selected from the group consisting of McrBC, McrA, and MrrA.

8. The method of claim 1, wherein the methylation-sensitive restriction endonuclease is HhaI.

9. The method of claim 2, wherein the reference DNA category is natural DNA or artificially-synthesized DNA, wherein at least one genomic locus in the natural DNA or artificially-synthesized DNA comprises a core short tandem repeat (STR) locus used for DNA profiling.

10. The method of claim 9, wherein the genomic locus comprises a human locus selected from the group consisting of D16S539 (SEQ ID NO. 1), D7S820 (SEQ ID NO. 2), D13S317 (SEQ ID NO. 3), D5S818 (SEQ ID NO. 4), CSF1PO (SEQ ID NO. 5), TPOX (SEQ ID NO. 6); TH01 (SEQ ID NO. 7), vWA (SEQ ID NO. 8), FGA (SEQ ID NO. 9), D21S11 (SEQ ID NO. 10), D8S1179 (SEQ ID NO. 11), D18S51 (SEQ ID NO. 12), and D3S1358 (SEQ ID NO. 13), Penta D (SEQ ID NO. 14), Penta E (SEQ ID NO. 15), and Amelogenin (SEQ ID NOs. 16 and 17), D2S1338 (SEQ ID No. 18), D19S433(SEQ ID No. 19), ACTBP2SE33 (SEQ ID No. 20), D10S1248 (SEQ ID No. 21), D1S1656 (SEQ ID No. 22), D22S1045 (SEQ ID No. 23), D2S441 (SEQ ID No. 24), and D12S391 (SEQ ID No. 25).

11. The method of claim 2, wherein the reference DNA category is natural DNA or artificially-synthesized DNA, wherein at least one genomic locus is known to be methylated in all tissues of natural DNA, or is known to be unmethylated in all tissues of natural DNA.

12. The method of claim 11, wherein the genomic locus known to be methylated in all tissues of natural DNA is TPOX (SEQ ID NO. 6) or FGA (SEQ ID NO. 9), and wherein the genomic locus known to be unmethylated in all tissues of natural DNA is Hypo23 (SEQ ID NO. 29), Hypo28 (SEQ ID NO. 30) or Hypo33 (SEQ ID NO. 31).

13. The method of claim 9, wherein the primers for amplification of at least one of the genomic loci are available in:

(1) a Promega Corporation commercial kit selected from the group consisting of PowerPlex® 16 HS (Cat.# DC2100, DC2101), PowerPlex® 16 (Cat.# DC6530, DC6531), PowerPlex® 2.1 (Cat.# DC6470, DC6471), PowerPlex® 16 BIO (Cat.# DC6540, DC6541), and PowerPlex® ES Systems (Cat.# DC6730, DC6731);
(2) an Applied Biosystems commercial kit selected from the group consisting of SGM, SGM+, AmpFISTR Identifiler; AmpFISTR Profiler, AmpFISTR ProfilerPlus, AmpFISTR ProfilerPlusID, AmpFISTR SEfiler, AmpFISTR SEfiler Plus, AmpFISTR Cofiler, AmpFISTR Identifiler Direct, AmpFISTR Identifiler Plus, AmpFISTR NGM, AmpFISTR Y-filer, and AmpFISTR Minifiler; or
(3) Investigator ESSPlex, Investigator ESSPlex SE, Investigator Nonaplex ESSPlex, Investigator Hexaplex ESSPlex, Investigator Triplex AFS QS, Investigator Triplex DSF, Investigator IDplex, Investigator Decaplex SE, Investigator HDplex, Investigator Argus X-12, Investigator Y-12 QS, Investigator DIPplex.

14. The method of claim 9, further comprising calculating at least one of the following ratios:

(1) D3S1358/D18S51,

(2) D3S1358/D7S820,
(3) D3S1358/Penta_D,
(4) D3S1358/TPOX,
(5) D3S1358/FGA,
(6) TH01/Penta_D,
(7) D21S11/D18S51,
(8) D21S11/D7S820,
(9) D21S11/Penta_D,
(10) D21S11/AMEL,
(11) D21S11/TPOX,
(12) D21S11/FGA,
(13) D5S818/D18S51,
(14) vWA/D18S51,
(15) D5S818/Penta_E,
(16) vWA/Penta_E,
(17) D5S818/D7S820,
(18) D5S818/Penta_D,
(19) D5S818/TPOX,
(20) D5S818/FGA,
(21) D13S317/D7S820,
(22) D13S317/Penta_D,
(23) D13S317/TPOX,
(24) D13S317/FGA,
(25) D16S539/D7S820,
(26) CSF1PO/D7S820,
(27) vWA/D7S820,
(28) D8S1179/D7S820,
(29) D16S539/TPOX,
(30) D16S539/FGA,
(31) CSF1PO/Penta_D,
(32) CSF1PO/TPOX,
(33) vWA/Penta_D,
(34) AMEL/TPOX,
(35) AMEL/FGA,
(36) vWA/D8S1179,
(37) vWA/TPOX,
(38) vWA/FGA,
(39) D8S1179/TPOX, and
(40) D8S1179/FGA.

**15.** The method of claim 1, wherein signal intensity is the amplification product's fluorescence level measured during capillary electrophoresis.

**16.** The method of claim 1, wherein steps (B) and (C) are performed by real-time PCR.

**17.** The method of claim 16, wherein the signal intensity of each amplification product is the fluorescence level of each amplification product.

**18.** The method of claim 1, further comprising assigning confidence levels to potential categories of a DNA sample, wherein the confidence level reflects the likelihood that a particular DNA category indicates the categorical source of the DNA sample; wherein the likelihood is calculated by:

(A) assigning a probability score to each comparison of a signal ratio to the reference values that correspond to the various categories of DNA, wherein the probability score is obtained by (i) assigning a gamma probability distribution function to the reference values of each potential category; wherein (ii) the probability score is equal to the value of the gamma probability distribution function assigned in step (A) at the observed signal ratio;
(B) for each category, calculating the category likelihood score, which is the product of the probability scores obtained in step (A); and

(C) normalizing likelihood scores by dividing the category likelihood score of each potential category by the sum of all category likelihood scores;

wherein the confidence level of each category is the normalized likelihood score of that category.

**19.** The method of claim 1, further comprising simultaneously performing DNA profiling with the DNA categorization.

**20.** A kit for categorizing a DNA sample as semen or non-semen, comprising:

(a) a primer mix, comprising the following primers:

SD1f (AAGAGCCCATCAGGCAGGTC);
SD1r (GTTTCTTGTCGAGCAGCACGTGGATGATG);
SD2f (CTCCAGAACTGGAACTTCCTG);
SD2r (GTTTCTTAACTTGGAGACGACGGCATC);
SD3f (TGGAGGACAATGCCCTGGTG);
SD3r (GTTTCTTGGCTTCACCTGCGACCGTCTC);
SD4f (CCCTCCGAGTGGCCAGCAG);
SD4r (GTTTCTGACCACTGCCGTGGGAATG);
SD5f (CTTCTCAGCCAATGGGAAGAG);
SD5r (ACGTAGAAGGACCCGAGGAC);

(b) reaction buffer;
(c) HhaI restriction endonuclease; and
(d) DNA polymerase;

wherein at least one pair of forward (f) and reverse (r) primer pair combinations is selected from the group consisting of:

(1) SD1f (AAGAGCCCATCAGGCAGGTC) and SD1r (GTTTCTTGTCGAGCAGCACGTGGATGATG);
(2) SD2f (CTCCAGAACTGGAACTTCCTG) and SD2r (GTTTCTTAACTTGGAGACGACGGCATC);
(3) SD3f (TGGAGGACAATGCCCTGGTG) and SD3r (GTTTCTTGGCTTCACCTGCGACCGTCTC);
(4) SD4f (CCCTCCGAGTGGCCAGCAG) and SD4r (GTTTCTGACCACTGCCGTGGGAATG);
(5) SD5f (CTTCTCAGCCAATGGGAAGAG) and SD5r (ACGTAGAAGGACCCGAGGAC).

**21.** The kit of claim 20, wherein the concentration of the primers in the primer mix are: 0.6μM SD1f, 0.6μM SD1r, 1.75μM SD2f, 1.75μM SD2r, 1.25μM SD3f, 1.25μM SD3r, 1.75μM SD4f, 1.75μM SD4r, 1.75μM SDSf and 1.75μM SD5r.

**22.** A kit for profiling, categorizing a DNA sample as semen or non-semen, and obtaining an associated categorization confidence level, comprising:

(a) primers for amplifying at least one semen-specific locus;
(b) primers for amplifying at least one locus used for DNA profiling;
(c) reaction buffer;
(d) HhaI restriction endonuclease, and
(e) DNA polymerase, wherein

the at least one semen-specific locus is the L68346 locus, and wherein the primers for amplifying a 70 bp semen-specific amplification product from L68346 are a forward primer comprising the sequence of CAGCAACAGCACCCAGCTTG (FAM) and a reverse primer comprising the sequence of CACAGGCTCAGTCGCGGATC; or. wherein the at least one semen-specific locus is the L16264 locus and wherein the primers for amplifying a 95 bp semen-specific amplification product from L16264 are a forward primer comprising the sequence of GGACGAGTTAACTTCCTTAATTTC (FAM) and reverse primer comprising the sequence of GTTTCTTCGCGGAACCTGGTTTAACTTC.

**23.** A kit for categorizing a DNA sample as blood, saliva, semen, or skin epidermis, and for obtaining an associated categorization confidence level, comprising:

(a) primer mix that comprises forward and reverse primers for amplifying the denoted loci as follows:

1. L91762 (forward GCAGCAGGCCGCGGAGAAG (FAM); reverse AGCAGCTGTGCCGGGCCAG)
2. L68346 (forward CAGCAACAGCACCCAGCTTG (JOE); reverse CACAGGCTCAGTCGCGGATC)
3. L50468 (forward AGGAAACCTCAGTAGCAAAATTG (JOE); reverse GCGAGACTTTAGGTGTGCATC)
4. L14432 (forward CGTAGGCTGCGGTGAGCTC (FAM); reverse GATCCATGCCCGCTGGGATG)
5. L4648 (forward CAGCCTAGACGTCAAGTTACAG (JOE); reverse ACGACCTCCGGATCCAACTG)
6. L39664 (forward CCCAGCTGGTTGGACATGTTG (FAM); reverse CACTTCCTTCGTGGACGCC)
7. L30139 (forward GAGAAGCGGGAGGATGAGAC (FAM); reverse CCGCATCTCCTCCGTCCTG)
8. L55429 (forward GCCTTCAGCAGGAAGTCCAC (JOE); reverse CCTGTGCCTCACACAGACTC)
9. L62086 (forward GTGCATGGTGTCTGGTACTTC (FAM); reverse GAAGCTCTCGTGGACTACTTG)
10. L76138 (forward CAGCCTGCTCTTCACTGCAG (JOE); reverse AGAGGCCGATGAAGCCGTAG)
11. L15952 (forward CTCCCTGATTTACGACAAGTTC (FAM); reverse GACAGTATGCTGATGCTTCTTG)
12. L36599 (forward AAGGGCAGAGTTCCGCTGTC (FAM); reverse CGGATGCAGGAGGATCCTAG)
13. L26688 (forward CGGACCAGATTGCTGGTCAC (JOE); reverse CGACCTTGCCAGATGTTTGAC)
14. L81528 (forward AGCCTCATCCACACTGACCAG (JOE); reverse TCAGAGCTCTCCTATCTGGAC)
15. L36556 (forward GCCAGGCCGTTGATGATGAC (JOE); reverse GAATATGGAGCCCTGGGCAG)

(b) reaction buffer;
(c) HhaI restriction endonuclease; and
(d) DNA polymerase.

## Patentansprüche

**1.** Verfahren zur Kategorisierung einer DNA-Probe durch Bestimmen der Signalverhältnisse zwischen genomischen Loci, die in der gleichen Reaktion coamplifiziert werden, umfassen:

(A) Verdau einer DNA-Probe mit einer methylierungssensitiven Restriktionsendonuclease oder einer methylierungsabhängigen Restriktionsendonuclease;
(B) Amplifizieren von mindestens zwei genomischen Loci aus der verdauten DNA, wobei mindestens einer der Loci ein Restriktions-Locus ist;
(C) Bestimmen der Signalintensität des Amplifikationsprodukts von jedem Locus;
(D) Ermitteln der Signalverhältnisse zwischen den Signalintensitäten der Amplifikationsprodukte; und
(E) Vergleichen der Signalverhältnisse mit Referenzwerten verschiedener DNA-Kategorien,

wobei die Annäherung des Wertes eines Signalverhältnisses an die Werte von Referenzverhältnissen einer bestimmten DNA-Kategorie auf die kategorische Quelle der DNA-Probe hinweist, wobei die genomischen Loci, die amplifiziert werden, so gewählt werden, dass für verschiedene potenzielle Kategorien eindeutige Signalverhältnisse erzeugt werden, wobei keine Standardkurve oder Referenz-DNA benötigt wird, und wobei durch das Verfahren bei keinem genomischen Locus ein tatsächlicher Methylierungsspiegel angezeigt wird.

**2.** Verfahren nach Anspruch 1, wobei die Referenzwerte, mit denen die Signalverhältnisse verglichen werden, aus mindestens einer DNA-Kategorie sind, die ausgewählt ist aus der Gruppe bestehend aus Gewebeart, Zelltyp, physiologischem Zustand, pathologischem Zustand, Alter, Ethnizität, Geschlecht, Methylierungsspiegel, Spezies, Zelllinien, natürlicher DNA und künstlich synthetisierter DNA, Risiko, einen pathologischen Zustand zu entwickeln, pränatalen Zuständen, Prognose, Tendenz, auf eine Behandlung anzusprechen, Effekten von Zelllinien-Subkultivierung, Ansprechen auf Medikamente.

**3.** Verfahren nach Anspruch 2, wobei die Gewebeart oder der Zelltyp ausgewählt ist aus der Gruppe bestehend aus Blut, Speichel, Sperma, Epidermis, Urin, Plasma, Haar, Menstruationsblut, Vaginalzellen und/oder -sekret, Schweiß, Fäkalien, Gehirn, Speiseröhre, Lunge, Magen, Herz, Zwölffingerdarm, Leber, Gallenblase, Darm, Niere, Nebenniere, Urinblase, Harnröhre, Dickdarm, Hoden, Eierstock, Gebärmutter, Vagina, Muskel, Sehne, Band, Fett, Knorpel, Knochen, Endothelzellen, Gebärmutterhals, Lymphe, Schilddrüse, Hypophyse, Kleinhirn und Brust.

**4.** Verfahren nach Anspruch 2, bei dem der pathologische Zustand Krebs, eine Entzündung, Autoimmunerkrankung, Stoffwechselstörung, Infektion, degenerative Erkrankung, Hormonstörungen, eine Erkrankung, die durch anormale DNA-Methylierung gekennzeichnet ist, Entwicklungsstörung von ICF (Immundefizienz, Centromer-Instabilität und Anomalien des Gesichts), Rett-Syndrom und Fragiles-X-Syndrom ist; und wobei der pränatale Zustand Prader-Willi-Syndrom, Angelman-Syndrom, Beckwith-Wiedemann-Syndrom, Fragiles-X-Syndrom, Russel-Silver-Syndrom, transiente neonatale Diabetes mellitus, Martin-Albright-Syndrom, McCune-Albright-Syndrom, familiäres nichtchromaffines Paragangliom, maternales und paternales Syndrom ist.

**5.** Verfahren nach Anspruch 1, wobei die Referenzwerte mit denen die Signalverhältnisse verglichen werden, Kategorien darstellen, die Gemische aus anderen Kategorien sind.

**6.** Verfahren nach Anspruch 5, wobei die Referenzwerte, mit denen die Signalverhältnisse verglichen werden, aus Gemischen aus Sperma und Nicht-Sperma bei verschiedenen Verhältnissen sind.

**7.** Verfahren nach Anspruch 1, wobei die methylierungssensitive Restriktionsendonuclease ausgewählt ist aus der Gruppe bestehend aus AatII, Acc651, AccI, AciI, ACII, AfeI, AgeI, ApaI, ApaLI, AscI, AsiSI, AvaI, AvaII, BaeI, BanI, BbeI, BceAI, BcgI, BfuCI, BglI, BmgBI, BsaAI, BsaBI, BsaHI, BsaI, BseYI, BsiEI, BsiWI, BslI, BsmAI, BsmBI, BsmFI, BspDI, BsrBI, BsrFI, BssHII, BssKI, BstAPI, BstBI, BstUI, BstZI7I, Cac8I, ClaI, DpnI, DrdI, EaeI, EagI, EagI-HF, EciI, EcoRI, EcoRI-HF, FauI, Fnu4HI, FseI, FspI, HaeII, HgaI, HhaI, HincII, HincII, HinfI, HinP1I, HpaI, HpaII, Hpy166ii, Hpyl88iii, Hpy99I, HpyCH4IV, KasI, MluI, MmeI, MspA1I, MwoI, NaeI, NarI, NgoNIV, Nhe-HFI, NheI, NlaIV, NotI, NotI-HF, NruI, Nt.BbvCI, Nt,BsmAI, Nt.CviPII, PaeR7I, PleI, PmeI, PmlI, PshAI, PspOMI, PvuI, RsaI, RsrII, SacII, SalI, SalI-HF, Sau3AI, Sau96I, ScrFI, SfiI, SfoI, SgrAI, SmaI, SnaBI, TfiI, TscI, TseI, TspMI und ZraI; und wobei die methylierungsabhängige Restriktionsendonuclease ausgewählt ist aus der Gruppe bestehend aus McrBC, McrA und MrrA.

**8.** Verfahren nach Anspruch 1, wobei die methylierungssensitive Restriktionsendonuclease HhaI ist.

**9.** Verfahren nach Anspruch 2, wobei die Referenz-DNA-Kategorie eine natürliche DNA oder eine künstlich synthetisierte DNA ist, wobei mindestens ein genomischer Locus in der natürlichen DNA oder künstlich synthetisierten DNA einen Core-Short-Tandem-Repeat (STR)-Locus umfasst, der für das DNA-Profiling verwendet wird.

**10.** Verfahren nach Anspruch 9, wobei der genomische Locus einen humanen Locus umfasst, der ausgewählt ist aus der Gruppe bestehend aus D16S539 (SEQ ID NO:1), D7S820 (SEQ ID NO:2), D13S317 (SEQ ID NO:3), D5S818 (SEQ ID NO:4), CSF1 PO (SEQ ID NO:5), TPOX (SEQ ID NO:6), TH01 (SEQ ID NO:7), vWA (SEQ ID NO:8), FGA (SEQ ID NO:9), D21S11 (SEQ ID NO:10), D8S1179 (SEQ ID NO:11), D18S51 (SEQ ID NO:12), und D3S1358 (SEQ ID NO:13), Penta D (SEQ ID NO:14), Penta E (SEQ ID NO:15) und Amelogenin (SEQ ID NOs:16 und 17), D2S1338 (SEQ ID NO:18), D19S433 (SEQ ID NO:19), ACTBP2SE33 (SEQ ID NO:20), D10S1248 (SEQ ID NO:21), D1S1656 (SEQ ID NO:22), D22S1045 (SEQ 1D NO:23), D2S441 (SEQ ID NO:24) und D12S391 (SEQ ID NO:25).

**11.** Verfahren nach Anspruch 2, wobei die Referenz-DNA-Kategorie eine natürliche DNA oder eine künstlich synthetisierte DNA ist, wobei mindestens ein genomischer Locus bekanntermaßen in allen Geweben natürlicher DNA methyliert ist oder bekanntermaßen in allen Geweben natürlicher DNA nicht methyliert ist.

**12.** Verfahren nach Anspruch 11, wobei der genomische Locus, der bekanntermaßen in allen Geweben natürlicher DNA methyliert ist, TPOX (SEQ ID NO:6) oder FGA (SEQ ID NO:9) ist, und wobei der genomische Locus, der bekanntermaßen in allen Geweben natürlicher DNA nicht methyliert ist, Hypo23 (SEQ ID NO:29), Hypo28 (SEQ ID NO:30) oder Hypo33 (SEQ ID NO:31) ist.

**13.** Verfahren nach Anspruch 9, wobei die Amplifikationsprimer von mindestens einem der genomischen Loci erhältlich sind in:

(1) einem von Promega Corporation kommerziell erhältlichen Kit, der ausgewählt ist aus der Gruppe bestehend aus PowerPlex® 16 HS (Cat.# DC2100, DC2101), PowerPlex® 16 (Cat.# DC6530, DC6531), PowerPlex® 2.1 (Cat.# DC6470, DC6471), PowerPlex® 16 BIO (Cat.# DC6540, DC6541) und PowerPlex® ES Systems (Cat.# DC6730, DC6731);
(2) einem von Applied Biosystems kommerziell erhältlichen Kit, der ausgewählt ist aus der Gruppe bestehend aus SGM, SGM+, AmpFISTR Identifiler, AmpFISTR Profiler, AmpFISTR ProfilerPlus, AmpFISTR ProfilerPlusID, AmpFISTR SEfiler, AmpFISTR SEfiler Plus, AmpFISTR Cofiler, AmpFISTR Identifiler Direct, AmpFISTR Identifiler Plus, AmpFISTR NGM, AmpFISTR Y-filer und AmpFISTR Minifiler; oder
(3) Investigator ESSPlex, Investigator ESSPlex SE, Investigator Nonaplex ESSPlex, Investigator Hexaplex ESSPlex, Investigator Triplex AFS QS, Investigator Triplex DSF, Investigator IDplex, Investigator Decaplex SE, Investigator HDplex, Investigator Argus X-12, Investigator Y-12 QS, Investigator DIPplex.

**14.** Verfahren nach Anspruch 9, des Weiteren umfassend das Berechnen von mindestens einem der folgenden Verhältnisse:

(1) D3S1358/D18851,
(2) D3S1358/D7880,
(3) D3S1358/Penta_D,
(4) D3S1358/TPOX,
(5) D3S1358/FGA,
(6) TH01/Penta_D,
(7) D21S11/D18851,
(8) D21S11/D7S820,
(9) D21S11/Penta_D,
(10) D21S11/AMEL,
(11) D21S11/TPOX,
(12) D21S11/FGA,
(13) D5S818/D18S51,
(14) vWA/D18S51,
(15) D5S818/Penta_E,
(16) vWA/penta_E,
(17) D5S818/D7S820,
(18) D5S818/Penta_D,
(19) D5S818/TPOX,
(20) D5S818/FGA,
(21) D13S317/D7S820,
(22) D13S317/Penta_D,
(23) D13S317/TPOX,
(24) D13S317/FGA,
(25) D16S539/D7SR20,
(26) CSF1PO/D7S820,
(27) vWA/D7S820,
(28) D8S1179/D7S820,
(29) D16S539/TPOX,
(30) D16S539/FGA,
(31) CSF1PO/Penta_D,
(32) CSF1PO/TPOX,
(33) vWA/Penta_D,
(34) AMEL/TPOX,
(35) AMEL/FGA,
(36) vWA/D8S1179,
(37) vWA/TPOX,
(38) vWA/FGA,
(39) D8S1179/TPOX, und
(40) D8S1179/FGA.

**15.** Verfahren nach Anspruch 1, wobei die Signalintensität der Fluroeszenzspiegel des Amplifikationsprodukts ist, der während der Kapillarelektrophorese gemessen wird.

**16.** Verfahren nach Anspruch 1, wobei die Schritte (B) und (C) mittels Real-Time-PCR durchgeführt werden.

**17.** Verfahren nach Anspruch 16, wobei die Signalintensität jedes Amplifikationsprodukts der Fluoreszenzspiegel jedes Amplifikationsprodukts ist.

**18.** Verfahren nach Anspruch 1, des Weiteren umfassend das Zuordnen eines Konfidenzniveaus zu potenziellen Kategorien einer DNA-Probe, wobei das Konfidenzniveau die Wahrscheinlichkeit widerspiegelt, dass eine bestimmte DNA-Kategorie auf die Kategoriequelle der DNA-Probe hinweist; wobei die Wahrscheinlichkeit berechnet wird durch:

(A) Zuordnen eines Wahrscheinlichkeitswertes zu jedem Vergleich eines Signalverhältnisses mit den Referenzwerten, die den verschiedenen DNA-Kategorien entsprechen, wobei der Wahrscheinlichkeitswert erhalten wird durch (i) Zuordnen einer Gamma-Wahrscheinlichkeits-Verteilungsfunktion zu den Referenzwerten jeder potenziellen Kategorie; wobei (ii) der Wahrscheinlichkeitswert mit dem Wert der in Schritt (A) zugeordneten Gamma-Wahrscheinlichkeits-Verteilungsfunktion bei dem beobachteten Signalverhältnis identisch ist;
(B) für jede Kategorie, Berechnen des Kategorie-Wahrscheinlichkeitswertes, der das Produkt der in Schritt (A) erhaltenen Wahrscheinlichkeitswerte ist; und
(C) Normalisieren der Wahrscheinlichkeitswerte durch Dividieren des Kategorie-Wahrscheinlichkeitswertes jeder potenziellen Kategorie durch die Summe aller Kategorie-Wahrscheinlichkeitswerte;

wobei das Konfidenzniveau jeder Kategorie der normalisierte Wahrscheinlichkeitswert dieser Kategorie ist.

**19.** Verfahren nach Anspruch 1, des Weiteren umfassend das gleichzeitige Durchführen von DNA-Profiling mit der DNA-Kategorisierung.

**20.** Kit für das Kategorisieren einer DNA-Probe als Sperma oder Nicht-Sperma, umfassend:

(a) ein Primer-Mix, umfassend die folgenden Primer:

SD1f (AAGAGCCCATCAGGCAGGTC);
SD1r (GTTTCTTGTCGAGCAGCACGTGGATGATG);
SD2f (CTCCACAACTGGAACTTCCTG);
SD2r (GTTTCTTAACTTGGAGACGACGGCATC);
SD3f (TGGAGGACAATGCCCTGGTG);
SD3r (GTTTCTTGGCTTCACCTGCGACCGTCTC);
SD4f (CCCTCCGAGTGGCCAGCAG);
SD4r (GTTTCTGACCACTGCCGTGGGAATG);
SD5f (CTTCTCAGCCAATGGGAAGAG);
SD5r (ACGTAGAAGGACCCGAGGAC);

(b) Reaktionspuffer;
(c) HhaI-Restriktionsendonuclease; und
(d) DNA-Polymerase;

wobei mindestens ein Paar der Forward (f)- und Reverse (r)-Primerpaar-Kombinationen ausgewählt ist aus der Gruppe bestehend aus:

(1) SD1f (AAGAGCCCATCAGGCAGGTC) und SD1r (GTTTCTTGTCGAGCAGCACGTGGATGATG);
(2) SD2f (CTCCAGAACTGGAACTTCCTG) und SD2r (GTTTCTTAACTTGGAGACGACGGCATC);
(3) SD3f (TGGAGGACAATGCCCTGGTG) und SD3r (GTTTCTTGGCTTCACCTGCGACCGTCTC);
(4) SD4f (CCCTCCGAGTGGCCAGCAG) und SD4r (GTTTCTGACCACTGCCGTGGAATG);
(5) SD5f (CTTCTCAGCCAATGGGAAGAG) und SD5r (ACGTAGAAGGACCCGAGGAC).

**21.** Kit nach Anspruch 20, wobei die Konzentration der Primer in dem Primer-Mix sind: 0,6 μM SD1f, 0,6 μM SD1r, 1,75 μM SD2f, 1,75 μM SD2r, 1,25 μM SD3f, 1,25 μM SD3r, 1,75 μM SD4f, 1,75 μM SD4r, 1,75 μM SD5f und 1,75 μM SD5r.

**22.** Kit zum Profiling, zur Kategorisierung einer DNA-Probe als Sperma oder Nicht-Sperma und zum Erhalten eines assoziierten Kategorisierungs-Konfidenzniveaus, umfassend:

(a) Primer zum Amplifizieren mindestens eines Sperma-spezifischen Locus;
(b) Primer zum Amplifizieren mindestens eines Locus, der für das DNA-Profiling verwendet wird;
(c) Reaktionspuffer;
(d) HhaI-Restriktionsendonuclease, und
(e) DNA-Polymerase, wobei

der mindestens eine Sperma-spezifische Locus der L68346-Locus ist, und wobei der Primer für die Amplifizierung eines 70 bp Sperma-spezifischen Amplifizierungsprodukts aus L68346 ein Forward-Primer umfassend die Sequenz CAGCAACAGCACCCAGCTTG (FAM) und ein Reverse-Primer umfassend die Sequenz CACAGGCTCAGTCGCGGATC sind; oder

wobei der mindestens eine Sperma-spezifische Locus der Locus L16264 ist, und wobei die Primer für die Amplifizierung eines 95 bp Sperma-spezifischen Amplifizierungsprodukts aus L16264 ein Forward-Primer umfassend die Sequenz GGACGAGTTAACTTCCTTAATTTC (FAM) und ein Reverse-Primer umfassend die Sequenz GTTTCTTCGCGGAACCTGGTTTAACTTC sind.

**23.** Kit zum Kategorisieren einer DNA-Probe als Blut, Speichel, Sperma oder Haut-Epidermis und zum Erhalten eines assoziierten Kategorisierungs-Konfidenzniveaus, umfassend.

(a) Primer-Mix, der Forward- und Reverse-Primer für die Amplifizierung der genannten Loci wie folgt umfasst:

1. L91762 (forward GCAGCAGGCCGCGGAGAAG (FAM); reverse AGCAGCTGTGCCGGGCCAG)
2. L68346 (forward CAGCAACAGCACCCAGCTTG (JOE); reverse CACAGGCTCAGTCGCGGATC)
3. L50468 (forward AGGAAACCTCAGTAGCAAAATTG (JOE); reverse GCGAGACTTTAGGTGTGCATC)
4. L14432 (forward CGTAGGCTGCGGTGAGCTC (FAM); reverse GATCCATGCCCGCTGGGATG)
5. L4648 (forward CAGCCTAGACGTCAAGTTACAG (JOE); reverse ACGACCTCCGGATCCAACTG)
6. L39664 (forward CCCAGCTGGTTGGACATGTTG (FAM); reverse CACTTCCTTCGTGGACGCC)
7. L30139 (forward GAGAAGCGGGAGGATGAGAC (FAM); reverse CCGCATCTCCTCCGTCCTG)
8. L55429 (forward GCCTTCAGCAGGAAGTCCAC (JOE); reverse CCTGTGCCTCACACAGACTC)
9. L62086 (forward GTGCATGGTGTCTGGTACTTC (FAM); reverse GAAGCTCTCGTGGACTACTTG)
10. L76138 (forward CAGCCTGCTCTTCACTGCAG (JOE); reverse AGAGGCCGATGAAGCCGTAG)
11. L15952 (forward CTCCCTGATTTACGACAAGTTC (FAM); reverse GACAGTATGCTGATGCTTCTTG)
12. L36599 (forward AAGGGCAGAGTTCCGCTGTC (FAM); reverse CGGATGCAGGAGGATCCTAG)
13. L26688 (forward CGGACCAGATTGCTGGTCAC (JOE); reverse CGAGCTTGCCAGATGTTTGAC)
14. L81528 (forward AGCCTCATCCACACTGACCAG (JOE); reverse TCAGAGCTCTCCTATCTGGAC)
15. L36556 (forward GCCAGGCCGTTGATGATGAC (JOE); reverse GAATATGGAGCCCTGGGCAG)

(b) Reaktionspuffer;
(c) Hhal-Restriktionsendonuclease; und
(d) DNA-Polymerase.

## Revendications

**1.** Procédé pour catégoriser un échantillon d'ADN en déterminant des rapports de signal entre des loci génomiques co-amplifiés dans la même réaction, comprenant :

(A) la digestion d'un échantillon d'ADN avec une endonucléase de restriction sensible à la méthylation ou une endonucléase de restriction méthylation-dépendante ;
(B) l'amplification d'au moins deux loci génomiques à partir de l'ADN digéré, où au moins un des loci est un locus de restriction ;
(C) la détermination de l'intensité de signal du produit d'amplification de chaque locus ;
(D) le calcul des rapports de signal entre les intensités de signal des produits d'amplification ; et
(E) la comparaison des rapports de signal à des valeurs de référence de différentes catégories d'ADN,

dans lequel l'approximation de la valeur d'un rapport de signal aux valeurs des rapports de référence d'une catégorie d'ADN particulière indique la source catégorielle de l'échantillon d'ADN dans lequel les loci génomiques qui sont amplifiés sont choisis pour produire des rapports de signal distincts pour différentes catégories potentielles, où aucune courbe d'étalonnage ou ADN de référence n'est nécessaire et où aucun véritable niveau de méthylation à un quelconque locus génomique n'est indiqué par ledit procédé.

**2.** Procédé selon la revendication 1, dans lequel les valeurs de référence contre lesquelles les rapports de signal sont comparés proviennent d'au moins une catégorie d'ADN sélectionnée dans le groupe consistant en un type tissulaire, un type cellulaire, un état physiologique, un état pathologique, l'âge, l'ethnicité, le sexe, le niveau de méthylation, l'espèce, des lignées cellulaires, de l'ADN naturel et de l'ADN synthétisé de manière artificielle, le risque de développement d'un état pathologique, des affections prénatales, un pronostic, une propension à répondre à un traitement, les effets d'une sous-culture de lignée cellulaire, la réponse à un médicament.

**3.** Procédé selon la revendication 2, dans lequel le type tissulaire ou le type cellulaire est sélectionné dans le groupe consistant en du sang, de la salive, du sperme, de l'épiderme, de l'urine, du plasma, un poil, du sang menstruel, des cellules et/ou sécrétions vaginales, de la sueur, des selles, le cerveau, l'oesophage, le poumon, l'estomac, le coeur, le duodénum, le foie, la vésicule biliaire, l'intestin, le rein, la glande surrénale, la vessie, l'urètre, le côlon, le testicule, l'ovaire, l'utérus, le vagin, un muscle, un tendon, un ligament, le tissu adipeux, le cartilage, l'os, des cellules endothéliales, le col de l'utérus, la lymphe, la thyroïde, l'hypophyse, le cervelet, et le sein.

**4.** Procédé selon la revendication 2, dans lequel l'état pathologique est le cancer, une inflammation, un trouble auto-immun, un trouble métabolique, une infection, une maladie dégénérative, des déséquilibres hormonaux, un trouble **caractérisé par** une méthylation de l'ADN anormale, un trouble neurodéveloppemental d'ICF (immunodéficience, instabilité centromérique et anomalies faciales), le syndrome de Rett, et le syndrome de l'X fragile ; et dans lequel l'affection prénatale est le syndrome de Prader-Willi, le syndrome d'Angelman, le syndrome de Beckwith-Wiedemann, le syndrome de l'X fragile, le syndrome de Silver-Russell, le diabète néonatal transitoire, l'ostéodystrophie héréditaire d'Albright, le syndrome de McCune-Albright, le paragangliome non chromaffine familial, des syndromes d'UPD14 maternelle et paternelle.

**5.** Procédé selon la revendication 1, dans lequel les valeurs de référence contre lesquelles les rapports de signal sont comparés représentent des catégories qui sont des mélanges d'autres catégories.

**6.** Procédé selon la revendication 5, dans lequel les valeurs de référence contre lesquelles les rapports de signal sont comparés proviennent de mélanges de sperme et de non sperme à divers rapports.

**7.** Procédé selon la revendication 1, dans lequel l'endonucléase de restriction sensible à la méthylation est sélectionnée dans le groupe consistant en AatII, Acc651, AccI, AciI, ACII, AfeI, AgeI, ApaI, ApaLI, AscI, AsiSI, AvaI, AvaII, BaeI, BanI, BbeI, BceAI, BcgI, BfuCI, BglI, BmgBI, BsaAI, BsaBI, BsaHI, BsaI, BseYI, BsiEI, BsiWI, BslI, BsmAI, BsmBI, BsmFI, BspDI, BsrBI, BsrFI, BssHII, BssKI, BstAPI, BstBI, BstUI, BstZ17I, Cac8I, ClaI, DpnI, DrdI, EaeI, EagI, EagI-HF, EciI, EcoRI, EcoRI-HF, FauI, Fnu4HI, FseI, FspI, HaeII, HgaI, HhaI, HincII, HincI, HinfI, HinPII, HpaI, HpaII, Hpy166ii, Hpy188iii, Hpy99I, HpyCH4IV, KasI, MluI, MmeI, MspAII, MwoI, NaeI, NarI, NgoNIV, Nhe-HFI, NheI, NlaIV, NotI, NotI-HF, NruI, Nt.BbvCI, Nt.BsmAI, Nt.CviPII, PaeR7I, PleI, PmeI, PmlI, PshAI, PspOMI, PvuI, RsaI, RsrII, SacII, SalI, SalI-HF, Sau3AI, Sau96I, ScrFI, SfiI, SfoI, SgrAI, SmaI, SnaBI, TfiI, TscI, TseI, TspMI, et ZraI ; et dans lequel l'endonucléase de restriction méthylation-dépendante est sélectionnée dans le groupe consistant en McrBC, McrA, et MrrA.

**8.** Procédé selon la revendication 1, dans lequel l'endonucléase de restriction sensible à la méthylation est HhaI.

**9.** Procédé selon la revendication 2, dans lequel la catégorie d'ADN de référence est de l'ADN naturel ou de l'ADN synthétisé de manière artificielle, dans lequel au moins un locus génomique dans l'ADN naturel ou l'ADN synthétisé de manière artificielle comprend un locus de courte répétition en tandem (STR) de noyau utilisé pour le profilage de l'ADN.

**10.** Procédé selon la revendication 9, dans lequel le locus génomique comprend un locus humain sélectionné dans le groupe consistant en D16S539 (SEQ ID NO: 1), D7S820 (SEQ ID NO: 2), D13S317 (SEQ ID NO: 3), D5S818 (SEQ ID NO: 4), CSF1 PO (SEQ ID NO: 5), TPOX (SEQ ID NO: 6), TH01 (SEQ ID NO: 7), vWA (SEQ ID NO: 8), FGA (SEQ ID NO: 9), D21S11 (SEQ ID NO: 10), D8S1179 (SEQ ID NO: 11), D18S51 (SEQ ID NO: 12), et D3S1358 (SEQ ID NO: 13), Penta D (SEQ ID NO: 14), Penta E (SEQ ID NO: 15), et l'amélogénine (SEQ ID NO: 16 et 17), D2S1338 (SEQ ID NO: 18), D19S433 (SEQ ID NO: 19), ACTBP2SE33 (SEQ ID NO: 20), D10S1248 (SEQ ID NO: 21), D1S1656 (SEQ ID NO: 22), D22S1045 (SEQ ID NO: 23), D2S441 (SEQ ID NO: 24), et D12S391 (SEQ ID NO: 25).

**11.** Procédé selon la revendication 2, dans lequel la catégorie d'ADN de référence est de l'ADN naturel ou de l'ADN synthétisé de manière artificielle, où au moins un locus génomique est connu pour être méthylé dans tous les tissus d'ADN naturel, ou est connu pour être non méthylé dans tous les tissus d'ADN naturel.

**12.** Procédé selon la revendication 11, dans lequel le locus génomique connu pour être méthylé dans tous les tissus d'ADN naturel est TPOX (SEQ ID NO: 6) ou FGA (SEQ ID NO: 9), et dans lequel le locus génomique connu pour être non méthylé dans tous les tissus d'ADN naturel est Hypo23 (SEQ ID NO: 29), Hypo28 (SEQ ID NO: 30) ou Hypo33 (SEQ ID NO: 31).

**13.** Procédé selon la revendication 9, dans lequel les amorces pour l'amplification d'au moins un des loci génomiques sont disponibles dans :

(1) un kit commercial de Promega Corporation sélectionné dans le groupe consistant en PowerPlex® 16 HS (n° réf. DC2100, DC2101), PowerPlex® 16 (n° réf. DC6530, DC6531), PowerPlex® 2.1 (n° réf. DC6470, DC6471), PowerPlex® 16 BIO (n° réf. DC6540, DC6541), et PowerPlex® ES Systems (n° réf. DC6730, DC6731) ;
(2) un kit commercial de Applied Biosystems sélectionné dans le groupe consistant en SGM, SGM+, AmpFISTR Identifiler, AmpFISTR Profiler, AmpFISTR ProfilerPlus, AmpFISTR ProfilerPlusID, AmpFISTR SEfiler, AmpFISTR SEfiler Plus, AmpFISTR Cofiler, AmpFISTR Identifiler Direct, AmpFISTR Identifiler Plus, AmpFISTR NGM, AmpFISTR Y-filer, et AmpFISTR Minifiler ; ou
(3) Investigator ESSPlex, Investigator ESSPlex SE, Investigator Nonaplex ESSPlex, Investigator Hexaplex ESSPlex, Investigator Triplex AFS QS, Investigator Triplex DSF, Investigator IDplex, Investigator Decaplex SE, Investigator HDplex, Investigator Argus X-12, Investigator Y-12 QS, Investigator DIPplex.

**14.** Procédé selon la revendication 9, comprenant en outre le calcul d'au moins un de rapports suivants :

(1) D3S1358/D18S51,
(2) D3S1358/D7S820,
(3) D3S1358/Penta_D,
(4) D3S1358/TPOX,
(5) D3S1358/FGA,
(6) TH01/Penta-D,
(7) D21S11/D18S51,
(8) D21S11/D7S820,
(9) D21S11/Penta_D
(10) D21S11/AMEL,
(11) D21S11/TPOX,
(12) D21S11/FGA,
(13) D5S818/D18S51,
(14) vWA/D18S51,
(15) D5S818/Penta_E,
(16) vWA/Penta_E,
(17) D5S818/D7S820,
(18) D5S818/Penta_D,
(19) D5S818/TPOX,
(20) D5S818/FGA,
(21) D135317/D7S820,
(22) D13S317/Penta_D,
(23) D13S317/TPOX,
(24) D13S317/FGA,
(25) D16S539/D7S820,
(26) CSF1PO/D7S820,
(27) vWA/D7S820,
(28) D8S1179/D7S820,
(29) D16S539/TPOX,
(30) D16S539/FGA,
(31) CSF1PO/Penta_D,
(32) CSF1PO/TPOX,
(33) vWA/Penta_D,
(34) AMEL/TPOX,
(35) AMEL/FGA,
(36) vWA/D8S1179,
(37) vWA/TPOX,
(38) vWA/FGA,
(39) D8S1179/TPOX, et
(40) D8S1179/FGA.

**15.** Procédé selon la revendication 1, dans lequel l'intensité de signal est le niveau de fluorescence du produit d'amplification mesuré pendant une électrophorèse capillaire.

**16.** Procédé selon la revendication 1, dans lequel les étapes (B) et (C) sont réalisées par une PCR en temps réel.

**17.** Procédé selon la revendication 16, dans lequel l'intensité de signal de chaque produit d'amplification est le niveau de fluorescence de chaque produit d'amplification.

**18.** Procédé selon la revendication 1, comprenant en outre le fait d'attribuer des niveaux de confiance aux catégories potentielles d'un échantillon d'ADN, dans lequel le niveau de confiance reflète la probabilité qu'une catégorie d'ADN particulière indique la source catégorielle de l'échantillon d'ADN, dans lequel la probabilité est calculée par :

(A) l'attribution d'un score de probabilité à chaque comparaison d'un rapport de signal aux valeurs de référence qui correspondent aux diverses catégories d'ADN, où le score de probabilité est obtenu par (i) l'attribution d'une fonction de distribution de probabilité gamma aux valeurs de référence de chaque catégorie potentielle ; où (ii) le score de probabilité est égal à la valeur de la fonction de distribution de probabilité gamma attribuée à l'étape (A) au rapport de signal observé ;
(B) pour chaque catégorie, le calcul du score de probabilité de catégorie, qui est le produit des scores de probabilité obtenus à l'étape (A) ; et
(C) la normalisation des scores de probabilité en divisant le score de probabilité de catégorie de chaque catégorie potentielle par la somme de tous les scores de probabilité de catégorie ;

où le niveau de confiance de chaque catégorie est le score de probabilité normalisé de cette catégorie.

**19.** Procédé selon la revendication 1, comprenant en outre la réalisation simultanée d'un profilage de l'ADN avec la catégorisation de l'ADN.

**20.** Kit pour catégoriser un échantillon d'ADN en tant que sperme ou non sperme, comprenant :

(a) un mélange d'amorces, comprenant les amorces suivantes :

SD1f (AAGAGCCCATCAGGCAGGTC) ;
SD1 r (GTTTCTTGTCGAGCAGCACGTGGATGATG) ;
SD2f (CTCCAGAACTGGAACTTCCTG) ;
SD2r (GTTTCTTAACTTGGAGACGACGGCATC) ;
SD3f (TGGAGGACAATGCCCTGGTG) ;
SD3r (GTTTCTTGGCTTCACCTGCGACCGTCTC) ;
SD4f (CCCTCCGAGTGGCCAGCAG) ;
SD4r (GTTTCTGACCACTGCCGTGGGAATG) ;
SD5f (CTTCTCAGCCAATGGGAAGAG) ;
SD5r (ACGTAGAAGGACCCGAGGAC) ;

(b) un tampon de réaction ;
(c) une endonucléase de restriction HhaI ; et
(d) une ADN polymérase ;

dans lequel au moins une paire de combinaisons de paires d'amorces sens (forward - f) et antisens (reverse - r) est sélectionnée dans le groupe consistant en :

(1) SD1f (AAGAGCCCATCAGGCAGGTC) et SD1r (GTTTCTTGTCGAGCAGCACGTGGATGATG) ;
(2) SD2f (CTCCAGAACTGGAACTTCCTG) et SD2r (GTTTCTTAACTTGGAGACGACGGCATC) ;
(3) SD3f (TGGAGGACAATGCCCTGGTG) et SD3r (GTTTCTTGGCTTCACCTGCGACCGTCTC) ;
(4) SD4f (CCCTCCGAGTGGCCAGCAG) et SD4r (GTTTCTGACCACTGCCGTGGGAATG) ;
(5) SD5f (CTTCTCAGCCAATGGGAAGAG) et SD5r (ACGTAGAAGGACCCGAGGAC).

**21.** Kit selon la revendication 20, dans lequel la concentration des amorces dans le mélange d'amorces est : SD1f 0,6 μM, SD1r 0,6 μM, SD2f 1,75 μM, SD2r 1,75 μM, SD3f 1,25 μM, SD3r 1,25 μM, SD4f 1,75 μM, SD4r 1,75 μM, SD5f 1,75 μM et SD5r 1,75 μM.

**22.** Kit pour profiler, catégoriser un échantillon d'ADN en tant que sperme ou non sperme, et obtenir un niveau de confiance de catégorisation associé, comprenant :

(a) des amorces pour amplifier au moins un locus spécifique au sperme ;
(b) des amorces pour amplifier au moins un locus utilisé pour le profilage de l'ADN ;
(c) un tampon de réaction ;
(d) une endonucléase de restriction Hhal, et
(e) une ADN polymérase, dans lequel

le au moins un locus spécifique au sperme est le locus L68346, et dans lequel les amorces pour amplifier un produit d'amplification spécifique au sperme de 70 pb de L68346 sont une amorce sens comprenant la séquence de CAGCAACAGCACCCAGCTTG (FAM) et une amorce antisens comprenant la séquence de CACAGGCTCAGTCGCGGATC ; ou
dans lequel le au moins un locus spécifique au sperme est le locus L16264 et dans lequel les amorces pour amplifier un produit d'amplification spécifique au sperme de 95 pb de L16264 sont une amorce sens comprenant la séquence de GGACGAGTTAACTTCCTTAATTTC (FAM) et une amorce antisens comprenant la séquence de GTTTCTTCGCGGAACCTGGTTTAACTTC.

**23.** Kit pour catégoriser un échantillon d'ADN en tant que sang, salive, sperme, ou épiderme de la peau, et pour obtenir un niveau de confiance de catégorisation associé, comprenant :

(a) un mélange d'amorces qui comprend des amorces sens et antisens pour amplifier les loci désignés de la manière suivante :

1. L91762 (sens GCAGCAGGCCGCGGAGAAG (FAM) ; antisens AGCAGCTGTGCCGGGCCAG)
2. L68346 (sens CAGCAACAGCACCCAGCTTG (JOE) ; antisens CACAGGCTCAGTCGCGGATC)
3. L50468 (sens AGGAAACCTCAGTAGCAAAATTG (JOE); antisens GCGAGACTTTAGGTGTGCATC)
4. L14432 (sens CGTAGGCTGCGGTGAGCTC (FAM) ; antisens GATCCATGCCCGCTGGGATG)
5. L4648 (sens CAGCCTAGACGTCAAGTTACAG (JOE); antisens ACGACCTCCGGATCCAACTG)
6. L39664 (sens CCCAGCTGGTTGGACATGTTG (FAM); antisens CACTTCCTTCGTGGACGCC)
7. L30139 (sens GAGAAGCGGGAGGATGAGAC (FAM); antisens CCGCATCTCCTCCGTCCTG)
8. L55429 (sens GCCTTCAGCAGGAAGTCCAC (JOE); antisens CCTGTGCCTCACACAGACTC)
9. L62086 (sens GTGCATGGTGTCTGGTACTTC (FAM); antisens GAAGCTCTCGTGGACTACTTG)
10. L76138 (sens CAGCCTGCTCTTCACTGCAG (JOE); antisens AGAGGCCGATGAAGCCGTAG)
11. L15952 (sens CTCCCTGATTTACGACAAGTTC (FAM); antisens GACAGTATGCTGATGCTTCTTG)
12. L36599 (sens AAGGGCAGAGTTCCGCTGTG (FAM); antisens CGGATGCAGGAGGATCCTAG)
13. L26688 (sens CGGACCAGATTGCTGGTCAC (JOE); antisens CGACCTTGCCAGATGTTTGAC)
14. L81528 (sens AGCCTCATCCACACTGACCAG (JOE); antisens TCAGAGCTCTCCTATCTGGAC)
15. L36556 (sens GCCAGGCCGTTGATGATGAC (JOE); antisens GAATATGGAGCCCTGGGCAG)

(b) un tampon de réaction ;
(c) une endonucléase de restriction Hhal ; et
(d) une ADN polymérase.

Figure 1

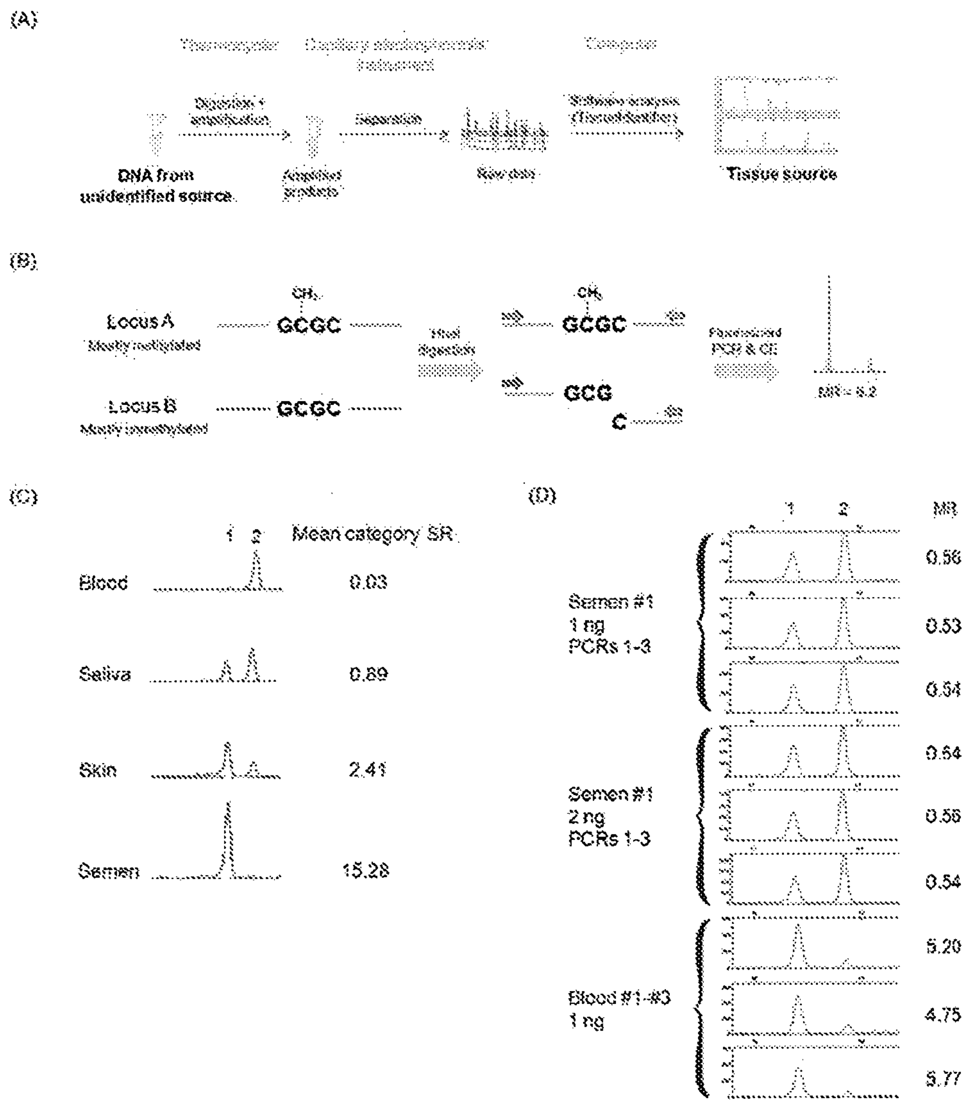
(A)
DNA from unidentified source
Tissue source
(B)
$CH_3$
Locus A
GCGC
Locus B
GCGC
GCGC
GCG
C
(C)
1 2 Mean category SR
Blood 0.03
Saliva 0.89
Skin 2.41
Semen 15.28
(D)
1 2 MR
Semen #1 1 ng PCRs 1-3
0.56
0.53
0.54
Semen #1 2 ng PCRs 1-3
0.54
0.56
0.54
Blood #1-#3 1 ng
5.20
4.75
5.77

Figure 2

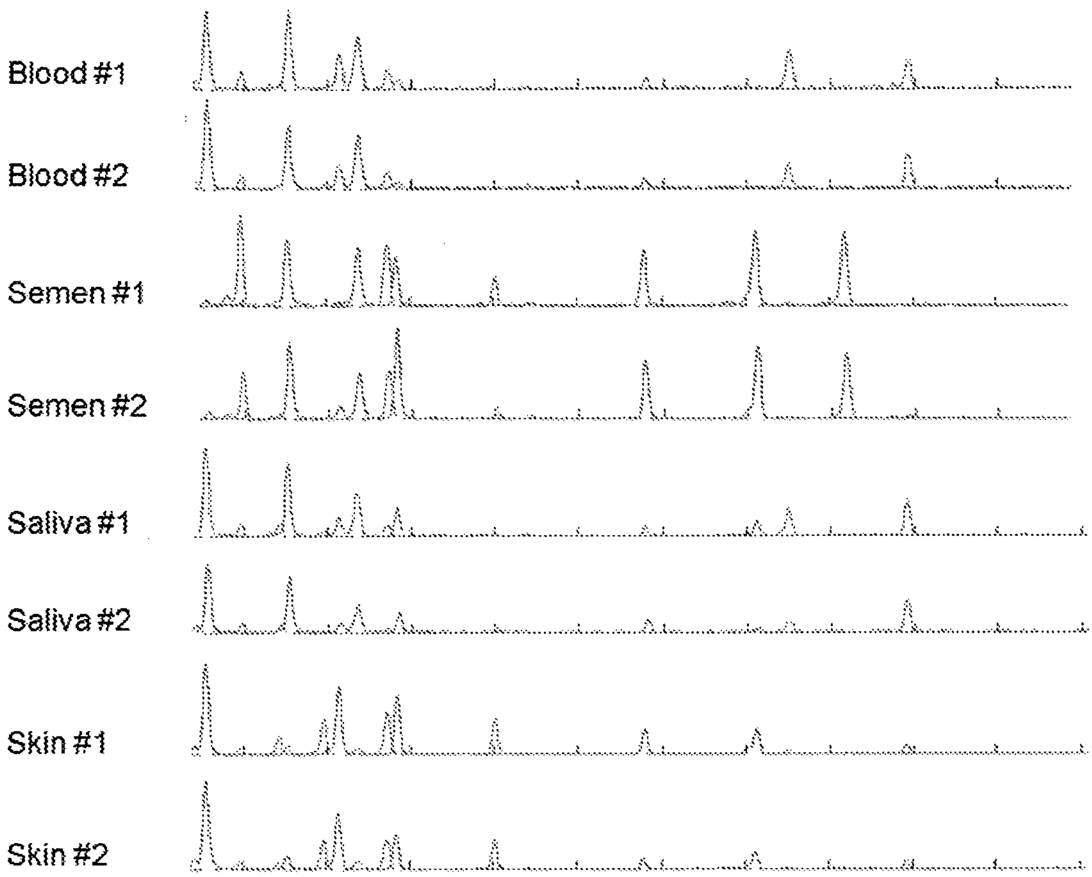

Figure 3

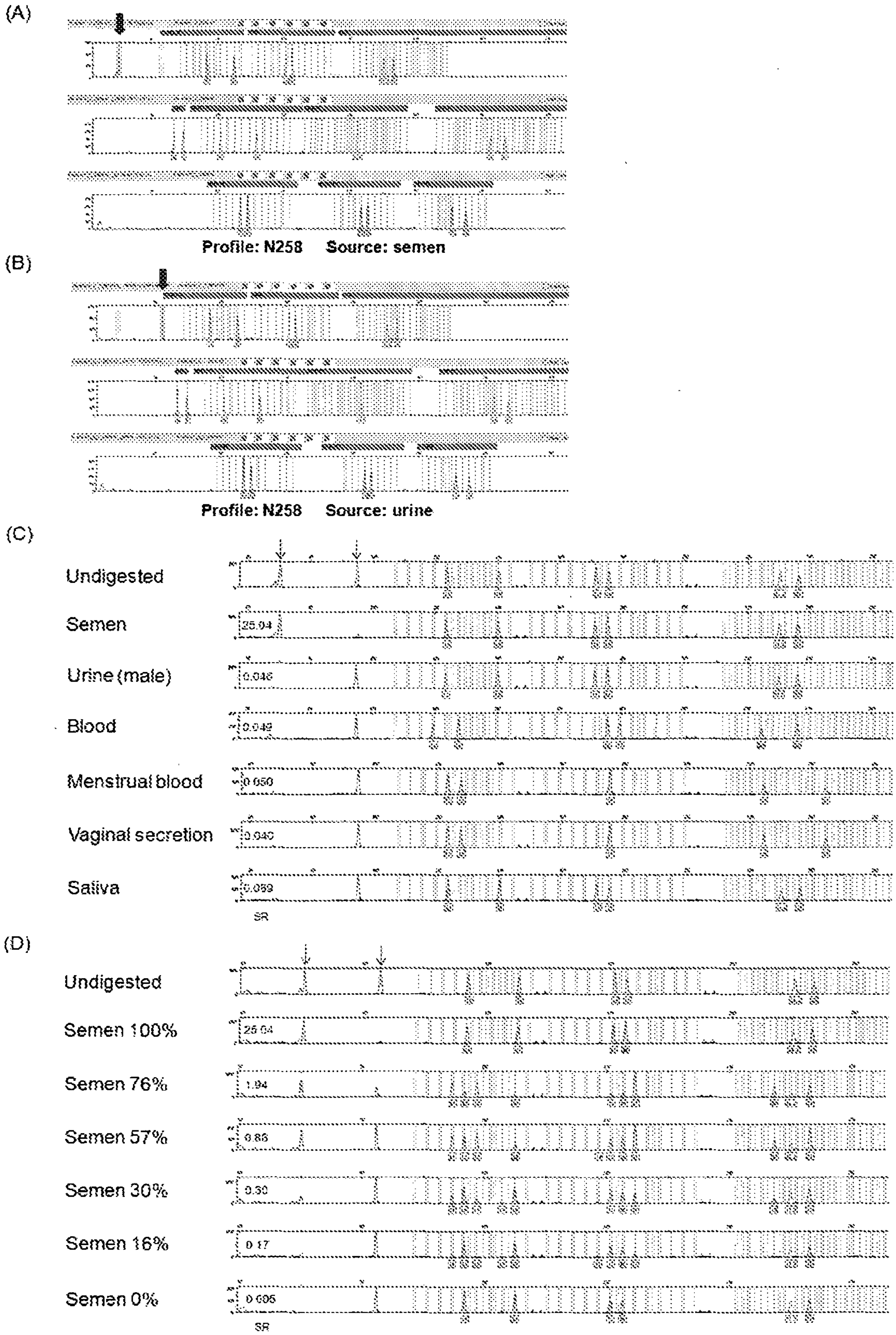
(A)
Profile: N258 Source: semen
(B)
Profile: N258 Source: urine
(C)
Undigested
Semen
25.04
Urine (male)
0.046
Blood
0.049
Menstrual blood
0.050
Vaginal secretion
0.040
Saliva
0.059
SR
(D)
Undigested
Semen 100%
25.04
Semen 76%
1.94
Semen 57%
0.86
Semen 30%
0.30
Semen 16%
0.17
Semen 0%
0.005
SR

Figure 4

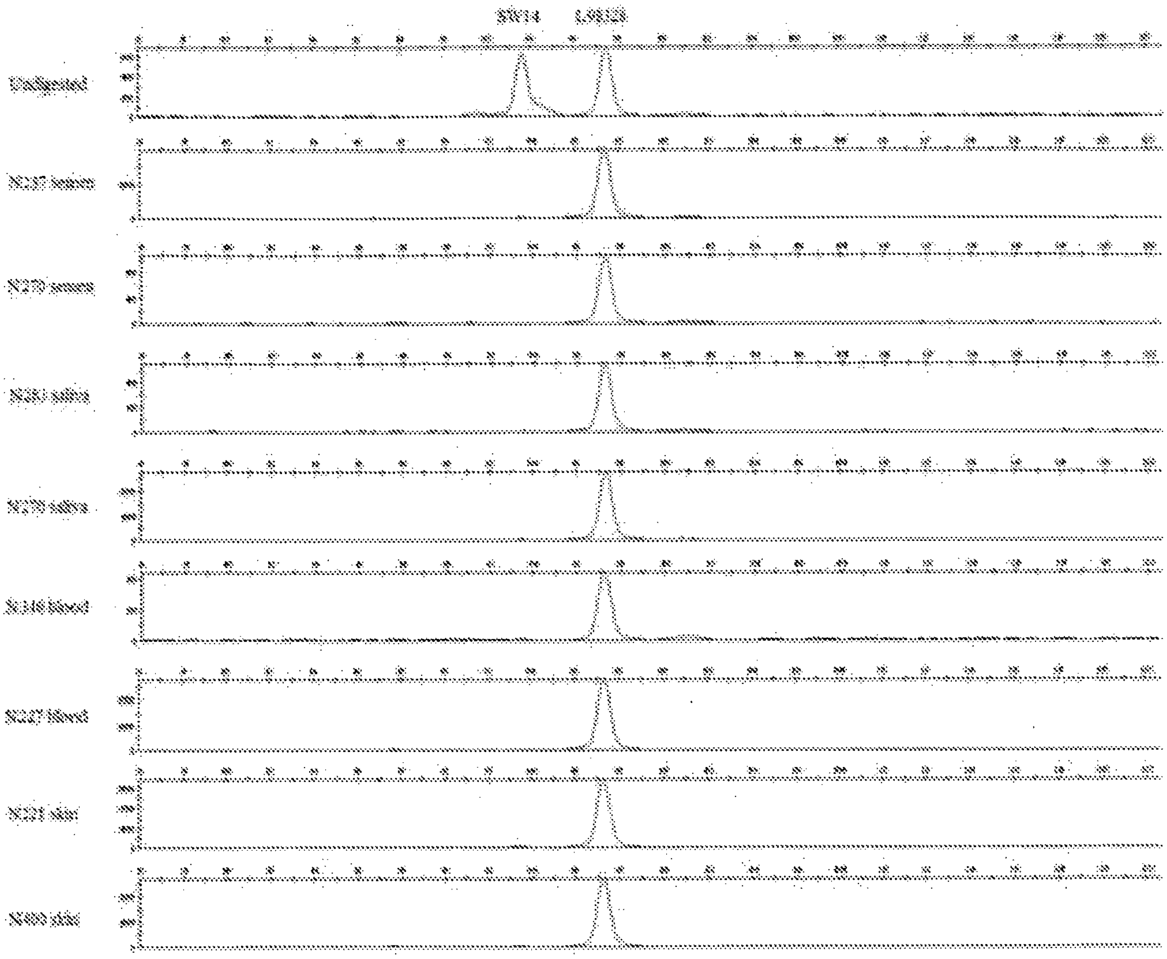

Figure 5

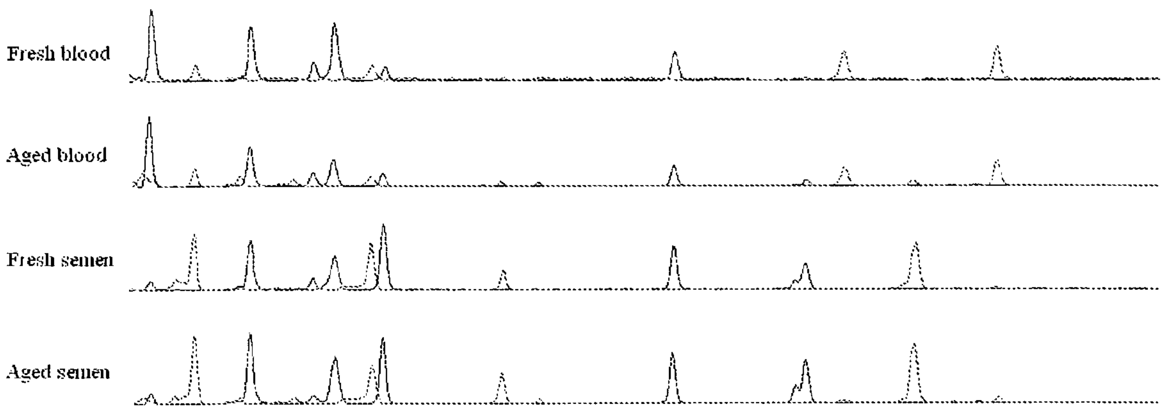

Figure 6

DNAseI-treated blood sample

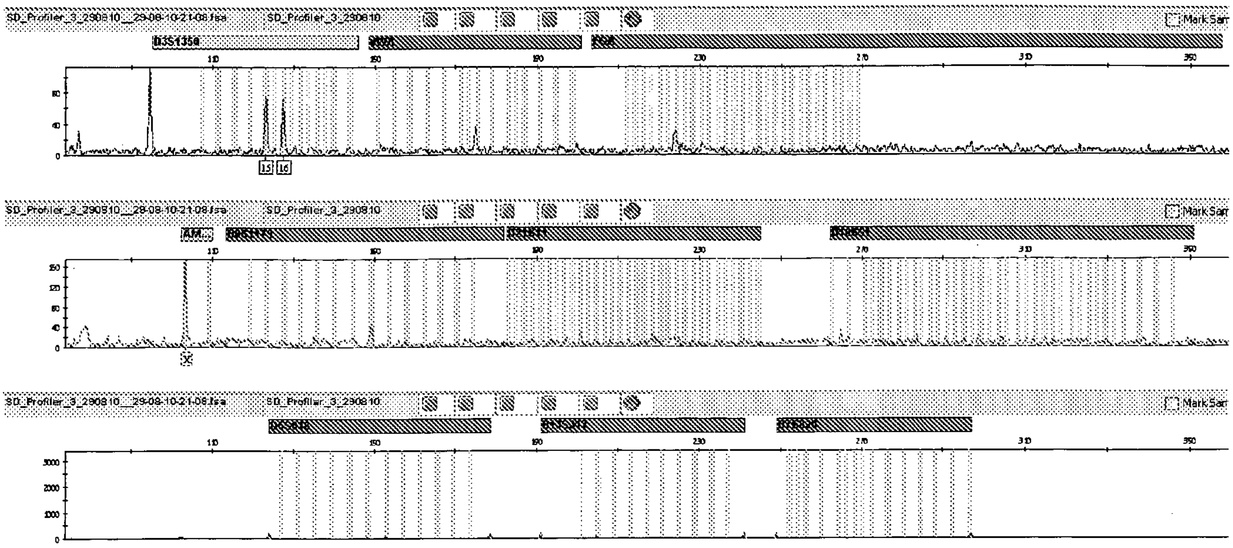


DNAseI-treated semen sample

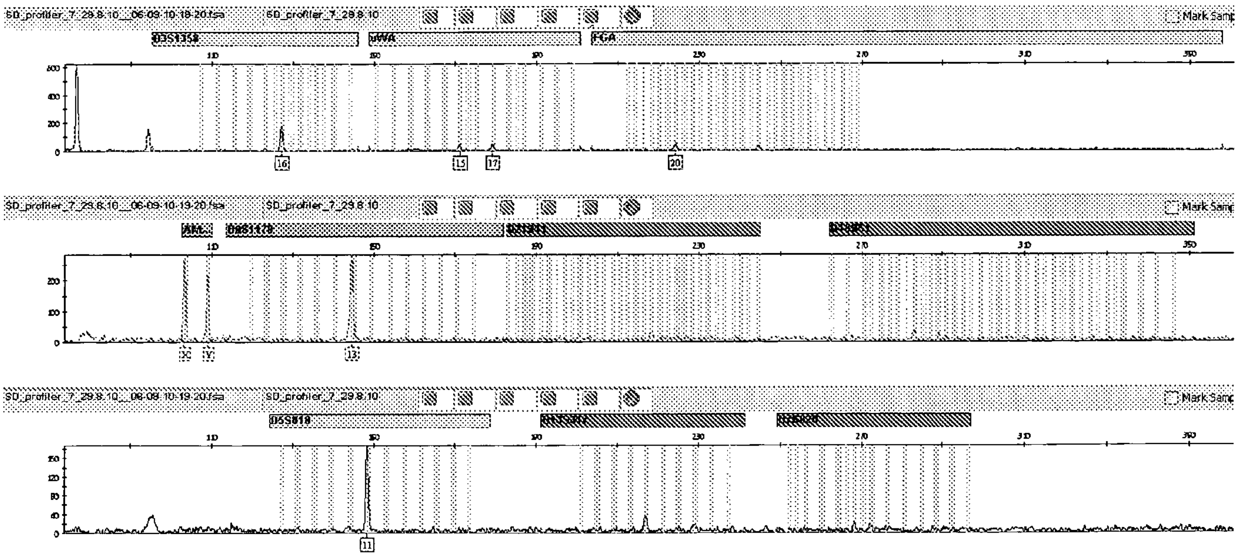

Figure 7

**Table 1 – Panel of loci used for tissue identification assay**

| # | Locus | Location [a] | Forward primer | Reverse primer | Size [b] | Conc. [c] |
|---|---|---|---|---|---|---|
| 1 | L91762 | Chr12 (73985697) | GCAGCAGGCCGCGGAGAAG (FAM) | AGCAGCTGTGCCGGGCCAG | 66 | 0.2μM |
| 2 | L68346 | Chr3 (12175317) | CAGCAACAGCACCCAGCTTG (JOE) | CACAGGCTCAGTCGCGGATC | 70 | 0.2μM |
| 3 | L50468 | Chr3 (55492965) | AGGAAACCTCAGTAGCAAAATTG (JOE) | GCGAGACTTTAGGTGTGCATC | 75 | 0.2μM |
| 4 | L14432 | Chr22 (29866149) | CGTAGGCTGCGGTGAGCTC (FAM) | GATCCATGCCCGCTGGGATG | 75 | 0.2μM |
| 5 | L4648 | Chr1 (35815331) | CAGCCTAGACGTCAAGTTACAG (JOE) | ACGACCTCCGGATCCAACTG | 80 | 0.2μM |
| 6 | L39664 | Chr17 (53710330) | CCCAGCTGGTTGGACATGTTG (FAM) | CACTTCCTTCGTGGACGCC | 82 | 0.2μM |
| 7 | L30139 | Chr17 (36996489) | GAGAAGCGGGAGGATGAGAC (FAM) | CCGCATCTCCTCCGTCCTG | 84 | 0.2μM |
| 8 | L55429 | Chr5 (1548043) | GCCTTCAGCAGGAAGTCCAC (JOE) | CCTGTGCCTCACACAGACTC | 88 | 0.2μM |
| 9 | L62086 | Chr19 (40478538) | GTGCATGGTGTCTGGTACTTC (FAM) | GAAGCTCTCGTGGACTACTTG | 89 | 0.1μM |
| 10 | L76138 | Chr19 (3130217) | CAGCCTGCTCTTCACTGCAG (JOE) | AGAGGCCGATGAAGCCGTAG | 100 | 0.2μM |
| 11 | L15952 | Chr7 (2741305) | CTCCCTGATTTACGACAAGTTC (FAM) | GACAGTATGCTGATGCTTCTTG | 117 | 0.2μM |
| 12 | L36599 | Chr19 (4867642) | AAGGGCAGAGTTCCGCTGTC (FAM) | CGGATGCAGGAGGATCCTAG | 130 | 0.2μM |
| 13 | L26688 | Chr17 (77844826) | CGGACCAGATTGCTGGTCAC (JOE) | CGACCTTGCCAGATGTTTGAC | 134 | 0.2μM |
| 14 | L81528 | Chr19 (47395603) | AGCCTCATCCACACTGACCAG (JOE) | TCAGAGCTCTCCTATCTGGAC | 141 | 0.2μM |
| 15 | L36556 | Chr19 (50962118) | GCCAGGCCGTTGATGATGAC (JOE) | GAATATGGAGCCCTGGGCAG | 149 | 0.3μM |

[a] Genomic location of the examined CG according to UCSC version hg18 is provided in brackets following the chromosome number

[b] in bps

[c] The concentration refers to both the forward and reverse primers

Figure 8

| **Table 2. Signal ratios of L91762/L68346 and L76138/L26688** | | | | |
|---|---|---|---|---|
| | L91762/L68346 | | L76138/L26688 | |
| | mean±std | range | mean±std | range |
| Semen | 0.16±0.14 | 0.04-0.53 | 6.50±5.80 | 2.04-19.00 |
| Blood | 4.36±1.69 | 2.15-7.73 | 0.30±0.20 | 0.08-0.76 |
| Saliva | 7.10±3.30 | 3.36-15.38 | 0.56±0.41 | 0.16-1.54 |
| Skin epidermis | 11.40±4.20 | 4.60-18.28 | 8.41±5.58 | 2.1-19.56 |

Figure 9

| Table 3. Loci used for assessing HhaI digestion | | | |
|---|---|---|---|
| **Location*** | **Forward primer** | **Reverse primer** | **Size (bp)** |
| Chr7 (141084738) | GCGAAGGAAGTGCTGGAGTC (FAM) | GTTTCTTGAAAGGGCCAGACAC | 94 |
| Chr11 (86241803) | CAAAGTACTGGGGTTACAGGTG (FAM) | GGATGAACCTTTAAGACATCATC | 97 |

* Genomic location of the examined CG according to UCSC version hg18 is provided in brackets following the chromosome number (in locus L98328, which does not have a HhaI recognition site, the location is of the beginning of the forward primer)

Figure 10

| Table 4. Panel of loci used for semen detection | | | | | |
|---|---|---|---|---|---|
| **Locus** | **Location** [a] | **Forward primer** | **Reverse primer** | **Size** [b] | **Conc.** [c] |
| L68346 | Chr3 (12175317) | CAGCAACAGCACCCAGCTTG (FAM) | CACAGGCTCAGTCGCGGATC | 70 | 0.2μM |
| L16264 | Chr2 (2734005) | GGACGAGTTAACTTCCTTAATTTC (FAM) | GTTTCTTCGCGGAACCTGGTTTAACTTC | 95 | 0.2μM |

[a] Genomic location of the examined CG according to UCSC version hg18 is provided in brackets following the chromosome number

[b] in bps

[c] The concentration refers to both the forward and reverse primers

Figure 11

| Table 5. Detection and quantification of semen component in semen-saliva mixtures | | | |
|---|---|---|---|
| **Sample number** | **% semen in sample (according to STR typing)** | **% semen in sample (according to our assay)** | **Semen detected?** |
| 1 | 100 | 100 | Yes |
| 2 | 100 | 100 | Yes |
| 3 | 100 | 100 | Yes |
| 4 | 76 | 66 | Yes |
| 5 | 57 | 47 | Yes |
| 6 | 30 | 23 | Yes |
| 7 | 16 | 15 | Yes |
| 8 | 0 | 0 | No |
| 9 | 0 | 0 | No |
| 10 | 0 | 0 | No |

Each row represents a single reaction

Figure 12

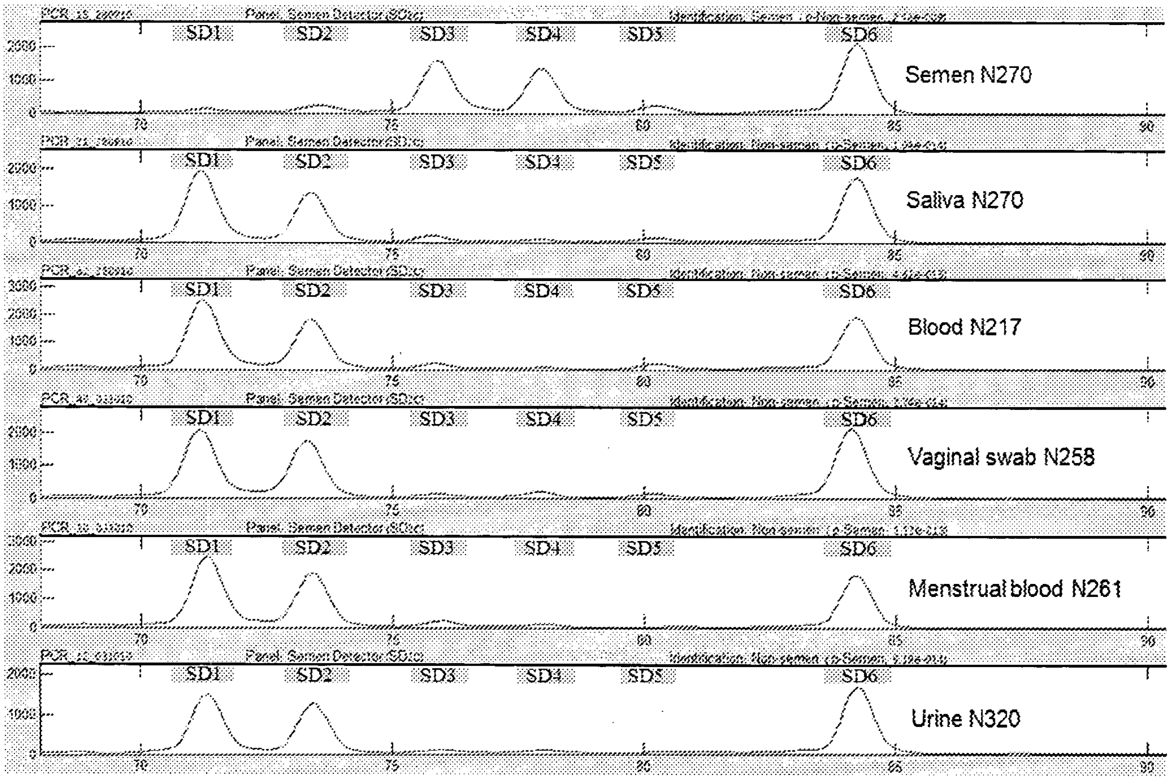
SD1
SD2
SD3
SD4
SD5
SD6
Semen N270
Saliva N270
Blood N217
Vaginal swab N258
Menstrual blood N261
Urine N320
70
75
80
85
90

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **D. FRUMKIN et al.** Authentication of forensic DNA samples. *Forensic Sci. Int. Genet.,* 2009
- Current Protocols in Molecular Biology. 1997, vol. I-III
- **SAMBROOK et al.** Molecular Cloning: A Laboratory Manual. Cold Spring Harbor Laboratory Press, 1989
- DNA Cloning: A Practical Approach. 1985, vol. I and II
- Oligonucleotide Synthesis. 1984
- Nucleic Acid Hybridization. 1985
- Transcription and Translation. 1984
- **PERBAL.** *A Practical Guide to Molecular Cloning*
- Meth. Enzymol. Academic Press, Inc, 1984
- Gene Transfer Vectors for Mammalian Cells. Cold Spring Harbor Laboratory, 1987
- Meth. Enzymol. vol. 154-155
- **OLEJNICZAK M ; KRZYZOSIAK WJ.** *Electrophoresis,* October 2006, vol. 27 (19), 3724-34
- **SAMBROOK et al.** Molecular Cloning: A Laboratory Manual. Cold Spring Harbor, 1989